(19) Europäisches Patentamt
European Patent Office
Office européen des brevets

(11) EP 0 521 985 B1

(12) **EUROPEAN PATENT SPECIFICATION**

(45) Date of publication and mention
of the grant of the patent:
**24.09.1997 Bulletin 1997/39**

(21) Application number: **91906955.9**

(22) Date of filing: **20.03.1991**

(51) Int Cl.$^6$: **C12N 15/13**, C12N 15/62,
C12N 15/17, C12N 15/18,
A61K 47/48, C07K 16/18,
A61K 39/395

(86) International application number:
**PCT/US91/01844**

(87) International publication number:
**WO 91/14438 (03.10.1991 Gazette 1991/23)**

(54) **CHIMERIC ANTIBODIES WITH RECEPTOR BINDING LIGANDS IN PLACE OF THEIR CONSTANT REGION**

CHIMÄRE ANTIKÖRPER MIT REZEPTOR-BINDENDEN LIGANDEN ANSTELLE IHRER KONSTANTEN REGION

ANTICORPS CHIMERIQUES UTILISANT DES LIGANDS DE LIAISON DE RECEPTEURS A LA PLACE DE LEUR REGION CONSTANTE

(84) Designated Contracting States:
**AT BE CH DE DK ES FR GB GR IT LI LU NL SE**

(30) Priority: **20.03.1990 US 496409**

(43) Date of publication of application:
**13.01.1993 Bulletin 1993/02**

(73) Proprietor: **THE TRUSTEES OF COLUMBIA UNIVERSITY IN THE CITY OF NEW YORK
New York, New York 10027 (US)**

(72) Inventors:
• **MORRISON, Sherie, L.
Los Angeles, CA 90048 (US)**
• **SHIN, Seung-Uon
Canoga Park, CA 91304 (US)**

(74) Representative: **VOSSIUS & PARTNER
Postfach 86 07 67
81634 München (DE)**

(56) References cited:
EP-A- 0 271 227    EP-A- 0 323 806
EP-A- 0 439 095    WO-A- 88/09344
US-A- 4 816 567

- WALDMANN H (ED):MONOCLONAL ANTIBODY THERAPY. PROGRESS IN ALLERGY vol. 45, 1988, pages 147 - 160 J.MENDELSOHN 'Growth Factor Receptors as Targets for Antitumor Therapy with Monoclonal Antibodies'
- NATURE. vol. 312, 13 December 1984, LONDON GB pages 604 - 608 NEUBERGER ET AL. 'Recombinant antibodies possessing novel effector functions'
- Methodol Surr. Biochem. Anal., Vol. 15, issued 1985, WINTER et al., "Anote on restructuring Enzymes and antibodies", pages 139-140, see Abstract No. 1364SOM.
- Proc. Natl. Acad. Sci., Vol. 87, issued July 1990, SHIN et al., "Expression and Characterization of an Antibody binding specificity joined to insulin-like growth factor 1: Potential applications for Cellular targeting", pages 5322-5326, see entire article.

- Nucleic Acid Research, Vol. 17 No. 24, issued 1989, CLARCKSON et al., "Sticky feet-directed mutagenesis and its application to swapping antibody domains", pages 10163-10170, see materials and methods.
- Science, Vol. 238, issued 20 November 1987, VITTETA et al., "Redesigning Natures Poisons to Create Anti-tumor Reagents", pages 1098-1104, see entire document.
- J.Biol.Chem., vol.269, pp.4979-4985 (S-U.Shin et al, 1994) Cell Biophysics vol.24-25, pp.45-50 (GT.Stevenson et al, 1994) Cell Biophysics vol.24-25, pp.209-218 (WC.Dougall and MI-Greene. 1994) Cell Biophysics, vol.24-25, pp.219-228 (SK.Sharma et al, 1994) Cancer Immunology, Immunotherapy, vol.32, pp.303-310 (RD.Blumenthal et al, 1991) Proc.Natl.Acad.Sci. USA vol.91, pp.9077-9080 (JH.Kordower et al,1994) Proc.Natl.Acad.Sci. USA vol.88, pp.4771-4775 (PM.Friden et al, 1991)
- J.Exp.Medicine, vol.161, pp.1315-1325 (G.Schulz et al, 1985) Cancer Research, vol.47, pp.2771-2776 (A.Eisenthal et al, 1987) Int.J.Cancer, Suppl.2, pp.89-94 (G.Schulz et al, 1988) J.Exp.Medicine, vol.168, pp.2193-2206 (M.Awwad et al, 1988) Int.J.Hyperthermia, vol.11, pp.59-72 (JM.Schuster et al, 1995) Proc.Natl.Acad.Sci. USA, vol.92, pp.2765-2769 (IH.Pastan et al, 1995)
- Cancer Research, vol.54, pp.4719-4725 (MR.Zalutsky et al, 1994) J.Neuroimmunol, vol.52, pp.127-137 (X.He et al, 1994) Progr. in Brain Res., vol.101, pp.213-223 (CJ.Wikstrand et al, 1994) Investigative Radiology vol.28, pp.488-496 (SC.Schold et al, 1993) Proc.Natl.Acad.Sci. USA, vol.92, pp.5689-5693 (YW.He et al, 1995) Blood, vol.84, pp.2457-2466 (DG.Maloney et al, 1994)

Remarks:
> The file contains technical information submitted after the application was filed and not included in this specification

**Description**

This application is a continuation-in-part of U.S. Serial No. 496,409, filed March 20, 1990, the contents of which are hereby incorporated by reference into the subject application.

This invention was made with support under Grant Number NIH-CA16858 from the National Institute of Health, U. S. Department of Health and Human Resources. Accordingly, the U.S. Government has certain rights in the invention.

## BACKGROUND OF THE INVENTION

Throughout this application, various publications are referenced by Arabic numerals within parentheses. Full citations for these publications may be found at the end of the specification immediately preceding the claims. The disclosures of these publications in their entireties are hereby incorporated by reference into this application in order to more fully describe the state of the art as known to those skilled therein as of the date of the invention described and claimed herein.

The challenge in cancer therapy has been to find a means of selectively killing malignant cells while leaving normal cells intact. Traditional chemotherapy has been directed against actively dividing cells with a drawback being that it also kills normal, actively proliferating cells, e.g. the bone marrow. Another challenge is to produce antibodies which have access to regions of the subject generally inaccessible to most molecules, e.g., the brain.

Antibodies, because of their remarkable specificity, have long had appeal as the "magic bullet" capable of selectively identifying and eliminating malignant cells.

Transfected cells (transfectomas) provide an approach to improving monoclonal antibodies. Genetically engineered antibodies can be expressed following gene transfection into lymphoid cells (1-5). One of the major advantages of expressing genetically engineered antibodies is that one is not limited to using antibodies as they occur in nature. In particular, nonimmunoglobulin sequences can be joined to antibody sequences, creating multifunctional molecules.

Among the problems encountered when investigators have attempted to use monoclonal antibodies as immunotherapeutic agents is efficiently targeting the antibodies to tumor cells while leaving normal cells untouched. In trying to overcome this problem, we have found that both the growth factor and an anti-tumor specificity can be contained in a single molecule and function synergistically. Therefore, we have joined growth factors to an antibody combining specificity in order to produce molecules which effectively target tumor cells possessing growth factor receptors. Growth factor receptors have also been reported to be on the blood-brain barrier therefore the molecules described herein may be able to utilize growth factor receptors for transcytosis into the brain (18, 49, 15). Growth factors are appropriate because cancer cells usually express growth factor receptors thus reflecting their increased capacity for proliferation.

Presently, antibodies directed to the IL-2 receptor have been used to target therapy to T cell malignancies (67, 68). Alternatively, receptor ligands have been used as a therapeutic to target a particular cell. Further, blocking cellular receptors effectively interferes with the cells' ability to proliferate.

The molecules described herein include genetically engineered antibodies having insulin or insulin-like growth factors type 1 and type 2 (IGF1 and IGF2) joined to an antibody combining specificity. Insulin and IGF1 and IGF2 are related polypeptides which affect cell metabolism and proliferation by binding to specific receptors on the plasma membrane. IGF1 is identical to somatomedin-C. The receptors for insulin and IGF1 are similar in molecular size and substructure.

Insulin can bind to the IGF1 receptor but binding is weaker than that of IGF1. Recently IGF1 has been shown to be an autocrine growth factor for certain human mammary carcinoma cells in culture (35). Both the insulin and IGF receptors are thought to be present on the blood-brain barrier and to effect transcytosis across it (1, 16-19, 48).

Additionally, the molecules described herein include genetically engineered antibodies having transferrin joined to an antibody combining specificity. Transferrin receptors are widely distributed on human tumors (20). In addition, the vascular endothelium of the brain capillaries express transferrin receptors whereas those of other tissues do not (26). Antibodies directed against transferrin receptors inhibit growth of tumor cells by crosslinking the transferrin receptor (54). However, only IgM antibodies were effective thereby suggesting that a polymer is important. Moreover, IgG antibodies increased the turnover of the transferrin receptor (69) suggesting that an alternative approach to inhibiting cell growth would be to increase receptor degradation during recycling.

The transferrin receptor binds to the major serum iron transport protein, i.e. transferrin, and mediates iron uptake into the cell. The basic mechanism by which the transferrin receptor mediates iron uptake has been established. After binding of iron-laden transferrin to the receptor, receptor-ligand complexes are taken up via coated pits and accumulate within endocytic vesicles (4, 23, 24). Acidification of this intracellular membrane compartment leads to the dissociation of iron from transferrin and the apotransferrin-transferrin receptor complex is then recycled back to the cell surface. Under neutral conditions, apotransferrin rapidly dissociates from the receptor which is then available to undergo another round of endocytosis (11, 30, 65, 70).

Endocytosis and recycling of receptor occur rapidly and efficiently. Most receptors are returned to the cell surface during each cycle and few are diverted into lysosomes and degraded. The mechanism by which iron is transported into the cytoplasm is unknown. In order to target antibodies to the cytoplasm, antibodies must be resistant to degradation in the endocytic vesicles.

The production of recombinant murine Fab-like antibodies comprising an active enzyme moiety or a polypeptide displaying c-myc antigenic determinants at the constant region of the heavy chain have been reported (43). In contrast, we have joined insulin-like growth factor 1 (IGF1) to a murine anti-dansyl (Dns) combining specificity and the heavy (H) chain constant (C) region $C_H2$ domain from human IgG3. When this chimeric H chain was transfected into a myeloma cell along with the dansyl-specific light (L) chain, the expected molecule was produced, assembled, and secreted. The resulting chimeric proteins bound the hapten Dns. They also were bound by the growth factor receptor, but with reduced efficiency, and exhibited some of the functions of IGF1 such as increasing uptake of α-aminoisobutyric acid and 2-deoxy-D-glucose (2-dGlc) (2) .

## SUMMARY OF THE INVENTION

The present invention provides a modified chimeric monoclonal antibody comprising two molecules of each of two different polypeptides. The shorter polypeptides function as the light chains of the antibody and the longer polypeptides function as the heavy chains of the antibody. Moreover, the polypeptide which functions as a heavy chain has a variable region characteristic of a first mammal and a constant region characteristic of a second mammal. Each polypeptide which functions as a light chain has a variable region characteristic of a mammal and a constant region characteristic of a mammal, wherein a receptor-binding ligand replaces at least a portion of the constant region of each of the polypeptides which function as the heavy chains of the antibody.

Additionally, the present invention provides an immunologically reactive complex comprising two different polypeptides, the shorter of which functions as a light chain and the longer of which functions as a heavy chain. The polypeptide which functions as the heavy chain has a variable region characteristic of a first mammal and a constant region characteristic of a second mammal and the polypeptide which functions as the light chain has a variable region characteristic of a mammal and a constant region characteristic of a second mammal. Moreover, a receptor-binding ligand replaces at least a portion of a constant region of one of the polypeptides.

The invention also provides a chimeric polypeptide capable of functioning as a heavy chain of an antibody. The chimeric polypeptide comprises a variable region characteristic of a first mammal and a constant region characteristic of a second mammal. Moreover, a receptor-binding ligand replaces at least a portion of the constant region of the polypeptide. Additionally, the present invention provides a chimeric polypeptide capable of functioning as a light chain of an antibody comprising a variable region characteristic of a first mammal and a constant region characteristic of a second mammal, wherein a receptor-binding ligand replaces at least a portion of the constant region of the polypeptide.

Moreover, the present invention additionally provides a modified chimeric monoclonal antibody comprising two molecules of each of two different polypeptides, the shorter of which functions as the light chains of the antibody and the longer of which polypeptides function as the heavy chains of the antibody. Each polypeptide which functions as a heavy chain has a variable region characteristic of a first mammal and a constant region characteristic of a second mammal. Further, each polypeptide which functions as a light chain has a variable region characteristic of a mammal and a constant region characteristic of a mammal, wherein a receptor-binding ligand is covalently attached to the ends of the constant regions of each of the polypeptides which function as the heavy chains of the antibody.

Further, another immunologically reactive complex is provided. The complex comprises two different polypeptides, the shorter of which functions as a light chain and the longer of which functions as a heavy chain, the polypeptide which functions as the heavy chain having a variable region characteristic of a first mammal and a constant region characteristic of a second mammal and the polypeptide which functions as the light chain having a variable region characteristic of a mammal and a constant region characteristic of a second mammal, wherein a receptor-binding ligand is covalently attached to the ends of a constant region of one of the polypeptides.

The present invention also provides a chimeric polypeptide capable of functioning as a heavy chain of an antibody comprising a variable region characteristic of a first mammal and a constant region characteristic of a second mammal, wherein a receptor-binding ligand is covalently attached to the constant region of the polypeptide.

Moreover, the present invention further provides a chimeric polypeptide capable of functioning as a light chain of an antibody comprising a variable region characteristic of a first mammal and a constant region characteristic of a second mammal, wherein a receptor-binding ligand is covalently attached to the constant region of the polypeptide.

Finally, the invention provides methods of using and producing the modified chimeric monoclonal antibodies.

## BRIEF DESCRIPTION OF THE FIGURES

**Figure 1**.

**(A)** Strategy for the construction of the IgG3-IGF1 fusion gene. The fourth base (C) of the gene for the $C_H2$ domain in human IgG3 was mutated to G by site-directed mutagenesis, resulting in the introduction of a unique Pvu II restriction enzyme site. In addition, a unique Pvu II site was introduced into the IGF1 gene by changing the last base (C) of the leader sequence (Leader Seq. or LS) of rat IGF1 cDNA (italic letters) to T. Human IgG3 and rat IGF1 cDNA digested with Pvu II were ligated, resulting in an in-frame IgG2-IGF1 fusion gene without any significant amino acid substitutions.

**(B)** Schematic representation of the transfection vectors, the genetically engineered IgG3-IGF1 fusion gene and a proposed chimeric antibody. The mouse-human κ L chain gene was cloned into [SV184▲H-neo, which is derived from pACYC184 and contains the pACY origin of replication, a chloramphenicol-resistant gene (Cm[r]) for selection in <u>Escherichia coli</u>, and the <u>neo</u> gene (the dotted box) with the simian virus 40 (SV40) early region promoter (the shaded box) for selection in eukaryotic cells. The mouse-human IgG3-rat IGF1 H chain gene is cloned into pSV2▲H-gpt gene (the stippled box). Boxes in the chimeric genes represent exons. The thick black solid line and boxes represent DNA of mouse origin, while the thin solid line and open boxes represent human DNA segments. The shaded box in the H chain genes represents the rat IGF1 cDNA. The sites of cleavage by restriction endonucleases EcoR1 (open triangle), BamHI (open circle), and HindIII (closed circle) are shown. The mouse-human IgG3-rat IGF1 chimeric protein produced by expression of both transfection vectors is shown at the bottom of B. The black region of the chimeric molecule represents the mouse V region domains specific for the hapten Dns, the open regions represent the C domains of human κ L chain and IgG3, and the hatched region represents the rat IGF1.

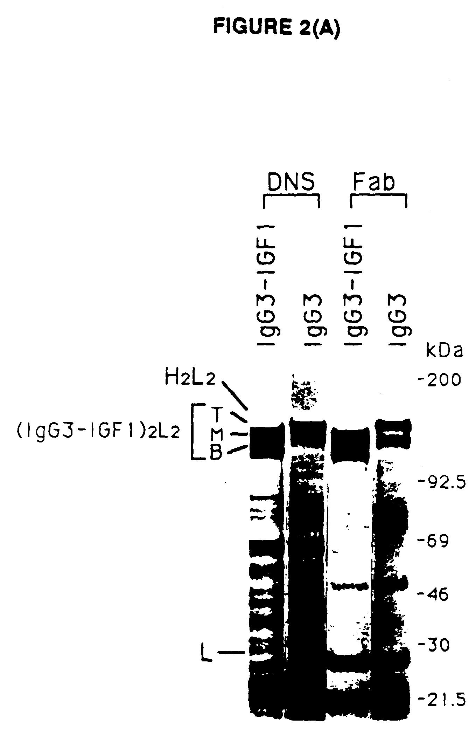

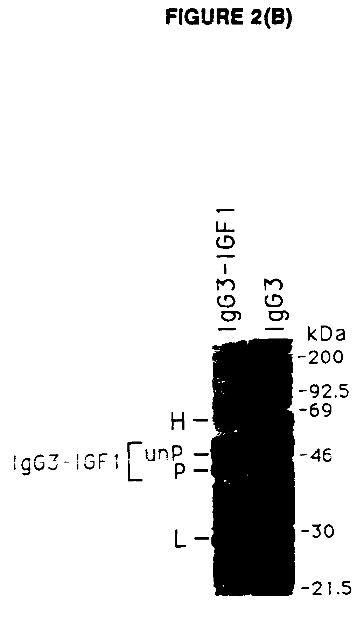

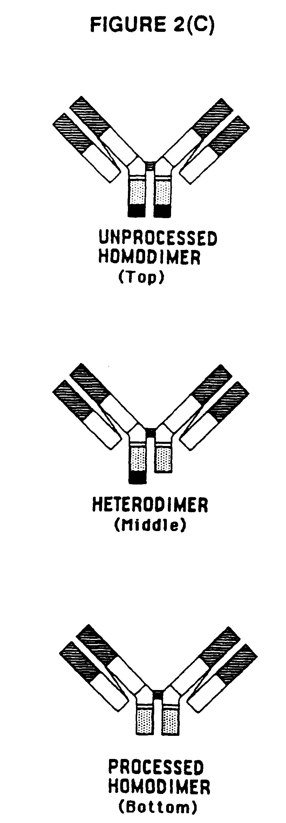

**Figure 2**. NaDodSO$_4$/PAGE analysis of the IgG3-IGF1 chimeric protein secreted by transfectomas. The secreted IgG3-IGF1 chimeric protein biosynthetically labeled with [$^{35}$S]methionine was analyzed under non reducing (**A**) and reducing (**B**) conditions. The labeled chimeric protein was precipitated with either Dns-Sepharose (lanes DNS in A) or anti-human IgG Fab antiserum/Staphylococcus protein A/IgGsorb (lanes Fab in A). Anti-Fab precipitates free L chains as well as L chains covalently attached to H chains. The secreted IgG3 chimeric antibody consisting of mouse V region-human IgG3 C region has the same basic structure as the IgG3-IGF1 chimeric protein and is used as a control. Under nonreducing conditions (A), the three bands represent the heterogeneous assembly of the processed and unprocessed chimeric protein: under reducing conditions, the processed (P) and unprocessed (unP) chimeric protein are seen (B). The schematic diagrams of heterogeneous assembly patterns are shown in C in which the hatched regions represent the mouse V regions, the open regions represent human C regions, the stippled regions represent the mature IGF1, and the black regions represent the carboxyl terminus of the unprocessed IGF1.

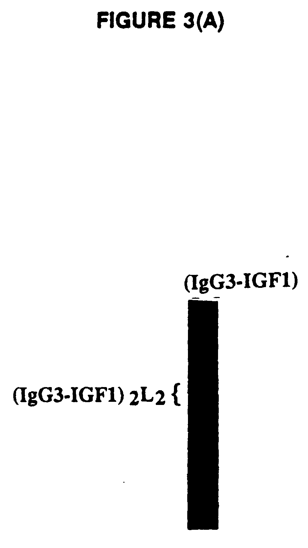

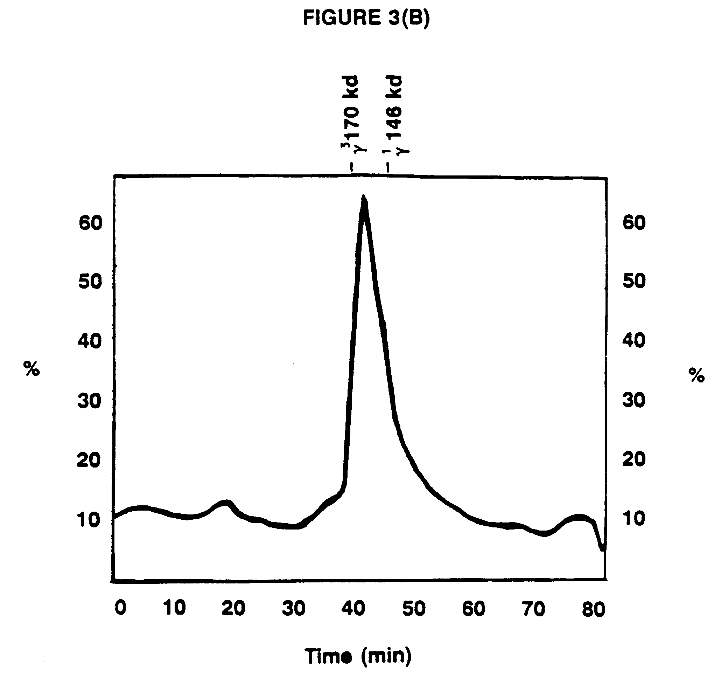

Figure 3. Purification of IgG$_3$-IGF1 fusion protein.

**A**. Purified IgG$_3$-IGF1 fusion protein was fractionated by SDS-PAGE under non-reducing conditions and visualized using silver staining.

**B**. The purified IgG$_3$-IGF1 fusion protein was fractionated using FPLC (Superose-12 column, flow rate: 0.25 ml/min). The elution time of IgG$_3$-IGF1 fusion protein is 42 min. The elution time of mouse-human IgG$_3$ chimeric antibody (170 KDa) is 40 min and that of IgG$_1$ chimeric antibody (146 KDa) is 45 min; they are indicated by arrows.

**Figure 4**. Competitive inhibition of binding of $^{125}$I-IGF1 to IM-9 lymphocytes. Approximately 3 X 10[6] IM-9 cells were incubated at 15°C with a constant amount of $^{125}$I-IGF1 and the indicated concentration of unlabeled competitors (recombinant IGF1, IgG3-IGF1 chimeric protein and IgG3 chimeric antibody). After 2 hr of incubation, the amount of receptor-bound radioactivity was determined. Values are expressed as the relative inhibition of binding compared to use of only labeled tracer $^{125}$I-IGF1. Results shown for each curve are the means of duplicate experiments.

**Figure 5**. Relationships between IGF1 and IgG3-IGF1 stimulatory effects on α-aminoisobutyric acid (AIB) (**A**) and dGlc (**B**) uptakes in KB cells. Uptakes were determined in the presence of the indicated concentrations of IGF1, IgG3-IGF1, and IgG3 as control.

**Figure 6**. Schematic diagram showing the differential centrifugation protocol which was used to obtain a nuclear/plasma membrane (Nuc/Pm) fraction, a Mitochondria (Mit) fraction, a high density microsomes (H. Micro) fraction, and a low density microsomes (L. Micro) and cytosolic (Cyto) fraction.

**Figure 7**. Diagram of pAG5018 comprising hinge-IL2 in an expression vector.

**Figure 8**. Line graph showing the relative activity of TUZ (an IL-2 fusion protein) versus IL-2.

**Figure 9**. Schematic of the Bluescript (KS) with the CD4-IGF1 insert.

**Figure 10**. Schematic of pSV2 V$_{DNS}$ HUG3 HTF comprising human transferrin.

**Figure 11**. Line graph showing IgG3-IGF1 Capillary Depletion.

## DETAILED DESCRIPTION OF THE INVENTION

Many problems have been encountered in the attempt to use monoclonal antibodies as immunotherapeutic agents. Among the problems have been the inability to (1) efficiently target antibodies to tumor cells while leaving normal cells untouched and (2) gain access to restricted locations in the body, e.g. the brain.

Accordingly, the monoclonal antibodies provided herein are structurally engineered permitting greater accessibility to the target region, e.g. the brain, than naturally occurring antibodies. Further, the subject chimeric monoclonal antibodies reproducibly bind to target cells.

The present invention provides a modified chimeric monoclonal antibody comprising two molecules of each of two different polypeptides. The shorter polypeptides function as the light chains of the antibody and the longer polypeptides function as the heavy chains of the antibody. Moreover, the polypeptide which functions as a heavy chain has a variable region characteristic of a first mammal and a constant region characteristic of a second mammal. Each polypeptide which functions as a light chain has a variable region characteristic of a mammal and a constant region characteristic of a mammal, wherein a receptor-binding ligand replaces at least a portion of the constant region of each of the polypeptides which function as the heavy chains of the antibody.

As used herein, modified chimeric monoclonal antibody means a genetically engineered protein comprising (1) four polypeptides, two of which are designated heavy chains and two of which are designated light chains; the polypeptides encoded by a nucleic acid molecule having diverse genetic constitutions; and (2) altered at the constant region of the heavy chain.

As used herein, the heavy (H) and light (L) chains of the modified chimeric monoclonal antibody refer to the differences in the molecular weight of the polypeptides which compose the above-described antibodies.

Further, as used herein, the variable (V) region of the modified chimeric monoclonal antibody are the sequences on both the light- and heavy-chain located at the amino terminal end of the molecule.

Moreover, as used herein, the constant (C) region of the modified chimeric monoclonal antibody are the sequences on both the light- and heavy-chain at the carboxyl terminal portion of the antibody. Accordingly, the chains of the immunoglobulin molecule can be divided into their V and C components so that a light chain consists of two parts, i.e. $V_L$ and $C_L$ (variable $_{light}$ and constant $_{light}$). Similarly, the heavy chain can be divided into $V_H$ and $C_H$ (variable $_{heavy}$ and constant $_{heavy}$).

One embodiment of the present invention provides that the first mammal is mouse and the second mammal is human. In another embodiment, the first mammal is human and the second mammal is mouse. Moreover, also within the scope of this invention are chimeric monoclonal antibodies which comprise polypeptides from mammals such as rats, moles, shrews, monkeys, bats, hares, rabbits, dogs, cats, whales, dolphins, elephants, horses, cows, deers or any combination thereof.

Further, in accordance with the practice of the invention, the variable region and the constant region of the light chain are both characteristic of the second mammal. Alternatively, the variable region and the constant region of the light chain are both characteristic of the first mammal. A further alternative provides that the variable region of the light chain is characteristic of either the first or the second mammal and the constant region of the light chain is characteristic of the other mammal.

Further, the subject invention provides that the receptor-binding ligand comprises a growth factor. Examples of growth factors include but are limited to insulin, insulin-like growth factor, platelet-derived growth factor, epidermal growth factor, transforming growth factor, nerve growth factor, and growth hormone.

As used herein, insulin means either (1) naturally-occurring insulin whether of human or animal origin or 2) biosynthetic insulin from human or animal whether produced by genetic engineering methods or otherwise.

Similarly, as used herein, insulin-like growth factor, platelet-derived growth factor, epidermal growth factor, transforming growth factor, nerve growth factor, and growth hormone means either the naturally-occurring or synthetic form thereof.

Further, examples of insulin-like growth factor include but are not limited to insulin growth factor 1 and insulin growth factor 2.

In the practice of the invention a growth hormone includes but is not limited to growth hormone releasing factor. Moreover, as used herein, examples of growth hormones include human, avian, equine, porcine, ovine, bovine, and piscine growth hormones.

Additionally, examples of transforming growth factor include but are not limited to transforming growth factor-α and transforming growth factor-β, i.e. transforming growth factor-β1, transforming growth factor-β2, and transforming growth factor-β3.

Additionally, in accordance with the practice of the invention, the receptor-binding ligand which replaces at least a portion of the constant region of the heavy chain polypeptides comprises tumor necrosis factor.

Alternatively, in another embodiment of the invention the receptor-binding ligand comprises transferrin. Further, in another embodiment of the invention the receptor-binding ligand comprises a lymphokine. Examples of suitable lymphokines are selected from a group consisting of macrophage inhibition factor, leukocyte migration inhibition factor, macrophage activating factor, macrophage cytotoxicity factor, interleukin-1, interleukin-2, interleukin-3, interleukin-4, interleukin.5, interleukin-6, lymphotoxin, monocyte-derived lymphocyte activating factor, and T helper cell replacing factor.

As used herein, a lymphokine means either (1) a naturally-occurring lymphokine whether of human or animal origin or (2) a biosynthetic lymphokine from human or animal whether produced by genetic engineering methods or otherwise.

Moreover, in the practice of this invention the antibody is selected from, but not limited to, any antibody from a group comprising an IgG, IgA, IgD, IgE or IgM antibody or any combination thereof.

Also, in accordance with the practice of the invention the variable region of the previously described chimeric monoclonal antibody comprises immunoglobulin-like ligand binding regions having a folded configuration. Examples of immunoglobulin-like ligand binding regions include, but are not limited to, domains from T cell receptors, major histocompatibility complex antigens, CD4, and CD8.

In accordance with the practice of the invention, the variable region comprises the domain of a T cell receptor. Alternatively, in another embodiment, the variable region comprises the domain of a MHC antigen, e.g. an HLA antigen or an H-2 antigen. In a further embodiment, the variable region comprises the domain of a surface glycoprotein CD4. Moreover, in another embodiment the variable region comprises the domain of a surface glycoprotein CD8.

The CD4 molecule is a cell surface glycoprotein which interact with targets bearing class II major histocompatibility complex (MHC) molecules (73). CD4 acts as a recognition molecule mediating appropriate interactions between the CD4+ T lymphocytes and its target. Additionally, CD4 is the cell surface receptor for the AIDS virus. Accordingly, the subject chimeric monoclonal antibodies having a variable region of the heavy chain comprising the CD4 domain is important as a therapeutic and diagnostic agent in mediating T lymphocyte function and alleviating AIDS.

Additionally, CD8 is a cell surface glycoprotein which is found on a subpopulation of T lymphocytes, i.e. CD8+ T lymphocytes. CD8+ T lymphocytes interacts with cells expressing class I MHC molecules. Accordingly, monoclonal antibodies comprising CD8 inhibit T cell function by specifically binding with molecules which are directed against CD8. In this regard, the subject chimeric monoclonal antibody is important in mediating T lymphocyte function, specifically, the embodiment of the invention, wherein the variable region of the heavy chain comprises the CD8 domain.

T cell receptors are special membrane-anchored cell surface proteins which are found on T cells and are analogous to antibodies. Moreover, T cell receptors are the mechanism by which T cells interact with antigen, thereby, allowing T cells to carry out cellular immune responses. In this regard, the subject chimeric monoclonal antibodies comprising a domain of the T cell receptor, compete with naturally-occurring T cell receptors for the antigen, thus, preventing T cells from mediating cellular immunity. This embodiment of the subject invention is useful in treating auto-immune diseases, e.g. lupus erythmatosus, AIDS.

As used herein, a major histocompatibility antigen means a transplantation antigen, i.e. of either mouse origin such as H-2 class proteins or of human origin such as HLA proteins. Transplantation antigens are structurally similar to antibody molecules.

Further, the subject chimeric monoclonal antibodies comprising a domain of MHC antigens are important in preventing rapid rejection of organ grafts between individuals by engaging in binding competition with transplantation antigens, i.e. HLA or H-2 antigens, thereby preventing rapid rejection of organ grafts between individuals.

Additionally, the present invention provides an immunologically reactive complex comprising two different polypeptides, the shorter of which functions as a light chain and the longer of which functions as a heavy chain. The polypeptide which functions as the heavy chain has a variable region characteristic of a first mammal and a constant region characteristic of a second mammal and the polypeptide which functions as the light chain has a variable region characteristic of a mammal and a constant region characteristic of a second mammal. Moreover, a receptor-binding ligand replaces at least a portion of a constant region of one of the polypeptides.

As used herein an immunologically reactive complex is a biosynthetic complex and is produced by genetic engineering methods or otherwise.

Moreover, the immunologically reactive complex comprises a first mammal which is human and a second mammal which is mouse. Alternatively, the first mammal is mouse and the second mammal is human. Moreover, also within the scope of this invention are immunologically reactive complexes which comprise polypeptides from mammals such as rats, moles, shrews, monkeys, bats, sloths, hares, rabbits, dogs, cats, whales, dolphins, elephants, horses, cows, deers or any combination thereof.

Further, in one embodiment of the invention, the variable region and the constant region of the light chain of the immunologically reactive complex are both characteristic of the second mammal. Alternatively, the variable region and the constant region of the light chain are both characteristic of the first mammal. Another alternative provides that the variable region of the light chain is characteristic of either the first or the second mammal and the constant region of the light chain is characteristic of the other mammal. For example, if the variable region of the light chain is characteristic of the first mammal then the constant region of the light chain is characteristic of the second mammal.

The invention provides the immunologically reactive complex, wherein the receptor-binding ligand replaces at least a portion of the constant region of the polypeptide which functions as the light chain. Alternatively, the receptor-binding ligand replaces at least a portion of the constant region of the polypeptide which functions as the heavy chain.

Also, in accordance with the invention the receptor-binding ligand of the immunologically reactive complex is a growth factor. Suitable examples of growth factors include, but are not limited to, insulin, insulin-like growth factor (insulin growth factor 1 or insulin growth factor 2), platelet-derived growth factor, epidermal growth factor, transforming growth factor (such as transforming growth factor-α, transforming growth factorβ1, transforming growth factor-β2, or transforming growth factor-β3), nerve growth factor, growth hormone (such as growth hormone releasing factor), tumor necrosis factor, transferrin, and lymphokines (such as macrophage inhibition factor, leukocyte migration inhibition factor, macrophage activating factor, macrophage cytotoxicity factor, interleukin-1, interleukin-2, interleukin-3, interleukin-4, interleukin-5, interleukin-6, interleukin-7, lymphotoxin, monocyte-derived lymphocyte activating factor, and T helper cell replacing factor).

Additionally, in accordance with the practice of the invention, the variable regions of the polypeptides of the immunologically reactive complex comprises domains of T cell receptors. Alternatively, the variable region of the polypeptides comprise domains of MHC antigens (such as an HLA antigen or an H-2 antigen). Further alternatively, the variable regions of the polypeptides comprise domains of surface glycoproteins CD4 or CD8.

Additionally, the present invention provides a chimeric polypeptide capable of functioning as a heavy chain of an antibody. The chimeric polypeptide comprises a variable region characteristic of a first mammal and a constant region characteristic of a second mammal, wherein a receptor-binding ligand replaces at least a portion of the constant region of the polypeptide. Alternatively, the receptor-binding ligand is joined to at least a portion of the constant region of the polypeptide.

Additionally, the present invention provides a chimeric polypeptide capable of functioning as a light chain of an antibody. The chimeric polypeptide comprises a variable region characteristic of a first mammal and a constant region characteristic of a second mammal, and a receptor-binding ligand which replaces at least a portion of the constant region of the polypeptide. Alternatively, the receptor-binding ligand is joined to at least a portion of the constant region of the polypeptide.

As used herein the chimeric polypeptide is a biosynthetic polypeptide and is made by genetic engineering methods or otherwise.

In accordance with the practice of the invention, the first mammal is human and the second mammal is mouse. Alternatively, the first mammal is mouse and the second mammal is human. Moreover, also within the scope of this invention are chimeric polypeptides which comprise polypeptides from mammals such as rats, moles, shrews, monkeys, bats, sloths, hares, rabbits, dogs, cats, whales, dolphins, elephants, horses, cows, deers or any combination thereof.

Further, the present invention provides a nucleic acid molecule encoding the above-described chimeric polypeptide. Moreover, the present invention provides an expression vector for producing a chimeric polypeptide comprising a nucleic acid encoding the chimeric polypeptide and suitable regulatory elements positioned within the vector so as to permit expression of the polypeptide in a suitable host.

It would be clear to those in the art that "suitable regulatory elements" would encompass the genetic elements that control gene expression, e.g. the origin of replication, promoter, and expression control sequences such as enhancers.

Further, the present invention provides the previously described modified human chimeric monoclonal antibody to which a moiety is attached, i.e. a drug or a detectable label, wherein attachment may be effected at the variable region of the molecule.

Moreover, examples of suitable drugs include but are not limited to a cytotoxic agent, e.g. methotrexate, decarbazine, toxins (such as ricin, diphtheria toxin, pseudomonas, exotoxin-A, abrin, supporin, and gelnoin), anti-infectious agents, anti-septic agents, and antimetabolites.

As used herein ricin and abrin means ricin and abrin isolated from a wide variety of natural sources, e.g. seeds, or synthesized by genetic engineering methods or otherwise.

An example of a suitable anti-metabolite includes 5-iodo-2'-deoxyuridine (IUdR). As used herein an anti-metabolite encompasses any chemical which interferes with the replication of DNA.

Moreover, examples of suitable detectable labels include but are not limited to an enzyme, biotin, a fluorophore, a chromophore, a heavy metal, a paramagnetic isotope, or a radioisotope. By suitable detectable labels applicants contemplate any label which would be conducive to the detection of a complex which are known in the art.

It would be clear to those skilled in the art that one method of attaching the subject chimeric monoclonal antibody to an enzyme, e.g. horseradish peroxidase, would be by a modification of the periodate method (74). Alternatively, it would be clear to those skilled in the art to attach biotin to the subject chimeric monoclonal antibodies by the method of Bayer et al. (75).

With regard to drug moieties, it would be clear to those skilled in the art that the drug may be bound to the variable region of the subject chimeric monoclonal antibodies.

Further, the present invention provides a pharmaceutical composition comprising the above-described chimeric monoclonal antibody, immunological complex or polypeptide to which a moiety is attached, i.e. a drug or a detectable label, in an amount sufficient to deliver an effective dose of the drug and a pharmaceutically acceptable carrier.

As used herein, the term "pharmaceutically acceptable carrier" encompasses any of the standard pharmaceutical carriers. Such carriers are well known in the art and may include, but are in no way and are not intended to be limited to, any of the standard pharmaceutical carriers such as a phosphate buffered saline solutions, water, emulsions such as oil/water emulsion, and various types of wetting agents. Other carriers may also include sterile solutions, tablets, coated tablets, and capsules.

Typically such carriers contain excipients such as starch, milk, sugar, certain types of clay, gelatin, stearic acid or salts thereof, magnesium or calcium sterate, talc, vegetable fats or oils, gums, glycols, or other known excipients. Such carriers may also include flavor and color additives or other ingredients. Compositions comprising such carriers are formulated by well known conventional methods.

In this method, the administration of the composition may be effected by any of the well known methods, including but not limited to intravenous, intramuscular, subcutaneous and oral administration.

Additionally, the present invention provides a method of producing the above-described modified chimeric monoclonal antibody. The method comprises: a) cotransfecting a suitable/nonantibody-producing host cell with two expression plasmids, (i) one of which encodes a polypeptide capable of functioning as the heavy chain of the antibody and having a variable region characteristic of a first mammal and a constant region characteristic of a second mammal, wherein a receptor-binding ligand replaces at least a portion of the constant region of the heavy chain polypeptide and (ii) the other of which encodes a polypeptide capable of functioning as the light chain of the antibody and having a variable region characteristic of a mammal and a constant region characteristic of a mammal; (b) treating the cotransfected host cell so as to effect expression of the polypeptides encoded by the plasmids and formation of the chimeric monoclonal antibody within the host cell and excretion into the culture medium of the antibody by the host cell; and (c) recovering the resulting excreted chimeric monoclonal antibody, from the culture medium.

Further, the present invention provides another method of producing the above-described modified chimeric monoclonal antibody. The method comprises: a) cotransfecting a suitable/nonantibody-producing host cell with an expression plasmid which encodes (i) a polypeptide capable of functioning as the heavy chain of the antibody and having a variable region characteristic of a first mammal and a constant region characteristic of a second mammal, wherein a receptor-binding ligand replaces at least a portion of the constant region of the heavy chain polypeptide and (ii) a polypeptide capable of functioning as the light chain of the antibody and having both a variable region characteristic of a mammal and a constant region characteristic of a mammal; (b) treating the cotransfected host cell so as to effect expression of the polypeptides encoded by the plasmid and formation of the chimeric monoclonal antibody within the host cell and excretion into the culture medium of the antibody by the host cell; and (c) recovering the resulting excreted chimeric monoclonal antibody, from the culture medium.

One procedure for preparing monoclonal antibodies involves constructing separate light and heavy chain immunoglobulin gene transfection vectors which compatibly replicate and amplify in host cells (45). This means that each plasmid can be manipulated separately, but still be maintained together in the host cell, facilitating gene transfer of both transfection vectors into mammalian cells using protoplast fusion or electroporation methods. Each protoplast fusion event then delivers both vectors into the same mammalian cell.

As used herein, cotransfection means the essentially simultaneous insertion of both heavy and light chain genes, either by means of one or two expression vectors, into a suitable/nonantibody-producing host cell. It would be clear to those skilled in the art that a suitable/nonantibody-producing host cell would encompass any cell, both eucaryotic or procaryotic, capable of effecting expression of the polypeptides encoded by the plasmids and formation of the chimeric monoclonal antibody within the host cell and excretion into the culture medium of the antibody by the host cell.

Applicants point out that it would be clear to those skilled in the art that the vectors herein comprise any vectors known in the art which are suitable for producing the modified chimeric monoclonal antibodies of this invention. Moreover, vectors as used herein comprise plasmids, viruses (including phage) and integratable DNA fragments, i.e. fragments that are integratable into the host genome by recombination. In one example of the present invention, the vector may be a plasmid which is cloned in a bacterial cell and integrates into the host cell genome upon cotransfection.

In one embodiment of the above-described method the suitable, nonantibody-producing host cell is a human cell, i.e. a myeloma cell. Alternatively, in another embodiment, the suitable, nonantibody-producing host cell is a murine cell. Moreover, although the preferred host cells are human or murine cells, in principle any higher eucaryotic cell is workable, whether from vertebrate or invertebrate culture.

As used herein "recovering the resulting excreted chimeric monoclonal antibody, from the culture medium" means any method, e.g. separation by immunoprecipitation, solvent fractionation, classical and high pressure liquid column chromatography, i.e. size exclusion, ion exchange, partition, and adsorption chromatography in normal and reverse phase, which are generally known and accepted by those in the art as a means to separate and isolate proteins.

The present invention provides a method of delivering a drug to a cell, e.g. a brain cell, an adipose cell, a blood cell, an epithelial cell, a muscle cell, a nerve cell, or a leukemic cell, having a receptor for a growth factor on a surface.

The method comprises contacting the cell with the chimeric monoclonal antibody, immunologically reactive complex, or chimeric polypeptide, to which a moiety is attached, i.e. a drug or a detectable label, wherein the receptor-binding ligand of the antibody, immunologically reactive complex, or chimeric polypeptide, comprises the growth factor which binds to the receptor so that the antibody binds to the cell and thereby delivers the drug to the cell.

As used herein, an adipose cell means those cells responsible for the production and storage of fat. Further, epithelial cells are cells which line the inner and outer surfaces of the body.

The chimeric monoclonal antibodies, immunologically reactive complexes, and chimeric polypeptides described herein bind to receptors so as to be transported across the BBB. Small foreign molecules introduced into the circulation rapidly distribute themselves throughout the body's extracellular fluids; however, they are generally unable to penetrate the tissues of the brain, i.e. the blood-brain barrier (BBB). The BBB is a functional barrier between the brain capillaries and the brain tissue which allows some substances from the blood to enter the brain rapidly while other substances either enter slowly or not at all. Further, the BBB effectively restricts transport between blood and the central nervous system of certain molecules; especially those which are water soluble, charged, and larger than about 200 daltons. The BBB has been found to function over all anatomical regions of the central nervous system, except for small areas around the pituitary stalk, the preoptic recess and the area postreme beneath the floor of the 4th ventricle. The basis for this barrier appears to be embodied in the endothelial cells of the blood capillaries in the brain.

The BBB is not a fixed barrier. It can be influenced by the metabolic requirements of the brain, in addition to insults such as mechanical trauma, cerebral embolism, hypercapnia, hypoxia, extensive stress, radiation, electroconvulsive shock, explosive decompression, and various toxic substances. All of these conditions may alter the permeability of the barriers and, subsequently, the composition of the extracellular fluid.

Various substances are transported across the BBB either by passive diffusion or, more often, by a carrier-mediated or active form of transport. However, movement is limited; even the movement of water across the capillary wall is limited. Accordingly, the antibodies, immunologically reactive complexes, and chimeric polypeptides of this invention comprise receptor binding ligands which bind to receptor capable of facilitating transport across the BBB.

In the practice of the above-described method of delivering a drug to a cell, the cell is a brain cell and the growth factor, i.e. insulin-like growth factor 1, insulin-like growth factor 2, insulin, and transferrin, which upon binding to the receptor results in transport of the antibody, immunologically reactive complex, or chimeric polypeptide across the blood-brain barrier.

Moreover, in the above-described method, the brain cell is abnormal and associated with progressive dementia and the contacting with the chimeric antibody, immunologically reactive complex, or chimeric polypeptide comprises contacting the cell with an amount of the antibody, immunologically reactive complex, or chimeric polypeptide effective to halt the progressive dementia.

As used herein, progressive dementia is defined as the gradual deterioration or loss of intellectual faculties, reasoning, power, and memory. Alternatively, the brain cell is abnormal and associated with cerebral cortical atrophy and the contacting with the chimeric antibody, immunologically reactive complex, or chimeric polypeptide comprises contacting the cell with an amount of the antibody, immunologically reactive complex, or chimeric polypeptide effective to halt the cerebral cortical atrophy.

As used herein, cerebral cortical atrophy is a symptom typically characteristic of Alzheimer's disease.

In another embodiment, the brain cell is malignant and associated with neurosarcoma and the contacting with the chimeric antibody, immunologically reactive complex, or chimeric polypeptide comprises contacting the cell with an amount of the antibody, immunologically reactive complex, or chimeric polypeptide effective to halt the neurosarcoma.

Further, in yet another embodiment, the brain cell is malignant and associated with a carcinoma and the contacting with the chimeric antibody, immunologically reactive complex, or chimeric polypeptide comprises contacting the cell with an amount of the antibody, immunologically reactive complex, or chimeric polypeptide effective to halt the carcinoma.

Alternatively, in another embodiment, the brain cell is malignant and associated with a carcinosarcoma and the contacting with the chimeric antibody, immunologically reactive complex, or chimeric polypeptide comprises contacting the cell with an amount of the antibody, immunologically reactive complex, or chimeric polypeptide effective to halt the carcinosarcoma.

Moreover, in accordance with the practice of the invention, the brain cell is malignant and associated with a sarcoma and the contacting with the chimeric antibody, immunologically reactive complex, or chimeric polypeptide comprises contacting the cell with an amount of the antibody, immunologically reactive complex, or chimeric polypeptide effective to halt the sarcoma.

Further, the brain cell is malignant and associated with a carcinomatous cerebellar degeneration and the contacting with the chimeric antibody, immunologically reactive complex, or chimeric polypeptide comprises contacting the cell with an amount of the antibody, immunologically reactive complex, or chimeric polypeptide effective to halt the carcinomatous cerebellar degeneration. As used herein carcinomatous cerebellar degeneration means a nonmetastic carcinoma taking the form of a diffuse degeneration of the cerebellar cortex and deep cerebellar nuclei.

Additionally, in the practice of the above-described method of delivering a drug to a cell, the cell is a cell selected from, but is not limited to, a group including a blood cell, a muscle cell, a nerve cell, a bone cell, and an epithelia cell. Moreover, in accordance with the above-described method the cell is malignant and associated with a melanoma and the contacting with the chimeric antibody, immunologically reactive complex, or chimeric polypeptide comprises contacting the cell with an amount of the antibody effective to halt the melanoma.

Alternatively, in accordance with the above-described method the cell is malignant and associated with a breast cancer and the contacting with the chimeric antibody, immunologically reactive complex, or chimeric polypeptide comprises contacting the cell with an amount of the antibody, immunologically reactive complex, or chimeric polypeptide effective to halt the breast cancer. Further, the cell is malignant and associated with a lymphoma and the contacting with the chimeric antibody, immunologically reactive complex, or chimeric polypeptide comprises contacting the cell with an amount of the antibody, immunologically reactive complex, or chimeric polypeptide effective to halt the lymphoma. Also in accordance with the above-described method the cell is malignant and associated with a carcinoma and the contacting with the chimeric antibody, immunologically reactive complex, or chimeric polypeptide comprises contacting the cell with an amount of the antibody, immunologically reactive complex, or chimeric polypeptide effective to halt the carcinoma. Additionally, in accordance with the above-described method the cell is malignant and associated with a sarcoma and the contacting with the chimeric antibody, immunologically reactive complex, or chimeric polypeptide comprises contacting the cell with an amount of the antibody, immunologically reactive complex, or chimeric polypeptide effective to halt the sarcoma.

The invention further provides a method of detecting a cell having a receptor for a growth factor on its surface which comprises contacting the cell with the chimeric monoclonal antibody, immunologically reactive complex, or chimeric polypeptide to which a detectable moiety is attached, wherein the receptor-binding ligand of the antibody, immunologically reactive complex, or chimeric polypeptide comprises the growth factor which binds to the receptor so that the antibody, immunologically reactive complex, or chimeric polypeptide binds to the cell and thereby detects the cell.

In one embodiment of the method of detecting a cell having a receptor for a growth fact on its surface, the cell is a brain cell and the growth factor upon binding to the receptor results is transport of the antibody, immunologically reactive complex, or chimeric polypeptide across the blood-brain barrier. Examples of suitable growth factors is selected from the group consisting of insulin-like growth factor 1, insulin-like growth factor 2, insulin, and transferrin.

Further, in accordance with the practice of the invention, the brain cell is abnormal and associated with argyrophil plaque and the contacting with the chimeric antibody, immunologically reactive complex, or chimeric polypeptide comprises contacting the cell with an amount of the antibody, immunologically reactive complex, or chimeric polypeptide effective to permit detection of the plaque.

Alternatively, the brain cell is abnormal and associated with a brain tumor and the contacting with the chimeric antibody, immunologically reactive complex, or chimeric polypeptide comprises contacting the cell with an amount of the antibody, immunologically reactive complex, or chimeric polypeptide effective to permit detection of the tumor.

It would be clear to those skilled in the art that the detection is accomplished using methods which depend upon the identity of the detectable moiety but which are nevertheless well known. For example, when the detectable moiety is radioactive, a liquid scintillation counter is employed. Alternatively, radioactive labels are detected by radiography or other methods which detect radioactive decay after separating the unreacted detectable antibody. Moreover, when the moiety is an enzyme, e.g. horseradish peroxidase in a standard assay, a spectrophotometer is employed. Further, when the moiety is fluorescent, a fluorometer may be used, e.g. fluorescence activated cell sorting.

The present invention additionally provides a modified chimeric monoclonal antibody comprising two molecules of each of two different polypeptides, the shorter of which functions as the light chains of the antibody and the longer of which polypeptides function as the heavy chains of the antibody. Each polypeptide which functions as a heavy chain has a variable region characteristic of a first mammal and a constant region characteristic of a second mammal. Further, each polypeptide which functions as a light chain has a variable region characteristic of a mammal and a constant region characteristic of a mammal, wherein a receptor-binding ligand is covalently attached to the ends of the constant regions of each of the polypeptides which function as the heavy chains of the antibody.

Further, another immunologically reactive complex is provided. The complex comprises two different polypeptides, the shorter of which functions as a light chain and the longer of which functions as a heavy chain, the polypeptide which functions as the heavy chain having a variable region characteristic of a first mammal and a constant region characteristic of a second mammal and the polypeptide which functions as the light chain having a variable region characteristic of a mammal and a constant region characteristic of a second mammal, wherein a receptor-binding ligand is covalently attached to the ends of a constant region of one of the polypeptides.

The invention also provides a chimeric polypeptide capable of functioning as a light chain of an antibody comprising a variable region characteristic of a first mammal and a constant region characteristic of a second mammal, wherein a receptor-binding ligand is covalently attached to the ends of a constant region of one of the polypeptides.

Further, also within the scope of the invention is a chimeric polypeptide capable of functioning as a heavy chain of an antibody comprising a variable region characteristic of a first mammal and a constant region characteristic of a second mammal, wherein a receptor-binding ligand is covalently attached to the constant region of the polypeptide.

In addition to the treatment of aberrant cells and infections in the brain which require the antibody, immunologically reactive complex, or chimeric polypeptide to cross the BBB, the subject chimeric monoclonal antibodies, immunologically reactive complex, or chimeric polypeptide are also of importance for the treatment of aberrant or infected cells in all other locations of the body. For example, the subject chimeric monoclonal antibodies with or without a covalently linked anti-metastatic agent is useful for the treatment of all forms of cancer such as leukemia, lymphomas, carcinomas, adenomas, and melanomas, that reside in any part of the body.

This invention is illustrated in the Experimental Details section which follows. This section is set forth to aid an understand of the invention but is not intended to, and should not be construed to, limit in any way the invention as set forth in the claims which follow.

## EXPERIMENTAL DETAILS

## Materials and Methods

### Cell Lines:

Human lymphoblasts, IM-9, which express IGF1 receptors on their surface (51), were obtained from American Type Culture Collection and maintained in RPMI 1640 medium with 10% fetal calf serum (Hyclone, Logan, UT). Human epidermoid carcinoma, KB, which expresses growth factor receptors on its surface (37) and myeloma cells, i.e. P3X63Ag8.653 cells, were cultured in Iscove's Modified Dulbecco's Medium (IMDM, GIBCO, Grand Island, NY) with 10% calf serum (Hyclone).

## Characterization of the resulting monoclonal antibodies

### Carotid artery injection technique: based on Journal of Clinical Investigation 74:745-752, 1984

Inject the $^{35}$S-test compound and $^3$HOH (a freely diffusible internal reference) into the common carotid artery of anesthetized adult rat (2-3 rats, male, Sprague-Dawley; 200-300 grams) or rabbits. Decapitate the rat (or rabbits) fifteen seconds after injection. Solubilize a sample of the injection solution and the hemisphere ipsilateral to the injection, in duplicate, in 1.5 ml soluene-350 at 50°C for 2 hours before double-isotope liquid model system. Use a similar protocol for rabbit.

### Cerebral spinal fluid (CSF) collection: based on Endocrinology 133 (6):2299-2301, 1983

Collect samples of CSF from the cisterna magna. Anesthetize a few animals (rats and rabbits) and place in a Kopf stereotaxic apparatus with the ear bars raised approximately 15 cm above the surface of the table. Place the incisor bar into lowest position so that the animal's head is maximally ventroflexed. Mount a 30-gauge needle connected to PE-10 tubing horizontally on the electrode carrier and direct the needle at the midline of the neck. Determine the dorsal ventral location of the cisterna magna by lowering the needle 6.3 to 6.8 cm (depending upon the size and strain of the animal) from the occipital crest. Advance the needle slowly through the incision in the skin and through the dorsal neck muscles while a slight suction is applied via a 1.0 ml syringe attached to the distal end of the tubing. When the needle penetrates the atlanto-occipital membrane and gains access to the cisterna magna, CSF flow will be initiated. Disconnect the syringe and collect the sample in a micropipette via gravity drainage. Collect a sample of from 50 to 200 µl in 30 minute period. At the completion of the CSF sampling procedure, take a 1.0 ml blood sample from the tail capillary plexus. The animal is killed following the procedure. The experiments on the transcytosis of the blood brain barrier and the determination of final location of recombinant molecules requires only a few animals (4-6).

## Determination of serum half-life

Inject $^{35}$S-labeled chimeric antibodies intravenously through the tail vein (5 mice/protein). Bleed the mice periodically from the retro-orbital sinus with heparinized 50 to 100 microliter capillary pipet. After the procedure, kill the mice and assay for the presence of $^{35}$S-labeled chimeric antibodies.

## Isolation of endothelial cells

Mince and homogenize cerebral cortices from anesthetized one-month-old Sprague-Dawley rats (2-3 rats) or rabbits (1 rabbit) after decapitation to isolate endothelial cells.

### Determination of final localization

Inject the purified chimeric fusion protein into the common carotid artery of the subject, i.e. rats or rabbits, as described in the section on carotid artery injection techniques. Decapitate subjects. Examine brain and organ tissue specimen for the presence of the chimeric fusion protein.

### Anesthesia

To relieve pain/discomfort, administer: Ketamine (45 mg/kg) and xylazine (88 mg/kg) to rabbits. Administer Ketamine (87 mg/kg) and xylazine (13 mg/kg) to rats. Check the heartbeat and respiratory rate neurological reflexes, and the color of mucous membrane to confirm a successful anesthesia.

### A successful euthanasia

To perform euthanasia, use carbon dioxide for rats and sodium pentobarbital (100 mg/kg) for rabbits. Kill mice by cervical dislocation, after ether anesthesia. To confirm a successful euthanasia, monitor the heartbeat.

### EXAMPLE 1

In creating antibody molecules with improved functional properties, the constant region of the antibody was replaced with a growth factor. The VH, CH1, hinge and first amino acid of CH2 from a chimeric mouse/human $IgG_3$ anti-dansyl antibody was joined to a cDNA encoding rat Insulin-like Growth Factor 1 (IGF1) immediately 3' to the leader sequence of IGF1. The chimeric heavy chain was introduced along with an anti-dansyl specific chimeric $\kappa$ light chain into the immunoglobulin non-producing myeloma P3X63Ag8.653. The immunoglobulin/non-immunoglobulin $IgG_3$-IGF1 chimeric protein was efficiently produced and secreted (up to 30 $\mu g/10^6$ cells/24 hours). The $IgG_3$-IGF1 chimeric proteins retain their specificity to the antigen dansyl and bind to the IGF1 receptors of human lymphoblast IM-9, albeit with reduced affinity. The chimeric proteins elicited some of the same biologic effects (increased glucose and amino acid uptake) in human epidermoid carcinoma KB cells as human IGF1, but with reduced specific activity.

The reduced affinity and biologic activity may result from (1) the presence of the unprocessed IGF1 moiety, (2) the large size of the $IgG_3$-IGF1 chimeric protein (160 KDa) compared to IGF1 (7 KDa) and (3) three amino acid substitutions in rat IGF1 compared to human IGF1 which may lead to decreased affinity for the human IGF1 receptor. Although the chimeric proteins were less effective on a molar basis than intact IGF2, they still exhibited the proper binding specificity and the ability to elicit the biologic effects associated with IGF1; thus, demonstrating a new family of immunotherapeutic molecules targeted to growth factor receptors.

### Construction and Characterization of the $IgG_3$-IGF1 Chimeric Protein:

By site directed mutagenesis a unique restriction enzyme site (Pvu II) was generated at the 5' site of the CH2 domain of the mouse/human $IgG_3$ heavy chain gene (72) and at the 3' of the leader sequence of rat IGF1 cDNA. IGF1 and $IgG_3$ were joined using the Pvu II sites. The chimeric $IgG_3$-IGF1 heavy chain gene and the chimeric $\kappa$ light chain gene were simultaneously transfected into P3X63Ag8.653 by protoplast fusion (45, 58). Then transfected cells were selected with G418 (GIBCO) at 1.0 mg/ml and screened by Enzyme-linked Immunosorbent Assay (ELISA) for transfectomas producing the chimeric protein (58). The $IgG_3$-IGF1 chimeric proteins biosynthetically labeled with $^{35}$S-Methionine (Amersham, Arlington Heights, IL) were analyzed by Sodium Dodecylsulfate-Polyacrylamide Gel Electrophoresis (SDS-PAGE) with/without 2-mercaptoethanol (Kodak, Rochester) NY). The $IgG_3$-IGF1 chimeric proteins were purified by affinity column as previously described (40). The purity of the $IgG_3$-IGF1 chimeric proteins were determined by silver staining gel (41) and Fast Performance Liquid Chromatography (FPLC, Pharmacia, Piscataway, NJ).

### Receptor Binding Assays:

IM-9 cells were washed twice with 100 mM HEPES buffer containing 120 mM NaCl, 5 mM KCl, 1.2 mM $MgSO_4$, 15mM Glucose, and 1% BSA, pH 7.5 (HEPES-BSA buffer), resuspended in HEPES-BSA buffer, and incubated for 1 hr at room temperature (36). Cells were counted and resuspended at a concentration of 4 - 6 x $10^7$ cells/ml. A 50 $\mu l$ aliquot of this cell suspension (2 - 3 x 106 cells) was removed and incubated with 50 $\mu l$ of human IGF1 purchased from Amgen (Thousand Oaks, CA), $IgG_3$-IGF1, $IgG_3$ or buffer and 100 $\mu l$ buffer containing 1 $\mu l$ of $^{125}$I-IGF1 (Amersham; 2.5 $\mu Ci$ $^{125}$I-IGF1 in 2 ml Phosphate Buffered Saline) rotating at 15°C for 2 hrs. All samples were duplicated. The cells were pelleted by 10 see centrifugation in a microcentrifuge. 100 µl of the supernatant (total 200 µl) was saved for determination of unbound counts (S) and the pellets (P) were washed with 0.5 ml ice-cold PBS and counted in a gamma-counter (Beckman Gamma 5500, Fullerton, CA). The bound counts were normalized by dividing by the total counts (2S + P). The percent inhibitions were calculated as:

$$[1\text{-}P/(2S + P)/C] \times 100\%$$

where C is the counts bound in the absence of a competitor.

## Determination of $\alpha$ - [1-$^{14}$C] - Aminoisobutyric Acid (AIB) and 2-Deoxy-D-[1-$^{14}$C] Glucose (2-DG) Uptake:

KB cells (5 x 10$^5$ cells/ml) were grown to confluence in a 24 well plate (FALCON, Lincoln Park, NJ) and washed three times with IMDM. After incubation with IMDM without serum overnight at 37°C, cells were washed with HEPES-BSA buffer and 100 µl same buffer added. HEPES-BSA buffer without glucose was used for 2-DG uptake. A 100 µl aliquot of various concentrations of the test samples (IGF1, IgG$_3$-IGF1, or IgG$_3$) was added into each well and incubated at 37°C for 6 hrs. After incubation, 24 µM $^{14}$C-AIB (Dupont, Boston, MA) or 15 µM $^{14}$C-DG (Amersham) was added into each well (2). The plates were rapidly washed with ice-cold PBS after a 15 min incubation at 37°C and cells lysed with 300 µl 1 N NaOH. Aliquots were counted for $^{14}$C and normalized to the amount of total protein determined by the BCA protocol (Pierce Chemical Co., Rockford, IL).

## Results

## Construction of a hybrid gene between human IgG$_3$ and rat insulin-like factor 1 (IGF1)

Experiments were undertaken to produce an antibody combining site joined to a growth factor so that the resulting molecule would retain its ability to bind to both antigen and the growth factor receptor. For the initial construction, rat insulin-like growth factor 1 (IGF1) was chosen as the growth factor. Mature IGF1 (a 70 amino acid protein) is formed from the IGF1 precursor (a 130 amino acid protein) through proteolytic processing of both its leader peptide and its carboxy terminus. The amino acid at which the leader peptide is cleaved is a convenient site for joining to the Ig molecule. Preferably, the Ig molecule is human IgG$_3$ which has an extended hinge region of 62 amino acids. Further, IGF1 was placed distal to the hinge region of human IgG3 thus producing a spacing which facilitates simultaneous antigen and receptor binding.

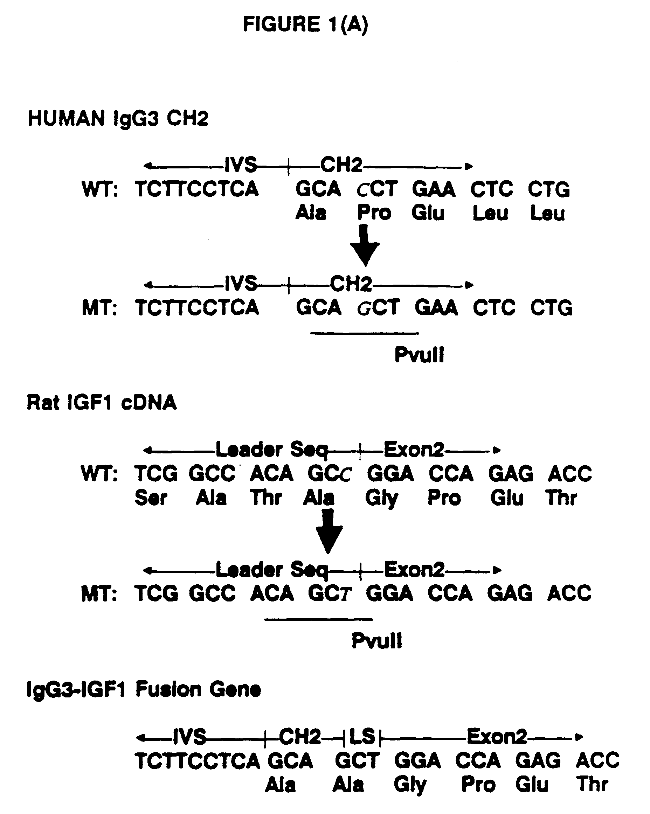

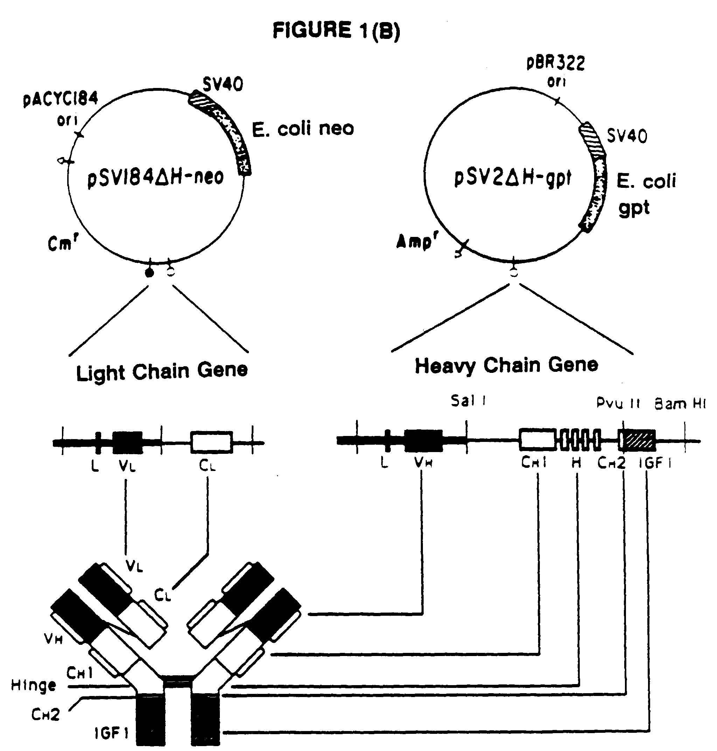

To facilitate construction of fused genes, a unique restriction site (PvuII) was generated by site directed mutagenesis at the 5' end of the leader sequence of the rat IGF1 cDNA (**Figure 1A**). These mutations were confirmed by sequencing. The human IgG$_3$ gene which contains C$_H$1, hinge and 4 basepairs of C$_H$2 exon was joined to the IGF1 gene which in turn contains 2 basepairs of leader sequence, exon 2, exon 3, and exon 5. A chimeric constant region gene was constructed with a human IgG$^3$/IGF-1 chimeric constant region gene joined to a mouse anti-dansyl (DNS) variable region gene cloned into a transfection vector pSV2 ΔHgpt (**Figure 1B**). The mouse/human κ light chain specific for dansyl is contained in pSV184 ΔH neo.

The strategy for the construction of IgG$_3$-IGF1 fusion gene was as follows (**Figure 1A**). The fourth base (C) of the C$_H$2 domain in human IgG3 was mutated to G by site-directed mutagenesis, resulting in the introduction of an unique PvuII restriction enzyme site. In addition, a unique PvuII site was introduced in IGF1 by changing the last base (C) of the leader sequence (Leader Seq. or LS) of rat IGF1 cDNA to T (thymidine). Human IgG$_3$ and rat IGF1 cDNA digested with PvuII were ligated, resulting in an in-frame IgG$_3$-IGF1 fusion gene without any significant amino acid substitutions.

**Figure 1B** is a schematic representation of the transfection vectors, the genetically engineered IgG$_3$-IGF1 fusion gene, and a proposed chimeric antibody. The mouse-human κ light chain gene is cloned into pSV184 ΔH neo, which is derived from the pACYC184, and contains the pACYC origin of replication, a chloramphenicol resistant gene (CM$^r$) for selection in E. coli and the neo gene (the dotted box) with the SV40 early region promoter (the shaded box) for selection in eucaryotic cells. The mouse-human IgG$_3$/rat IGF 1 heavy chain genes is cloned into pSV2 ΔH gpt gene (the dotted box). Boxes in the chimeric genes represent exons. The thick black solid line and boxes represent DNA of mouse origin while the thin solid and open boxes represent human DNA segments. The shaded box in the heavy chain genes represents the rat IGF1 cDNA. The sites of cleavage by restriction endonucleases open triangle), BamHI (open circle) and Hind III (closed circle) are shown. The mouse-human IgG$_3$/rat IGF1 chimeric protein produced by expression of both transfection vectors is shown at the bottom of the panel B. The black region of the chimeric molecule represents the mouse variable region domains specific for the hapten dansyl, the open regions represent the constant domains of human κ and IgG$_3$, and the shaded region represents the rat IGF1.

### Transfection of the chimeric IgG$_3$-IGF1 gene

These heavy and light chain vectors were transformed into E. coli HB101, and the bacteria containing both vectors were used to transfect a non-producing myeloma cell line (P3X63 Ag8.653) by protoplast fusion. Alternatively, transfection may be effected by electroporation or the standard calcium phosphate precipitation method.

Stable transfectant cells (transfectomas) were selected using G418 and the supernatants of stable transfectomas were tested for the presence of an antibody protein by an Enzyme Linked Immunosorbent Assay (ELISA) using anti-human $\kappa$ chain antibody. Seventy-seven stable transfectomas secreting complete antibody molecules were identified and recovered for further characterization.

The frequency of the desired transfectomas was $1.54 \times 10^6$ recipient myeloma cells. These transfectomas secreted 0.5-30 µg of chimeric molecule $10^6$ cells/24 hours. The production level of the immunoglobulin/non-immunoglobulin chimeric molecules is not different from that of wild type chimeric antibodies.

### Characterization of the IgG$_3$-IGF1 chimeric protein

The secreted IgG$_3$-IGF1 chimeric proteins were biosynthetically labeled with $^{35}$S-methionine and the labeled proteins were purified by immunoprecipitation using dansyl-BSA (dansyl; 5-dimethylamino naphthalene-1-sulfonyl chloride) conjugated Sepharose beads or a rabbit anti-human IgG Fab antiserum and IgG Sorb. Reactivity of the fusion proteins with dansyl-BSA demonstrated that they retained their ability to react with their specific antigen. SDS-PAGE analysis (**Figures 2A and 2B**) demonstrated that the stable transfectomas produce novel chimeric molecules. As expected, the size of these chimeric molecules is smaller than that of a normal IgG$_3$ antibody. However, the secreted chimeric heavy chain appeared heterogeneous in size because the recipient cells partially process the IGF1 precursor to mature IGF1. The incomplete proteolytic processing at the carboxy terminal of IgG$_3$-IGF1 heavy chain results in two distinctive heavy chains; a processed and an unprocessed heavy chain (**Figure 2B**).

When the chimeric molecules assemble to form the novel H$_2$L$_2$ antibody molecule, assembly of two unprocessed heavy chains results in the highest molecular weight chimeric molecule (top band in **Figure 2A**) and assembly of two processed heavy chains results in the lowest molecular weight chimeric molecule (bottom band in **Figure 2A**). Assembly between a processed and an unprocessed heavy chain results in intermediate sized chimeric molecule (middle band in **Figure 2A**). The IgG$_3$-IGF1 chimeric proteins were recognized by anti-IGF1 antisera, demonstrating that the IGF1 in the fusion protein assumes a native configuration.

**Figures 2A and 2B** represent SDS-PAGE analysis of IgG$_3$-IGF1 chimeric protein secreted by transfectomas. The secreted IgG$_3$-IGF1 chimeric protein biosynthetically labeled with $^{35}$S-methionine was analyzed under non-reducing (panel A) and reducing (panel B) conditions. The labeled chimeric protein was precipitated with either dansyl-Sepharose (DNS-Sepharose: left in panel A) or anti-human IgG Fab antisera/Staph A/IgG Sorb (a HUG/Fab-IgG-Sorb in panel A). The secreted IgG$_3$ chimeric antibody which is the basic structure of IgG$_3$-IGF1 chimeric protein is used as control. Under non-reducing conditions (Panel A) the three bands represent the heterogeneous assembly of the processed and unprocessed chimeric protein; under reducing conditions the processed and unprocessed chimeric protein are seen (Panel B).

IgG$_3$-IGF1 chimeric proteins were purified from culture supernatants using an affinity column with 2-dimethylamino naphthalene-5-sulfonyl chloride (MW 269; dansyl isomer) coupled to AH-Sepharose 4B. Bound protein was eluted with N-5-carboxypentyl-2-dimethylamino naphthalene-5-sulfonamide (MW 364; dansyl isomer) and free hapten removed by extensive dialysis. The concentrated purified proteins were tetrameric and heterogeneous in size as expected. No other protein was observed in silver stained gel (**Figure 3A**). When purified chimeric proteins were fractionated by Superose-12 chromatography, the three heterogeneous chimeric proteins were eluted in a broad peak between IgG1 chimeric antibody (murine variable region - human IgG1 constant region; M. W. 146 KDa) and IgG$_3$ chimeric antibody (murine variable region - human IgG$_3$ constant region; M.W. 170 KDa (**Figure 3B**). The approximate molecular weight of the (IgG$_3$-IGF1)$_2$L$_2$ chimeric protein is 160 KDa.

**Figure 3A** illustrates purified IgG3-IGF1 fusion protein fractionated by SDS-PAGE under non-reducing conditions and visualized using silver staining. **Figure 3b** is a chromatogram of the purified IgG$_3$-IGF1 chimeric protein fractionated using FPLC (Superose-12 column, flow rate:0.25 ml/min). The elution time of IgG$_3$-IGF1 chimeric protein is 42 min. The elution of time of mouse-human IgG$_3$ chimeric antibody (170 KDa) is 40 min and that of IgG1 chimeric antibody (146 KDa) is 45 min as indicated by arrows.

### Binding of the IgG$_3$-IGF1 Chimeric Protein to the IGF1 Receptor

A critical attribute of the fusion protein is whether it retains its ability to bind to the IGF1 receptor. To assess this, unlabeled recombinant human IGF1, wild type chimeric IgG$_3$ and the IgG$_3$-IGF1 chimeric protein were used to inhibit the binding of $^{125}$I-IGF1 to human lymphoblast IM-9 cells (20). Both IGF1 and IgG$_3$-IGF1 inhibited the binding of $^{125}$I-

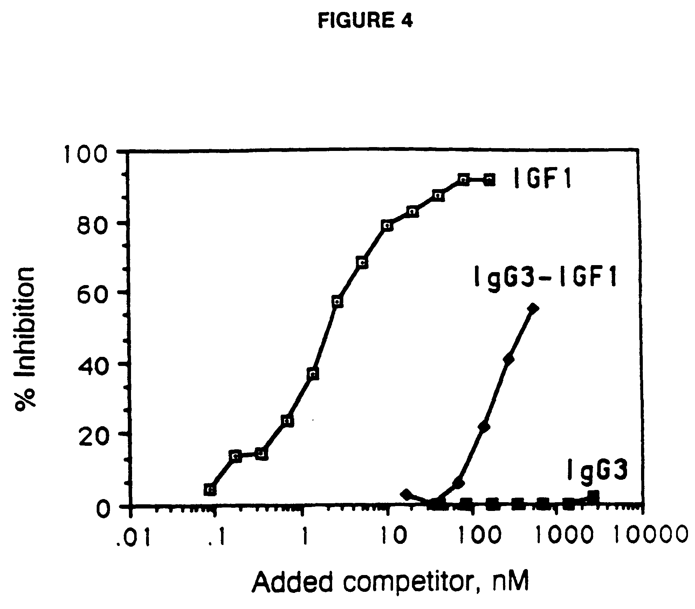

IGF1 in a dose dependent manner (**Figure 4**). The 50% inhibition of $^{125}$I-IGF1 binding occurred at recombinant IGF1 concentration of 2.25 x 10$^{-9}$ M and at a IgG$_3$-IGF1 chimeric protein concentration of 3.15 x 10$^{-7}$ M. Therefore, the IgG$_3$-IGF1 chimeric protein was 0.7% as effective as recombinant human IGF1 in inhibiting $^{125}$I-IGF1 binding. However, the wild type chimeric IgG$_3$ did not show any inhibition of $^{125}$I-IGF1 binding, even at a concentration as high as 2.7 x 10$^{-6}$ M. Therefore, the competition by the chimeric protein was a consequence of the presence of the IGF1 moiety.

**Figure 4** is a graph showing competitive inhibition of binding of $^{125}$I-IGF1 to IM-9 lymphocytes. Approximately 3x10$^{-6}$ IM-9 cells were incubated at 15°C with a constant amount of $^{125}$I-IGF1 and the indicated concentration of unlabeled competitors (recombinant IGF1, IgG3-IGF1 chimeric protein and IgG$_3$ chimeric antibody). After 2 hours of incubation, the amount of receptor-bound radioactivity was determined. Values are expressed as the relative inhibition of binding compared to using only labeled tracer $^{125}$I-IGF1. Results shown for each curve are the mean of duplicate experiments.

The IgG$_3$-IGF1 chimeric proteins have been tested for binding to two different primary cultures of human brain endothelial cells; one at an early passage ($\approx$13) and one at a later passage ($\approx$22). Comparable levels of binding with both of these cell lines have been observed (data now shown). The IgG$_3$-IGF1 chimeric proteins bound to both these cell lines in a specific manner, but the control antibody, chimeric IgG$_3$, does not bind to these cells.

## IgG$_3$-IGF1 Chimeric Protein Stimulation of Hexose and Amino Acid Uptake

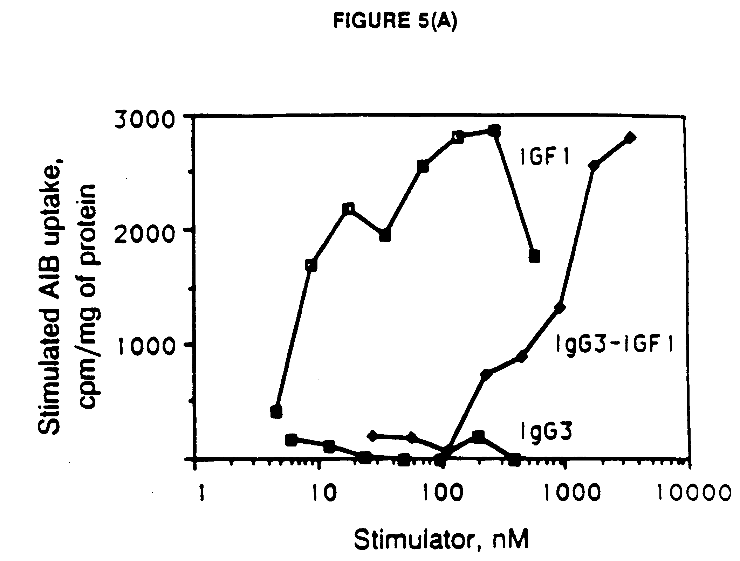

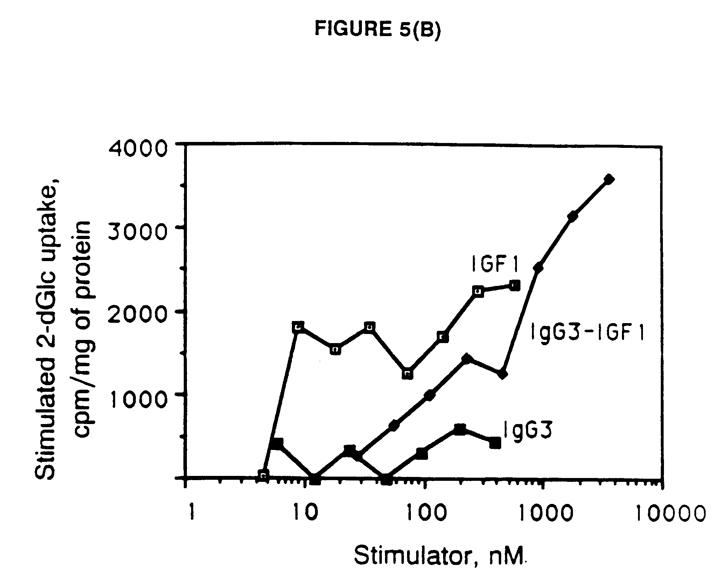

In human epidermoid carcinoma KB cells, fluid-phase endocytosis and exocytosis are stimulated by growth hormones (Insulin, Insulin-like growth factor 1, and epidermal growth factor); these cells possess 7.5 x 10$^4$/cell Type I IGF receptors (13). Therefore, the ability of IgG$_3$-IGF1 chimeric protein to stimulate 2-Deoxy-D-Glucose (2-DG) and $\alpha$-Aminoisobutyric acid (AIB) uptake (11) was investigated and compared to IGF1 and IgG$_3$. The dose-response relationships of IgG$_3$-IGF1 chimeric protein stimulation of 2-DG and AIB uptake in K cells are shown in **Figure 5**. Based on half-maximal effective concentration, the relative potencies of IGF1 and IgG$_3$-IGF1 were 200:1 for AIB uptake and 25 - 100:1 for 2-DG uptake. IgG$_3$ alone did not affect 2-DG and AIB uptake by KB cells. Therefore, the chimeric proteins exert the expected biological effects, but are less potent than the human IGF1 standard.

**Figure 5** shows the relationship between IGF1 and IgG3-IGF1 and their stimulatory effects on AIB and 2-DG uptakes in $\kappa$B cells. AIB (panel A) and 2-DG (panel B) uptakes were determined in the presence of various concentrations of IGF1, IgG3-IGF1, and IgG3 as control.

## Discussion

Many problems have been encountered when investigators have attempted to use monoclonal antibodies as immunotherapeutic agents. Among the problems are efficiently targeting the antibodies to tumor cells while leaving normal cells untouched and gaining access to restricted locations in the body, e.g. the brain, lymph, liver, kidney, lung, adrenal, skin, and pancreas. Thus, the subject chimeric monoclonal antibody was constructed to overcome these problems. The chimeric protein was cotransfered together with a dansyl specific chimeric light chain and was efficiently produced and secreted (up to 30 μg/10$^6$ cells/24 hrs) IgG$_3$-IGF1 chimeric proteins in a recipient non-producing murine myeloma. Thus high level expression of these recombinant molecules are feasible.

In this example, the constant region of the antibody was replaced with a growth factor e.g. insulin, IGF1, IGF2 and transferrin. The VH, CH1, hinge and first amino acid of CH2 from a chimeric mouse/human IgG$_3$ anti-dansyl antibody was joined to a cDNA encoding rat Insulin-like Growth Factor 1 (IGF1) immediately 3' to the leader sequence of IGF1. The chimeric heavy chain was introduced along with an anti-dansyl specific chimeric $\kappa$ light chain into the immunoglobulin non-producing myeloma P3X63Ag8.653. The immunoglobulin/non-immunoglobulin IgG$_3$-IGF1 chimeric protein was efficiently produced and secreted (up to 30 μg/10$^6$ cells/24 hours). The IgG$_3$-IGF1 chimeric proteins retain their specificity to the antigen dansyl and bind to the IGF1 receptors of human lymphoblast IM-9, albeit with reduced affinity. The chimeric proteins elicited some of the same biologic effects (increased glucose and amino acid uptake) in human epidermoid carcinoma KB cells as human IGF1, but with reduced specific activity.

The reduced affinity and biologic activity may result from (1) the presence of the unprocessed IGF1 moiety, (2) the large size of the IgG$_3$-IGF1 chimeric protein (160 KDa) compared to IGF1 (7 KDa) and (3) three amino acid substitutions in rat IGF1 compared to human IGF1 which may lead to decreased affinity for the human IGF1 receptor. Although the chimeric proteins were less effective on a molar basis than intact IGF2, they still exhibited the proper binding specificity and the ability to elicit the biologic effects associated with IGF1; thus, demonstrating a new family of immunotherapeutic molecules targeted to growth factor receptors.

The novel IgG$_3$-IGF1 chimeric proteins retain their specificity for the antigen dansyl, the ability to bind to the IGF1 receptor with reduced affinity and the ability to exert some of the biologic effects of receptor binding. The reduced affinity for receptor and biologic activity may result from (1) the presence of the unprocessed IGF1 moiety thus resulting in a heterogenous population of IgG$_3$-IGF1 chimeric protein, (2) the large size of the IgG$_3$-IGF1 chimeric protein (160 KDa) compared to IGF1 (7 KDa) thus leading to decreased accessibility to the IGF1 receptor and decreased binding affinity; and (3) three amino acids substitutions in rat IGF1 compared to human IGF1 may lead to lower affinity for the human receptor.

In determining whether reduced affinity for receptor and biologic activity results from the presence of the unprocessed IGF1 moiety a second generation of proteins in which a stop codon is introduced at the end of the mature protein is engineered. Also, creating a size-reduced size Fab-IGF1 chimeric facilitates the determination of whether reduced affinity for receptor and biologic activity results from the large size of the $IgG_3$-IGF1 chimeric protein (160 KDa) compared to IGF1 (7 KDa). Moreover, altering three amino acids in rat IGF1 as compared to human IGF1 by site-directed mutagenesis additionally facilitates the determination of whether the reduced affinity is from the three amino acid substitutions.

The flexibility and accessibility of IGF1 also play an important role in the chimeric protein. The IGF1 moiety was disposed close to the carboxy terminal of the extended hinge of human $IgG_3$. $IgG_3$ is the most flexible human IgG (61). Accordingly, this flexibility optimize the ability to simultaneously bind the IGF1 receptor and antigen. However, the position of IGF1 immediately carboxy terminal to the hinge brought the IGF1 molecules into contact with each other and interfered with their binding. The molecules are improved by placing the IGF1 on a β-strand in $C_H2$ more distal to the hinge (9). Since the $C_H2$ domains do not normally contact each other this places the two IGF1 moieties at some distance from each other and thereby improves their binding efficiency.

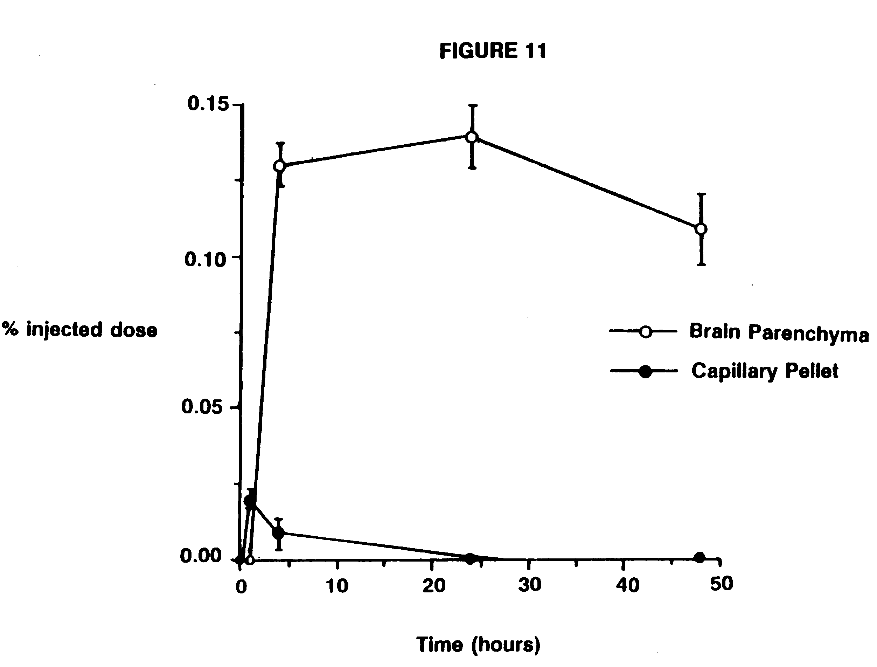

The BBB in a normal brain effectively restricts transport between blood and the central nervous system of certain molecules, especially those which are water soluble, charged and larger than several hundred daltons (56). The $IgG_3$-IGF1 chimeric proteins bind specifically to the IGF1 receptor and to human brain endothelial cells (**Figure 11**). **Figure 11** is a line graph wherein the X axis is the time in hours after injection and the Y axis is the % injected dose of the IgG3-IGF1 chimeric construct. **Figure 11** shows that after injection in a rat animal model the IgG3-IGF1 chimeric construct was found and persisted in brain parenchyma cells compared to the capillary pellet. Accordingly, this chimeric construct crossed the BBB.

## EXAMPLE 2

### A. METHODS

### Construction of chimeric fusion genes

Antibody molecules with a combining specificity, a growth factor to target to receptor-bearing cells, and immune effector functions contained in one molecule and antibody molecules with bifunctional combining specificities belong to a family of multi-functional chimeric antibodies which are directed to targeting therapy to malignant cells.

A suitable protocol to create a family of multi-functional chimeric antibodies is:

a. Create antibodies having a variable region from dansyl (DNS) specific hybridomas which have the advantage of high binding affinity. Alternatively, use anti-dextran variable (V) regions or domains having anti-tumor specificities.

b. Incorporate DNS-Cephalin into the cell membrane of eukaryotic cells so as to simulate cell surface markers (8).

c. Use the human constant regions of $IgG_1$ and $IgG_3$, both of which bind the high affinity Fc receptor and activate complement.

d. Use IGF1, IGF2, insulin and transferrin as replacements for at least a portion of the $F_c$ region of the heavy chain (see Example 1).

e. Substitute IGF1 and insulin into the Fc of anti-DNS or anti-tumor chimeric $IgG_3$ to produce:

$$V_{\text{DNS/anti-tumor specificity}} - C_H1 - \text{hinge} - \text{IGF}$$

$$V_{\text{DNS/anti-tumor specificity}} - C_H1 - \text{hinge} - \text{insulin}$$

Preferably, use $IgG_3$ for the immunoglobulin sequences because of its extended hinge region allows the molecule greater flexibility and facilitates binding of the growth factor to its receptor.

Molecules capable of recruiting human effector functions are:

$$V_{\text{DNS/anti-tumor specificity}} - C_H1 \text{ hinge} - C_H2 - \text{ligand}$$

$$V_{DNS/anti-tumor\ specificity} - C_H1 - hinge - C_H2\ C_H3 - ligand$$

$C_H2$ contains the binding site for the high affinity Fc receptor and for Clq.

## Gene Transfection

The modified transfection vectors developed by Oi and Morrison (45) were used. Insert the reconstructed chimeric Ig/receptor ligand gene into the transfection vector (pSV2ΔHgpt), a derivative of pSVgpt. pSV2ΔHgpt contains the pBR322 origin of replication and has an ampicillin resistant gene for selection in E. coli and the gpt gene for selection in eukaryotic cells.

Clone the mouse/human light chimeric gene into the light chain vector (pSV184ΔH neo), a derivative of the pACYC184 plasmid, which contains the pACYC origin of replication a chloramphenicol resistant gene for selection in E. coli, and the neo gene for selection in eukaryotic cells.

Transform these plasmids into E. coli. Select the desired bacteria with chloramphenicol and ampicillin and fuse selected bacteria with a nonproducing myeloma cell line by protoplast fusion (39,58). Alternatively, use electroporation or calcium phosphate precipitation to introduce DNA into myeloma cells (39, 58).

Isolate stable transfectomas using selectible drug markers. Screen the culture supernatant of stable transfectomas by ELISA using any of the antigen dansyl, anti-idiotypic antibody, human κ chain or anti-ligand antibodies to identify clones secreting high levels of the chimeric antibodies.

## Analysis of Transfectomas.

Expression of chimeric genes require efficient transcription, proper RNA processing, and exportation from the nucleus, so that the mRNA is stable and translated, and that the protein product is property processed through the cell and is secreted without being degraded in the cytoplasm or in the medium.

To study expression of transfected genes analyze the total RNA from stable transfectomas on Northern blots using probes specific for various regions of the transfected gene (mouse $V_H$ probe, human $IgG_3$ $C_H1$ probe, and ligand probe).

Further, to analyze the cytoplasmic and secreted chimeric proteins label selected transfectomas with $^{35}$S--methionine, after which purify the labeled proteins by immunoprecipitation using dansyl conjugated beads, anti-idiotypic antibody conjugated beads or anti-ligand antibody conjugated beads.

Analyze these precipitated proteins by SDS-polyacrylamide gel electrophoresis under reducing and non-reducing conditions. Isolate large quantities of protein from either culture supernatants or from ascitic fluid of tumor bearing mice. Determine whether transfectomas remain tumorigenic.

## Characterization of chimeric fusion molecules

a. Determination of Fc receptor binding. Use anti-dansyl chimeric fusion molecules in a hapten-enzyme binding assay (40). Briefly, this involves the following: incubating the chimeric fusion molecules at varying concentrations after allowing sufficient time for the antibody to bind to the Fc receptors with the human monocyte-like cell line U937, which bears high-affinity Fc receptors. Wash the cells. Incubate cells with dansyl conjugated with B-galactosidase. Allow the antigen-antibody reaction to reach equilibrium and remove the unbound antigen by centrifuging the cells through a sucrose pad. Spectrophotometrically, determine amount of bound IgG-β-Gal after incubating the cells with the substrate o-nitrophenyl galactoside and measure the absorbance at 420 nm. Using Scatchard analysis determine the apparent association of the antibody for the Fc receptor and the number of receptors per cell. Iodinate chimeric proteins without a combining specificity for dansyl and either determine direct binding or assay ability of recombinant protein to inhibit binding.

b. Complement (C') activation. Assay complement activation according to Oi, et al. (44), using dansyl coupled to BSA as antigen (Ag) for anti-dansyl chimerics or appropriate antigen for chimerics of different specificity. Mix titered guinea pig complement, a fixed amount of chimeric fusion molecules (Ab) and serially diluted dansyl-BSA in U bottomed microtiter wells. Incubate mixture. After incubation, add hemolysin-sensitized and $^{51}$Cr-radiolabeled red blood cells to mixture, continue incubation, and pellet unlysed red blood cells. Collect supernatants and count radioactivity in a scintillation counter. Complement consumption is calculated as:

[1-cpm of (Ag+Ab+C')/cpm of (Ab+C')] x 100%.

c. Clg binding assay. Coat flexible microtiter plates with dansyl-BSA then incubate with saturating amounts of anti-dansyl chimeric fusion molecules. For antibodies with different specificities use appropriate antigen to coat the microtiter plate. Wash plates and allow several dilutions of $^{125}$I-labeled human Clq to bind the Ag-Ab complex. After washing, cut the wells out of the plates and counted in a gamma counter (62).

d. Sensitivity to proteases. Digest chimeric fusion molecules with a variety of proteases, e. g. papain, pronase, trypsin, or pepsin. Allow digestions to occur for varying lengths of time (from 4 hours up to 24 hours). Analyze the degree of digestion of the protease-treated chimeric fusion molecules using SDS-PAGE and determine the amount of color released by enzyme labeling by scanning the stained gel with a spectrophotometer.

e. Determination of serum half-life of chimeric fusion molecules. In order to understand how the immunoglobulin/non-immunoglobulin chimeric fusion molecules behave under in vivo conditions, compare the half-lives of the immunoglobulin/non-immunoglobulin molecules and the chimeric mouse-human immunoglobulin molecules to determine the role of the constant region in determining half-life (62).

Intravenously inject purified radioactive chimeric molecules through the mouse tail vein and periodically bleed the mice into heparinized capillary pipets. Using $^{35}$S-methionine biosynthetically label the proteins so that the labeling procedure will not alter the structure of the protein and thereby affect its metabolism. Collect blood and determine the radioactivity both before and after immunoprecipitation. Calculate the serum half-life of each chimeric molecule.

## Comparison of the Biological Function of Chimeric Fusion Molecules to Specific Antigen Associated Cell

To determine whether the molecules exert any biological effect, e.g. antibody-dependent cell-mediated cytotoxicity (ADCC) of target cells (57) couple the target cells, i.e. 3T3-L1, IM-9 and K-562, with DNS hapten using DNS-Cephalin (8). Label target cells with $^{51}$Cr chromate. Obtain human peripheral blood mononuclear cells from healthy donors and prepare human peripheral blood mononuclear cells by centrifugation on Ficoll-Hypaque (22). Add cells to tissue culture dishes coated with autologous serum and incubate for 90 min at 37°C in a humidified atmosphere of 8% $CO_2$-air. Pool nonadherent cells, i.e. lymphocytes, and harvest adherent cells, i.e. monocytes. Add lymphocyte and monocyte suspensions to target cells coupled/uncoupled with DNS in several ratios of effector:target cell. Centrifuge suspension. After centrifugation at 600 xg for 3 min, remove buffy coat and incubate it for 4 hrs at 37°C in humidified atmosphere of 8% $CO_2$-air. After centrifugation at 600 xg for 10 min remove and count an aliquot of supernatant from each sample. Calculate the percentage specific $^{51}$Cr release as:

Percent release = 100X[(test release) - (spontaneous release)]/[(total radioactivity) - [spontaneous release)].

These experiments indicate the relative effectiveness of multifunctional molecules in targeting cells for ADCC and the necessary modification for a more effective chimeric protein.

## Binding of Chimeric Fusion Molecules to Ligand Receptors

Since IGF1 insulin and transferrin receptors were identified on brain blood vessels both in vivo (64) and in vitro (16), isolated brain microvessels have been used as a model system to test for binding and internalization of those ligands (16, 17, 18, 49). However those receptors also occur on non-brain cells. In particular the cultured human lymphoblast cell line IM-9 expresses large numbers of IGF1 receptors (52). The adipose cell line 3T3-LI expresses large numbers of insulin receptors (50, 53, 54) and the human chronic myelogenous leukemia cell line K-562 expresses large numbers of transferrin receptors (63, 66).

Carry out initial binding studies (16) of chimeric fusion molecules onto receptors at 15°C with the cultured cell lines (IM-9, 3T3-LI, and K562) and purified radioactive chimeric molecules. Only a small amount of ligand bound to receptor is internalized at 15°C. To determine non-specific binding, prepare reactions identically except for the addition of a high concentration (100 µg/ml) of unlabeled ligands. Withdraw aliquots periodically, determine the radioactivity of the pellets, and assay the amount of protein in each pellet by the method of Lowry (34). Carry out a competitive displacement experiment with the purified chimeric molecules and the appropriate dilution of unlabeled ligands. Monitor ligand degradation by precipitation in 10% TCA. From all these experiments, analyze the specific binding of the ligand moiety of the chimeric fusion molecules to their receptors.

When necessary to isolate brain endothelial cells to study endothelial transcytosis, first produce microvessels and then separate the endothelial cells. Isolate brain microvessels from mouse, rat, and/or rabbit using mechanical homogenization (6, 48). These preparations do not always exclude trypan blue. These preparation are metabolically active and have been studied with respect to glucose, lactate, and fatty acid metabolism in vitro (3, 60). To culture endothelial cells (7, 21, 29) clean cortical tissue from brain of meninges and superficial blood vessels, mince cortical tissue from brain to small cubes, incubate cubes in proper medium containing 0.5% dispase for 3 hours at 37°C, and collect endothelial cells by centrifugation at 1,000 g for 1 minute. Suspend the pellets in medium containing 13% dextran. Separate microvessels from other brain tissue by centrifugation of the suspension at 5,800 g for 10 minutes. To remove the basement membrane and most pericytes treat the microvessel to a further 9-12 hour treatment with collagenase/dispase in medium. Pellet microvessels at 1,000 g for 20 minutes, suspend microvessels in medium, and keep suspension in liquid nitrogen until use. Filter some of the microvessel suspension through a 250 μM nylon mesh to remove non-digested material. Add proper medium. Remove the top layer of these cells and pellet the remaining cells. Resuspend the pellet and refilter it through 30 μM nylon mesh. Retain the endothelial cell in the mesh. Collect the endothelial cells in a plastic tube containing 40 ml of PBS 1.0 mM Ca and 5% BSA. Allow the sample to stand. Remove the top 10 ml and centrifuge the remaining suspension. Plate the pellet containing endothelial cells into gelatin coated tissue culture plates. Identify these primary cultured cells by morphology and their positive reaction for Factor VIII antigen.

## Processing of Bound Complexes

At 37°C most ligands bound to their receptor will be internalized. At 4°C very little internalization takes place. Assess the internalization (endocytosis) of the purified chimeric fusion molecules with an acid-wash technique (18, 27, 46, 49). Incubate the cultured cells with trace amounts of the purified radioactive chimeric fusion molecules at 37°C, withdraw aliquots, quickly count the pellets and resuspend it in ice-cold pH 3 barbital buffer. Acid-wash the pellets and then recount and assay it for the amount of protein. Acid washing removes the bound but not internalized proteins. Analyze both bound and internalized proteins by SDS-PAGE to determine whether degradation had taken place.

To determine the externalization (exocytosis) of the purified radioactive chimeric fusion molecules, reincubate the cells, after internalization at 37°C, in the presence of unlabeled ligands, to allow the surface-bound chimeric fusion molecules to be internalized or displaced from their receptor. Wash and rewash the cells and the resuspend them in fresh assay buffer, containing unlabelled ligands to prevent rebinding and incubate at 37°C. Withdraw aliquots periodically and count and assay pellets for amount of protein. Assay externalized protein by SDS-PAGE to determine whether the protein is intact.

For transcytosis studies involving rabbits, mice, and rats. Use experimental protocol similar to those which have been described (17). Inject the purified radioactive ($^{35}$S-methionine) chimeric molecules through the carotid artery, and decapitate animals after 15 seconds, sufficient time for a single passage through the brain (17). Rapidly remove the ipsilateral hemisphere, homogenize it through a needle, dissolve, and simultaneously count the ipsilateral hemisphere for $^{35}$S and [3]H. From these results, calculate the brain uptake index (BUI).

Further, procure the CSF from the subarachnoid space or the cisterna magna in anesthetized animals. Obtain blood from the same animals by cardiac puncture. Determine brain chimeric molecule levels by the method of Frank et al. (17). Determine the levels of chimeric fusion molecules in the CSF and the blood by radioimmunoassay using dansyl hapten or anti-idiotypic antibody. BUI and chimeric molecule levels in serum, brain, and CSF is indicative of whether chimeric molecules pass through the blood-brain barrier in vivo.

## Determination of the Chimeric Fusion Protein Distribution of Target Cell Lines with Isolated Subcellular Fractions

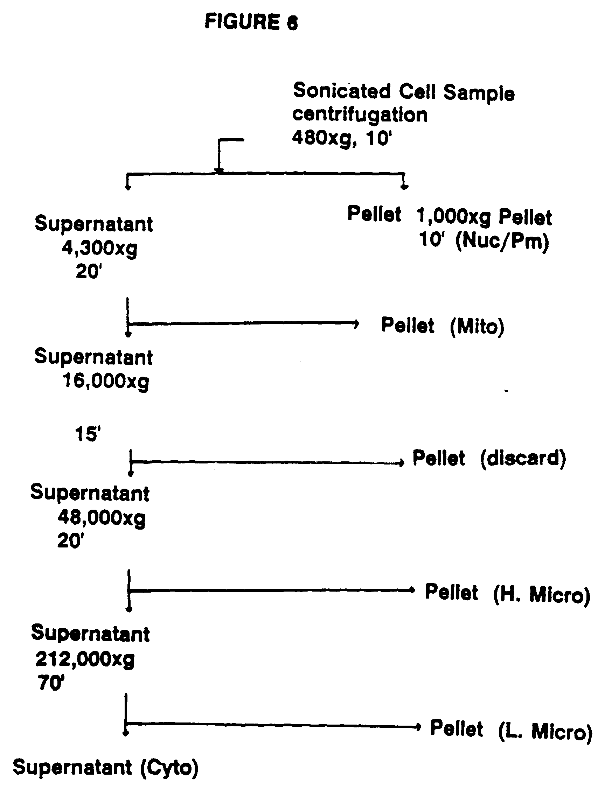

To examine the intracellular targeting site of chimeric fusion molecules, analyze several subcellular fractions. Prepare samples by sonication after internalization of $^{35}$S- or $^{125}$I-labeled chimeric fusion molecule into 3T3-LI, IM-9 and K-562 cells coupled with DNS. Perform differential centrifugation to obtain nuclear/plasma membrane (Nuc/Pm) fraction; Mitochondria (Mit) fraction, high density microsomes (H. Micro) fraction, low density microsomes (L. Micro) and the cytosolic (Cyto) fraction (10, 59) (**Figure 6**).

Count each fraction by TCA precipitation. Furthermore, analyze the chimeric fusion molecules by SDS-PAGE to determine the location of intracellular target sites and to determine whether any degradation has taken place. Analyze the purity of each fraction by determining the enzymes which were present. Use 5'-nucleotidase and adenylate cyclase as a plasma membrane marker, Rotenone-insensitive NADH-cytochrome c reductase and glucose-6-phosphate phosphatase as an endoplasmic reticulum marker in H. Micro, galactosyl transferase as a Golgi apparatus marker in L. Micro and citrate synthase as a mitochondrial marker.

An alternative approach to cellular fractionation is immunohistochemical analysis and electron microscopy in order to identify the subcellular localization of the chimeric fusion molecules (14).

## EXAMPLE 3

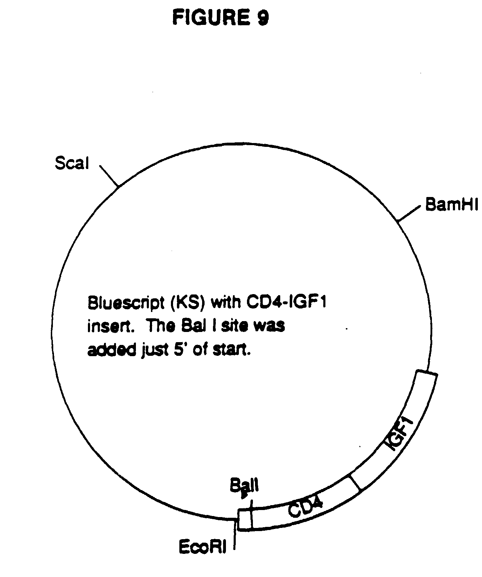

The first two domains of CD4 have been joined to IGF1 (**Figure 9**). The fusion protein was synthesized and secreted.

### Mutagenesis of CD4

Convenient sites were created in the CD4 cDNA to allow the two, NH2 terminal, extracellular domains of CD4 to be ligated into the proposed construct.

### 5' CD4

A Bal I site was created upstream of the translation start site.

```
                                              Met  Asn  Arg  Gly
        5'...TTC CTC CCT CGG CAA GGC CACA ATG AAC CGG GGA...3'
                                       *
        Primer 3'...GGA GCC GTA CCG GTGT TAC T...
                    Bal I
```

### 3' CD4

A Sca I site was created one base after the codon for Glutamine 114.

```
           Gln  Gln  Gly  Ser  Leu  Thr  Leu  Thr  Leu  Glu
        5'...CAG  GGG  CAG  AGC  CTG  ACC  CTG  ACC TTG  GAG
                             **    *
        Ser   Pro
        AGC   CCC...3'

        Primer 3'...CCC  GTC  ATG  AAC  TGG  GAC  TG...5'
                    Sca I
```

The resultant CD4 fragment will contain the 23 amino acids of the leader peptide and the first 114 amino acids of the mature protein. One additional base pair at the 3' ends of this fragment will combine with two bases 5' for the mature IGF1 to encode a threonine between glutamine 114 of CD4 and glycine 1 of IGF1. The first codon of the C-terminal propeptide of IGF1 has been replaced with a stop codon.

### Construction of CD4-IGF1

Following the mutagenesis in pBluescript of CD4 to create the 3' Sca I site, IGF1 was inserted into this plasmid between this Sca I site and a Bam HI site in the poly linker, thus removing all downstream regions of CD4 (**Figure 9**). The IGF1 insert contains the coding region from two base pairs before the first codon of the mature protein through a string of Adenines not present in the genomic gene, and linked to 600 bp of the 3' untranslated (UT) region of IgG3. This 3' region was included for the poly A signal it contains. Questions have arisen as to whether or not this region actually contains the poly A signal. Within the 3' UT region of IGF1 are the Poly A signal like sequences AATGAAA and AAGTAAA. These sequences seem to function as a Poly A signal in that the cDNA shows that the message was polyadenylated about 40 bases downstream. Also, the IgG3-IGF1 chimeric constructs contain only these signals and appear to be expressed normally.

The final construct encodes the 23 amino acid propeptide and first 114 residues of CD4, a threonine, and the entire 70 residues of mature IGF1.

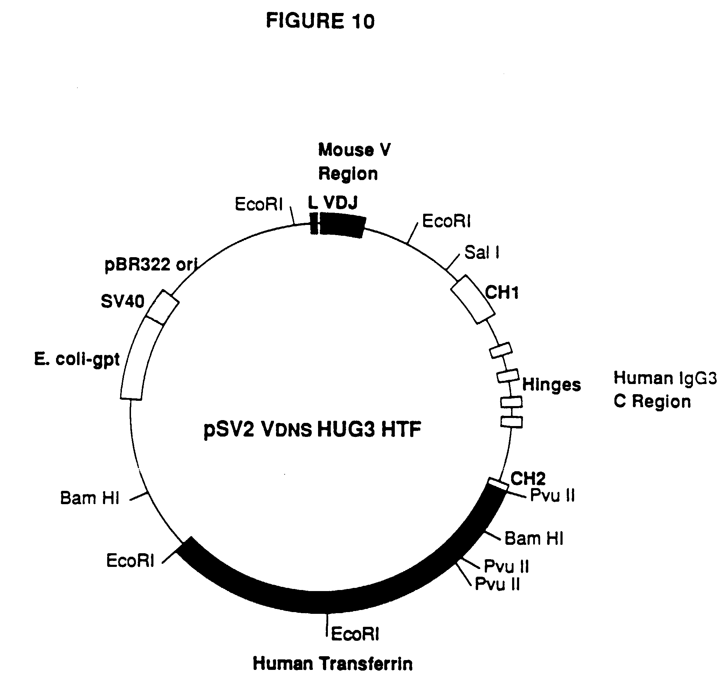

The construct described above has been inserted into the vector pAG4235 immediately downstream of an IgG3 promoter. This plasmid also contains a heavy chain enhancer as well as the pSV2-gpt selectable marker (**Figure 10**).

### Example 4

An antibody has been produced in which transferrin is joined to a mouse/human IgG3 chimeric anti-dansyl heavy chain immediately following the hinge region (**Figure 10**). This fusion protein assembles with the light chain and is secreted as an $H_2L_2$ molecule.

### Example 5

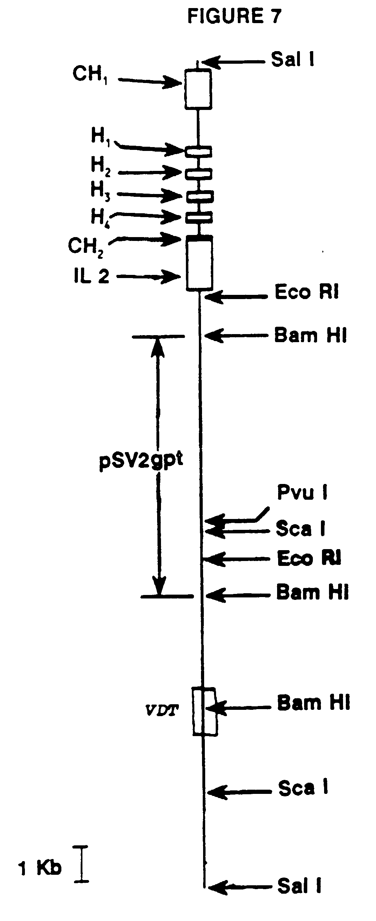

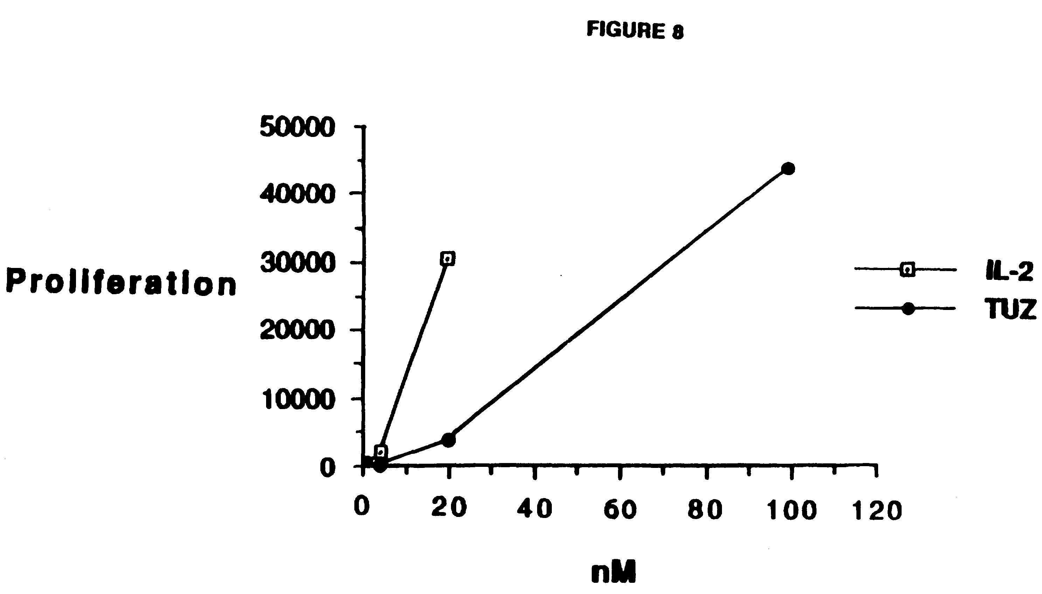

Human IL-2 has been cloned using PCR technology with IL-2 specific oligomers. Cloned IL-2 was joined to a mouse/human IgG chimeric anti-dansyl heavy chain immediately following the hinge region using the methods described herein (**Figure 7**). This fusion heavy chain assembles with light chain and is secreted as an $H_2L_2$ molecule. **Figure 8** shows that the IL-2 fusion protein, designated TU2 has activity. Culture supernatants from a murine myeloma cell line transfectant synthesizing this protein will support the growth of an IL-2 dependent T cell line. Culture supernatants from the un-transfected myeloma cell line or from the myeloma cell line transfected with a chimeric Ig lacking IL-2 failed to support growth.

### References

1. Beck, D.W., Vinters, H.V., Hart, M.N., and Cancilla, P.A. (1984) J. Neuropathol. Exp. Neurol. 43: 211-224.

2. Bequinot, F. et al. (1985) J. Biol. Chem. 260(29):15892.

3. Betz, L.A., and Goldstein, G.W. (1981) J. Physiol. (London) 312:365-376.

4. Bleil, J., and Bretsher, M.D. (1982) Eur. Molec. Biol. Orig. J. 1:351.

5. Boulianne, G.L., et al. (1984) Nature (London) 312:643-646.

6. Bowman, P.O., et al. (1981) In vitro, 17:353-362.

7. Bowman, P.O., et al. (1982) In Vitro 18:626-632.

8. Bruggemann, M., et al. (1987) J. Exp. Med. 166:1351-1361.

9. Burton, D. R. (1987) In: Molecular Genetics of Immunoglobulin (Calabi, F. and Neuberger, M. S., Eds.) Ch. 1 Elsevier Science Publishers, B. V.

10. Cotgreave, I.A. et al. (1988) J. Biochem Biophys. Meth. 16:247-254.

11. Dautry-Varsal, A. et al. (1983) Proc. Natl. Acad. Sci. USA 80:2258. USA 80:2258, 1983.

12. Duncan, A.R. et al. (1988) Nature, 332:363.

13. Duncan, et al. (1988) Nature 332:738.

14. Forti, G. et al. (1989) J. Steroid Biochem. 321: 135-144.

15. Fishman, J. B. et al. (1987) J. Neurosci. Res. 18:299.

16. Frank, H.J.L. et al. (1981) Diabetes 30:757-761.

17. Frank, H.J.L. et al. (1985) Diabetes 34:728-733.

18. Frank, H.J.L. et al. (1986) Diabetes 35:654-661.

19. Frank, H.J.L., et al. (1986) Ann. Intern. Med. 105:82-95.

20. Gatter, K.C. et al. (1983) J. Clin. Path. 36:539.

21. Gordon, P. et al. (1983) In Vitro, 19: 661-671.

22. Hale, G. et al. (1983) Blood 62:873.

23. Harding, C. et al. (1983) J. Cell Biol., 97:329.

24. Hopkins, C.R. et al. and Trowbridge, I.S. (1983) J. Cell Biol., 97:508.

25. Houghton, A.N., and Scheinberg, D.A. (1986) Seminars in Oncology, 13(2):165-172.

26. Jeffries, W.A. et al. (1984) Nature (London) 312:162.

27. Jialal., I. et al. (1984) Diabetes 33:784-522. 33:784-800.

28. Jones, P.T. et al. (1986) Nature, 321:522.

29. Kern, P.A. et al. (1983) J. Clin. Invest. 71:1822-1829.

30. Klausner, R.D. et al. (1983) Proc. Natl. Acad. Sci. USA 80:2263.

31. Kohler, G., and Milstein, C. (1975) Nature (London) 256:485-486.

32. Liu, A.Y. et al. (1987) Proc. Natl. Acad. Sci. USA, 84:3439.

33. LoBuglio, A. F., et al. (1989) Proc. Natl. Acad. Sci. USA 86:4220.

34. Lowry, O.H. et al. (1951) J. Biol. Chem. 183:265.

35. Mendelsohn, J. (1988) J. Prog. Allergy 45:147.

36. Misra, P. et al. (1986) J. Clin. Endocrin. and Metab. 63(6):1400.

37. Miyata, Y. et al. (1988) Exp. Cell Res. 178:73.

38. Morrison, S.L. (1984) Proc. Natl. Acad. Sci. U.S.A., 84:6851.

39. Morrison, S.L. et al. (1987) Ann. N.Y. Acad. Sci. 507:187.

40. Morrison, S.L. et al. (1988) Clin. Chem. 34:1668.

41. Morrisey, J.H. (1981) Anal. Biochem. 117:307.

42. Neuberger, et al. (1984) Nature (London), 312:604.

43. Neuberger, M.J. et al. (1985) Nature (London), 314:268-270.

44. Oi, V.T., et al. (1984) Nature 307:136-140.

45. Oi, V.T., and Morrison, S.L. (1986) BioTechniques 4:214.

46. Olefsky, J.M., and Kao, M. (1982) J. Biol. Chem. 257:8667-8673.

47. Itabor, E. et al. (1989) <u>Science</u>, <u>243</u>:51.

48. Pardridge, W.M. et al. (1985) <u>J</u>. <u>Neurochem</u>. <u>44</u>:1178-1184.

49. Pardridge, W.M. et al. (1985) <u>J</u>. <u>Neurochem</u>. <u>44</u>:1771-1778.

50. Reed, B.C. et al. (1977) <u>Natl. Acad. Sci. USA 74</u>:4876-4880.

51. Riechmann, L. et al. <u>Nature</u> (1988) <u>332</u>:323.

52. Rosenfeld, R.G., and Hintz, R.L. (1980) <u>Endocrin</u>. <u>107</u> (6):1841-1848.

53. Rubin, C.S. et al. (1978) <u>J</u>. <u>Biol. Chem. 253</u>:7570.

54. Salzman, A. et al. (1987) <u>Biochemistry 23</u>:6555-6565.

55. Sauvage, C.A. et al. (1987) <u>Cancer Res</u>. <u>47</u>:747.

56. Shapiro, W. R. and Shapiro, J. R. (1986) <u>Seminars in Oncology 13</u>(1):56.

57. Shaw, D.R. et al. (1988) <u>J. Biol. Response Modifiers, 7</u>:204.

58. Shin, S.-U., and Morrison, S.L. (1989) <u>Method in Enzym</u>., <u>178</u>:451

59. Simpson,I.A. et al. (1983) <u>Biochemica et Biophysica Acta 763</u>:393-407.

60. Spatz, M., et al. (1978) <u>J. Brain Res</u>., <u>151</u>:619.

61. Tan, L. K. et al. (1989) Proc. Natl. Acad Sci. USA, in press.

62. Tao, M.-H., and Morrison, S.L. Manuscript (1989) <u>J. Immunol.</u>, <u>143</u>:2595.

63. Tsavaler, L. Stein et al. (1986) <u>J. Cell</u>. <u>Phys</u>. <u>128</u>:1.

64. Van Houten, M. et al. (1979) <u>Nature</u> (London) <u>282</u>:623.

65. Van Reswoude, J. et al. (1982) <u>Proc. Natl. Acad. Sci. USA 79</u>:6186.

66. Vodineliah, L. et al. (1983) <u>Proc. Natl. Acad. Sci. USA 80</u>:835-839.

67. Waldmann, T.A., Longo, D.L., Leonard, W.J., et al. (1985) <u>Cancer Res</u>. <u>45</u>:595.

68. Waldmann, T.A. (1986) <u>Science 232</u>:727.

69. Weissman, A. M. et al. (1986) <u>J. Cell Biol</u>. <u>102</u>:951.

70. Yamashiro, P.J. et al. (1984) <u>Cell 37</u>:789.

71. Yelton, D.E. (1981) <u>Ann. Rev. Biochem</u>., <u>50</u>:657-680.

72. Zoller, M.J., and Smith, M. (1982) <u>Nucl. Acids. Res</u>., <u>10</u>(20):6487-6500.

73. V. Oi and S. Morrison (1986) <u>BioTechniques</u>, <u>4</u>:214.

73. P. J. Maddon et al. (1985) <u>Cell</u>, <u>42</u>:93.

74. Wilson, M.B. and Nakane, P.K. (1978) <u>Immunofluorescence and Related Staining Techniques</u> (Elsevier/North Holland Biomedical Press, Amsterdam) 215.

75. Bayer et al. (1979) Methods in Enzymology, 62:308.

76. Yelton, D.E. et al. (1981) J. Exp. Med., 156:1151.

77. Zack, D.J., et al. (1981) J. Exp. Med.. 154:1554.

78. Dangl, J.L., et al. (1988) EMBO J., 7:1989.

**Claims**

1. A modified chimeric monoclonal antibody comprising two molecules of each of two different polypeptides, the shorter of which functions as the light chains of the antibody and the longer of which polypeptides function as the heavy chains of the antibody, each polypeptide which functions as a heavy chain having a variable region characteristic of a first mammal and a constant region characteristic of a second mammal, and each polypeptide which functions as a light chain having a variable region characteristic of a mammal and a constant region characteristic of a mammal, wherein a receptor-binding ligand is covalently attached to the ends of the constant regions of each of the polypeptides which function as the heavy chains of the antibody.

2. An immunologically reactive complex comprising two different polypeptides, the shorter of which functions as a light chain and the longer of which functions as a heavy chain, the polypeptide which functions as the heavy chain having a variable region characteristic of a first mammal and a constant region characteristic of a second mammal, and the polypeptide which functions as the light chain having a variable region characteristic of a mammal and a constant region characteristic of a second mammal, wherein a receptor-binding ligand is covalently attached to the ends of a constant region of one of the polypeptides.

3. A chimeric polypeptide capable of functioning as a heavy chain of an antibody comprising a variable region characteristic of a first mammal and a constant region characteristic of a second mammal, wherein a receptor-binding ligand is covalently attached to the constant region of the polypeptide.

4. A chimeric polypeptide capable of functioning as a light chain of an antibody comprising a variable region characteristic of a first mammal and a constant region characteristic of a second mammal, wherein a receptor-binding ligand is covalently attached to the constant region of the polypeptide.

5. The modified chimeric monoclonal antibody of claim 1 or the immunologically reactive complex of claim 2, wherein the variable region and the constant region of the light chain are both characteristic of the second mammal.

6. The modified chimeric monoclonal antibody of claim 1 or the immunologically reactive complex of claim 2, wherein the variable region and the constant region of the light chain are both characteristic of the first mammal.

7. The modified chimeric monoclonal antibody of claim 1 or the immunologically reactive complex of claim 2, wherein the variable region of the light chain is characteristic of either the first or the second mammal and the constant region of the light chain is characteristic of the other mammal.

8. The modified chimeric monoclonal antibody of any one of claims 1 and 5 to 7 or the immunologically reactive complex of any one of claims 2 and 5 to 7 or the chimeric polypeptide of claim 3 or 4, wherein the first mammal is mouse and the second mammal is human.

9. The modified chimeric monoclonal antibody of any one of claims 1 and 5 to 7 or the immunologically reactive complex of any one of claims 2 and 5 to 7 or the chimeric polypeptide of claim 3 or 4, wherein the first mammal is human and the second mammal is mouse.

10. A modified chimeric monoclonal antibody comprising two molecules of each of two different polypeptides, the shorter of which functions as the light chains of the antibody and the longer of which polypeptides function as the heavy chains of the antibody, each polypeptide which functions as a heavy chain having a variable region characteristic of a first mammal and a constant region characteristic of a second mammal, and each polypeptide which functions as a light chain having a variable region characteristic of a mammal and a constant region characteristic of a mammal,

wherein a receptor-binding ligand is covalently attached to the ends of the constant regions of each of the polypeptides which function as the heavy chains of the antibody, or replaces at least a portion of the constant region of each of the polypeptides which function as the heavy chains of the antibody; or

an immunologically reactive complex comprising two different polypeptides, the shorter of which functions as a light chain and the longer of which functions as a heavy chain, the polypeptide which functions as the heavy chain having a variable region characteristic of a first mammal and a constant region characteristic of a second mammal, and the polypeptide which functions as the light chain having a variable region characteristic of a mammal and a constant region characteristic of a second mammal,

wherein a receptor-binding ligand is covalently attached to the ends of a constant region of one of the polypeptides, or replaces at least a portion of a constant region of one of the polypeptides; or

a chimeric polypeptide capable of functioning as a heavy chain or light chain of an antibody comprising a variable region characteristic of a first mammal and a constant region characteristic of a second mammal,

wherein a receptor-binding ligand is covalently attached to the constant region of the polypeptide, or replaces at least a portion of the constant region of the polypeptide;

wherein the receptor-binding ligand comprises a growth factor.

11. The modified chimeric monoclonal antibody, immunologically reactive complex, or chimeric polypeptide of claim 10, wherein the growth factor comprises insulin.

12. The modified chimeric monoclonal antibody, immunologically reactive complex, or chimeric polypeptide of claim 10, wherein the growth factor comprises insulin-like growth factor.

13. The modified chimeric monoclonal antibody, immunologically reactive complex, or chimeric polypeptide of claim 12, wherein the insulin-like growth factor comprises insulin-like growth factor 1.

14. The modified chimeric monoclonal antibody, immunologically reactive complex, or chimeric polypeptide of claim 13, wherein the insulin-like growth factor comprises insulin-like growth factor 2.

15. The modified chimeric monoclonal antibody, immunologically reactive complex, or chimeric polypeptide of claim 10, wherein the growth factor comprises platelet-derived growth factor.

16. The modified chimeric monoclonal antibody, immunologically reactive complex, or chimeric polypeptide of claim 10, wherein the growth factor comprises epidermal growth factor.

17. The modified chimeric monoclonal antibody, immunologically reactive complex, or chimeric polypeptide of claim 10, wherein the growth factor comprises transforming growth factor.

18. The modified chimeric monoclonal antibody, immunologically reactive complex, or chimeric polypeptide of claim 17, wherein the transforming growth factor comprises transforming growth factor-$\alpha$.

19. The modified chimeric monoclonal antibody, immunologically reactive complex, or chimeric polypeptide of claim 17, wherein the transforming growth factor comprises transforming growth factor-$\beta$.

20. The modified chimeric monoclonal antibody, immunologically reactive complex, or chimeric polypeptide of claim 19, wherein the transforming growth factor-$\beta$ comprises transforming growth factor-$\beta$1.

21. The modified chimeric monoclonal antibody, immunologically reactive complex, or chimeric polypeptide of claim 19, wherein the transforming growth factor-$\beta$ comprises transforming growth factor-$\beta$2.

22. The modified chimeric monoclonal antibody, immunologically reactive complex, or chimeric polypeptide of claim 19, wherein the transforming growth factor-$\beta$ comprises transforming growth factor-$\beta$3.

23. The modified chimeric monoclonal antibody, immunologically reactive complex, or chimeric polypeptide of claim 10, wherein the growth factor comprises a nerve growth factor.

24. The modified chimeric monoclonal antibody, immunologically reactive complex, or chimeric polypeptide of claim 10, wherein the growth factor comprises growth hormone.

**25.** The modified chimeric monoclonal antibody, immunologically reactive complex, or chimeric polypeptide of claim 10, wherein the growth factor comprises a growth hormone releasing factor.

**26.** A modified chimeric monoclonal antibody comprising two molecules of each of two different polypeptides, the shorter of which functions as the light chains of the antibody and the longer of which polypeptides function as the heavy chains of the antibody, each polypeptide which functions as a heavy chain having a variable region characteristic of a first mammal and a constant region characteristic of a second mammal, and each polypeptide which functions as a light chain having a variable region characteristic of a mammal and a constant region characteristic of a mammal,
wherein a receptor-binding ligand is covalently attached to the ends of the constant regions of each of the polypeptides which function as the heavy chains of the antibody, or replaces at least a portion of the constant region of each of the polypeptides which function as the heavy chains of the antibody; or
an immunologically reactive complex comprising two different polypeptides, the shorter of which functions as a light chain and the longer of which functions as a heavy chain, the polypeptide which functions as the heavy chain having a variable region characteristic of a first mammal and a constant region characteristic of a second mammal, and the polypeptide which functions as the light chain having a variable region characteristic of a mammal and a constant region characteristic of a second mammal,
wherein a receptor-binding ligand is covalently attached to the ends of a constant region of one of the polypeptides, or replaces at least a portion of a constant region of one of the polypeptides; or
a chimeric polypeptide capable of functioning as a heavy chain or light chain of an antibody comprising a variable region characteristic of a first mammal and a constant region characteristic of a second mammal,
wherein a receptor-binding ligand is covalently attached to the constant region of the polypeptide, or replaces at least a portion of the constant region of the polypeptide;
wherein the receptor-binding ligand comprises tumor necrosis factor.

**27.** A modified chimeric monoclonal antibody comprising two molecules of each of two different polypeptides, the shorter of which functions as the light chains of the antibody and the longer of which polypeptides function as the heavy chains of the antibody, each polypeptide which functions as a heavy chain having a variable region characteristic of a first mammal and a constant region characteristic of a second mammal, and each polypeptide which functions as a light chain having a variable region characteristic of a mammal and a constant region characteristic of a mammal,
wherein a receptor-binding ligand is covalently attached to the ends of the constant regions of each of the polypeptides which function as the heavy chains of the antibody, or replaces at least a portion of the constant region of each of the polypeptides which function as the heavy chains of the antibody; or
an immunologically reactive complex comprising two different polypeptides, the shorter of which functions as a light chain and the longer of which functions as a heavy chain, the polypeptide which functions as the heavy chain having a variable region characteristic of a first mammal and a constant region characteristic of a second mammal, and the polypeptide which functions as the light chain having a variable region characteristic of a mammal and a constant region characteristic of a second mammal,
wherein a receptor-binding ligand is covalently attached to the ends of a constant region of one of the polypeptides, or replaces at least a portion of a constant region of one of the polypeptides; or
a chimeric polypeptide capable of functioning as a heavy chain or light chain of an antibody comprising a variable region characteristic of a first mammal and a constant region characteristic of a second mammal,
wherein a receptor-binding ligand is covalently attached to the constant region of the polypeptide, or replaces at least a portion of the constant region of the polypeptide;
wherein the receptor-binding ligand comprises transferrin.

**28.** The modified chimeric monoclonal antibody of any one of claims 1 and 5 to 9, the immunologically reactive complex of any one of claims 2 and 5 to 9, or the chimeric polypeptide of claim 3, 4, 8 or 9, wherein the receptor-binding ligand comprises a lymphokine.

**29.** The modified chimeric monoclonal antibody, immunologically reactive complex or chimeric polypeptide of claim 28, wherein the lymphokine is selected from the group consisting of macrophage inhibition factor, leukocyte migration inhibition factor, macrophage activating factor, macrophage cytotoxicity factor, interleukin-1, interleukin-2, interleukin-3, interleukin-4, interleukin-5, interleukin-6, interleukin-7, lymphotoxin, monocyte-derived lymphocyte activating factor, and T helper cell replacing factor.

**30.** A modified chimeric monoclonal antibody comprising two molecules of each of two different polypeptides, the shorter of which functions as the light chains of the antibody and the longer of which polypeptides function as the heavy chains of the antibody, each polypeptide which functions as a heavy chain having a variable region characteristic of a first mammal and a constant region characteristic of a second mammal, and each polypeptide which functions as a light chain having a variable region characteristic of a mammal and a constant region characteristic of a mammal,

wherein a receptor-binding ligand is covalently attached to the ends of the constant regions of each of the polypeptides which function as the heavy chains of the antibody, or replaces at least a portion of the constant region of each of the polypeptides which function as the heavy chains of the antibody; or

an immunologically reactive complex comprising two different polypeptides, the shorter of which functions as a light chain and the longer of which functions as a heavy chain, the polypeptide which functions as the heavy chain having a variable region characteristic of a first mammal and a constant region characteristic of a second mammal, and the polypeptide which functions as the light chain having a variable region characteristic of a mammal and a constant region characteristic of a second mammal,

wherein a receptor-binding ligand is covalently attached to the ends of a constant region of one of the polypeptides, or replaces at least a portion of a constant region of one of the polypeptides;

wherein the variable regions of the polypeptides comprise a domain of a T cell receptor.

31. A modified chimeric monoclonal antibody comprising two molecules of each of two different polypeptides, the shorter of which functions as the light chains of the

antibody and the longer of which polypeptides function as the heavy chains of the antibody, each polypeptide which functions as a heavy chain having a variable region characteristic of a first mammal and a constant region characteristic of a second mammal, and each polypeptide which functions as a light chain having a variable region characteristic of a mammal and a constant region characteristic of a mammal, wherein a receptor-binding ligand is covalently attached to the ends of the constant regions of each of the polypeptides which function as the heavy chains of the antibody, or replaces at least a portion of the constant region of each of the polypeptides which function as the heavy chains of the antibody; or

an immunologically reactive complex comprising two different polypeptides, the shorter of which functions as a light chain and the longer of which functions as a heavy chain, the polypeptide which functions as the heavy chain having a variable region characteristic of a first mammal and a constant region characteristic of a second mammal, and the polypeptide which functions as the light chain having a variable region characteristic of a mammal and a constant region characteristic of a second mammal,

wherein a receptor-binding ligand is covalently attached to the ends of a constant region of one of the polypeptides, or replaces at least a portion of a constant region of one of the polypeptides;

wherein the variable regions of the polypeptides comprise a domain of a MHC antigen.

32. The modified chimeric monoclonal antibody or the immunologically reactive complex of claim 31, wherein the MHC antigen is an HLA antigen.

33. The modified chimeric monoclonal antibody or the immunologically reactive complex of claim 31, wherein the MHC antigen is an H-2 antigen.

34. A modified chimeric monoclonal antibody comprising two molecules of each of two different polypeptides, the shorter of which functions as the light chains of the antibody and the longer of which polypeptides function as the heavy chains of the antibody, each polypeptide which functions as a heavy chain having a variable region characteristic of a first mammal and a constant region characteristic of a second mammal, and each polypeptide which functions as a light chain having a variable region characteristic of a mammal and a constant region characteristic of a mammal, wherein a receptor-binding ligand is covalently attached to the ends of the constant regions of each of the polypeptides which function as the heavy chains of the antibody, or replaces at least a portion of the constant region of each of the polypeptides which function as the heavy chains of the antibody; or

an immunologically reactive complex comprising two different polypeptides, the shorter of which functions as a light chain and the longer of which functions as a heavy chain, the polypeptide which functions as the heavy chain having a variable region characteristic of a first mammal and a constant region characteristic of a second mammal, and the polypeptide which functions as the light chain having a variable region characteristic of a mammal and a constant region characteristic of a second mammal,

wherein a receptor-binding ligand is covalently attached to the ends of a constant region of one of the polypeptides, or replaces at least a portion of a constant region of one of the polypeptides;

wherein the variable regions of the polypeptides comprise a domain of a surface glycoprotein CD4.

**35.** A modified chimeric monoclonal antibody comprising two molecules of each of two different polypeptides, the shorter of which functions as the light chains of the antibody and the longer of which polypeptides function as the heavy chains of the antibody, each polypeptide which functions as a heavy chain having a variable region characteristic of a first mammal and a constant region characteristic of a second mammal, and each polypeptide which functions as a light chain having a variable region characteristic of a mammal and a constant region characteristic of a mammal, wherein a receptor-binding ligand is covalently attached to the ends of the constant regions of each of the polypeptides which function as the heavy chains of the antibody, or replaces at least a portion of the constant region of each of the polypeptides which function as the heavy chains of the antibody;
wherein the variable regions of the polypeptides comprise a domain of a surface glycoprotein CD8.

**36.** The chimeric monoclonal antibody of any one of claims 1 and 5 to 35, wherein the antibody is an IgG antibody.

**37.** A modified chimeric monoclonal antibody comprising two molecules of each of two different polypeptides, the shorter of which functions as the light chains of the antibody and the longer of which polypeptides function as the heavy chains of the antibody, each polypeptide which functions as a heavy chain having a variable region characteristic of a first mammal and a constant region characteristic of a second mammal, and each polypeptide which functions as a light chain having a variable region characteristic of a mammal and a constant region characteristic of a mammal, wherein a receptor-binding ligand is covalently attached to the ends of the constant regions of each of the polypeptides which function as the heavy chains of the antibody, or replaces at least a portion of the constant region of each of the polypeptides which function as the heavy chains of the antibody;
wherein the antibody is an IgA antibody.

**38.** A modified chimeric monoclonal antibody comprising two molecules of each of two different polypeptides, the shorter of which functions as the light chains of the antibody and the longer of which polypeptides function as the heavy chains of the antibody, each polypeptide which functions as a heavy chain having a variable region characteristic of a first mammal and a constant region characteristic of a second mammal, and each polypeptide which functions as a light chain having a variable region characteristic of a mammal and a constant region characteristic of a mammal, wherein a receptor-binding ligand is covalently attached to the ends of the constant regions of each of the polypeptides which function as the heavy chains of the antibody, or replaces at least a portion of the constant region of each of the polypeptides which function as the heavy chains of the antibody;
wherein the antibody is an IgD antibody.

**39.** A modified chimeric monoclonal antibody comprising two molecules of each of two different polypeptides, the shorter of which functions as the light chains of the antibody and the longer of which polypeptides function as the heavy chains of the antibody, each polypeptide which functions as a heavy chain having a variable region characteristic of a first mammal and a constant region characteristic of a second mammal, and each polypeptide which functions as a light chain having a variable region characteristic of a mammal and a constant region characteristic of a mammal, wherein a receptor-binding ligand is covalently attached to the ends of the constant regions of each of the polypeptides which function as the heavy chains of the antibody, or replaces at least a portion of the constant region of each of the polypeptides which function as the heavy chains of the antibody;
wherein the antibody is an IgE antibody.

**40.** A modified chimeric monoclonal antibody comprising two molecules of each of two different polypeptides, the shorter of which functions as the light chains of the antibody and the longer of which polypeptides function as the heavy chains of the antibody, each polypeptide which functions as a heavy chain having a variable region characteristic of a first mammal and a constant region characteristic of a second mammal, and each polypeptide which functions as a light chain having a variable region characteristic of a mammal and a constant region characteristic of a mammal, wherein a receptor-binding ligand is covalently attached to the ends of the constant regions of each of the polypeptides which function as the heavy chains of the antibody, or replaces at least a portion of the constant region of each of the polypeptides which function as the heavy chains of the antibody;
wherein the antibody is an IgM antibody.

**41.** An immunologically reactive complex comprising two different polypeptides, the shorter of which functions as a light chain and the longer of which functions as a heavy chain, the polypeptide which functions as the heavy chain having a variable region characteristic of a first mammal and a constant region characteristic of a second mammal, and the polypeptide which functions as the light chain having a variable region characteristic of a mammal and a constant region characteristic of a second mammal,
wherein a receptor-binding ligand is covalently attached to the ends of a constant region of one of the polypeptides, or replaces at least a portion of a constant region of one of the polypeptides;

wherein the receptor-binding ligand replaces at least a portion of the constant region of the polypeptide which functions as the light chain.

42. A modified chimeric monoclonal antibody comprising two molecules of each of two different polypeptides, the shorter of which functions as the light chains of the antibody and the longer of which polypeptides function as the heavy chains of the antibody, each polypeptide which functions as a heavy chain having a variable region characteristic of a first mammal and a constant region characteristic of a second mammal, and each polypeptide which functions as a light chain having a variable region characteristic of a mammal and a constant region characteristic of a mammal,

wherein a receptor-binding ligand is covalently attached to the ends of the constant regions of each of the polypeptides which function as the heavy chains of the antibody, or replaces at least a portion of the constant region of each of the polypeptides which function as the heavy chains of the antibody; or

an immunologically reactive complex comprising two different polypeptides, the shorter of which functions as a light chain and the longer of which functions as a heavy chain, the polypeptide which functions as the heavy chain having a variable region characteristic of a first mammal and a constant region characteristic of a second mammal, and the polypeptide which functions as the light chain having a variable region characteristic of a mammal and a constant region characteristic of a second mammal,

wherein a receptor-binding ligand is covalently attached to the ends of a constant region of one of the polypeptides, or replaces at least a portion of a constant region of one of the polypeptides; or

a chimeric polypeptide capable of functioning as a heavy chain or light chain of an antibody comprising a variable region characteristic of a first mammal and a constant region characteristic of a second mammal,

wherein a receptor-binding ligand is covalently attached to the constant region of the polypeptide, or replaces at least a portion of the constant region of the polypeptide;

to which a moiety is covalently attached.

43. The chimeric monoclonal antibody, immunologically reactive complex or chimeric polypeptide of claim 42, wherein the moiety comprises a drug.

44. The chimeric monoclonal antibody, immunologically reactive complex or chimeric polypeptide of claim 43, wherein the drug is a cytotoxic agent.

45. The chimeric monoclonal antibody, immunologically reactive complex or chimeric polypeptide of claim 44, wherein the cytotoxic agent is methotrexate.

46. The chimeric monoclonal antibody, immunologically reactive complex or chimeric polypeptide of claim 44, wherein the cytotoxic agent is a toxin.

47. The chimeric monoclonal antibody, immunologically reactive complex or chimeric polypeptide of claim 42, wherein the moiety comprises a detectable label.

48. The chimeric monoclonal antibody, immunologically reactive complex or chimeric polypeptide of claim 47, wherein the detectable label is biotin, a fluorophore, a chromophore, a heavy metal, a paramagnetic isotope, or a radioisotope.

49. A pharmaceutical composition comprising the chimeric monoclonal antibody, immunologically reactive complex, or chimeric polypeptide of any one of claims 43 to 46, and a pharmaceutically acceptable carrier.

50. A nucleic acid molecule encoding the chimeric polypeptide of any one of claims 3, 4, and 8 to 28.

51. An expression vector for producing the chimeric polypeptide of any one of claims 3, 4, and 8 to 28, comprising a nucleic acid encoding the chimeric polypeptide and suitable regulatory elements positioned within the vector so as to permit expression of the polypeptide in a suitable host.

52. A method of producing a modified chimeric monoclonal antibody which comprises:

(a)

(i) cotransfecting a suitable/nonantibody-producing host cell with two expression plasmids, (A) one of which encodes a polypeptide capable of functioning as the heavy chain of the antibody and having a variable region characteristic of a first mammal and a constant region characteistic of a second mammal, wherein a receptor-binding ligand replaces at least a portion of the constant region of the heavy chain polypeptide and (B) the other of which encodes a polypeptide capable of functioning as the light chain of the antibody and having a variable region characteristic of a mammal and a constant region characteristic of a mammal, or

(ii) cotransfecting a suitable/nonantibody-producing host cell with an expression plasmid, which encodes (A) a polypeptide capable of functioning as the heavy chain of the antibody and having a variable region characteristic of a first mammal and a constant region characteistic of a second mammal, wherein a receptor-binding ligand replaces at least a portion of the constant region of the heavy chain polypeptide and (B) a polypeptide capable of functioning as the light chain of the antibody and having a variable region characteristic of a mammal and a constant region characteristic of a mammal;

(b) treating the cotransfected host cell so as to effect expression of the polypeptides encoded by the plasmid or plasmids and formation of the chimeric monoclonal antibody within the host cell and excretion into the culture medium of the antibody by the host cell; and

(c) recovering the resulting excreted chimeric monoclonal antibody, from the culture medium;

wherein the human cell is a myeloma cell.

53. Use of the chimeric monoclonal antibody, immunologically reactive complex or chimeric polypetide of any one of claims 43 to 46 for the preparation of a pharmaceutical composition according to claim 49 for delivering a drug to a cell having a receptor for a growth factor on its surface, wherein the receptor-binding ligand of the antibody comprises the growth factor which binds to the receptor so that the antibody binds to the cell and thereby delivers the drug to the cell.

54. The use of claim 53, wherein the cell is a brain cell and wherein the growth factor upon binding to the receptor results in transport of the antibody across the blood-brain barrier.

55. The use of claim 53, wherein the cell is a blood cell.

56. The use of claim 53, wherein the cell is a muscle cell.

57. The use of claim 53, wherein the cell is a nerve cell.

58. The use of claim 53, wherein the cell is a bone cell.

59. The use of claim 53, wherein the cell is an epithelial cell.

60. The use of claim 53, wherein the growth factor is selected from the group consisting of insulin like growth factor 1, insulin-like growth factor 2, insulin, and transferrin.

61. The use of claim 54, wherein the brain cell is abnormal and associated with progressive dementia and wherein the pharmaceutical composition comprises an amount of the chimeric antibody effective to halt the progressive dementia.

62. The use of claim 54, wherein the brain cell is abnormal and associated with a cerebral cortical atrophy and wherein the pharmaceutical composition comprises an amount of the chimeric antibody effective to halt the cerebral cortical atrophy.

63. The use of claim 54, wherein the brain cell is malignant and associated with a neurosarcoma and wherein the pharmaceutical composition comprises an amount of the chimeric antibody effective to halt the neurosarcoma.

64. The use of any one of claims 54 to 59, wherein the brain cell is malignant and associated with a lymphoma and wherein said pharmaceutical composition comprises an amount of the chimeric antibody effective to halt the lymphoma.

**65.** The use of claim 54, wherein the brain cell is malignant and associated with a carcinosarcoma and wherein the pharmaceutical composition comprises an amount of the chimeric antibody effective to halt the carcinosarcoma.

**66.** The use of any one of claims 54 to 59, wherein the brain cell is malignant and associated with a sarcoma and wherein said pharmaceutical composition comprises an amount of the chimeric antibody effective to halt the sarcoma.

**67.** The use of claim 54, wherein the brain cell is malignant and associated with a carcinomatous cerebellar degeneration and wherein the pharmaceutical composition comprises an amount of the chimeric antibody effective to halt the carcinomatous cerebellar degeneration.

**68.** The use of claim 53, wherein the cell is malignant and associated with a melanoma and wherein said pharmaceutical composition comprises an amount of the chimeric antibody effective to halt the melanoma.

**69.** The use of claim 53, wherein the cell is malignant and associated with a breast cancer and wherein said pharmaceutical composition comprises an amount of the chimeric antibody effective to halt the breast cancer.

**70.** The use of claim 53, wherein the cell is malignant and associated with a carcinoma and wherein said pharmaceutical composition comprises an amount of the chimeric antibody effective to halt the carcinoma.

**71.** Use of the chimeric monoclonal antibody, immunologically reactive complex or chimeric polypeptide of claim 47 or 48 for the preparation of a diagnostic composition for detecting a cell having a receptor for a growth factor on its surface, wherein the receptor-binding ligand of the antibody comprises the growth factor which binds to the receptor so that the antibody binds to the cell and thereby detects the cell.

**72.** The use of claim 71, wherein the cell is a brain cell and wherein the growth factor upon binding to the receptor results in transport of the antibody across the blood-brain barrier.

**73.** The use of claim 71 or 72, wherein the growth factor is selected from the group consisting of insulin-like growth factor 1, insulin-like growth factor 2, insulin, and transferrin.

**74.** The use of any one of claims 71 to 73, wherein the brain cell is abnormal and associated with an argyrophil plaque and the diagnostic composition comprises an amount of the antibody effective to permit detection of the plaque.

**75.** The use of any one of claims 71 to 73, wherein the brain cell is abnormal and associated with a brain tumor and the diagnostic composition comprises an amount of the antibody effective to permit detection of the tumor.

**76.** The use of claim 53 or 71, wherein the cell is an adipose cell.

**77.** The use of claim 53 or 71, wherein the cell is a chronic myelogenous leukemia cell.

**78.** The modified chimeric monoclonal antibody or immunologically reactive complex of any one of claims 10 to 27, 30 to 35, and 37 to 48 wherein the variable region and the constant region of the light chain are both characteristic of the second mammal.

**79.** The modified chimeric monoclonal antibody or immunologically reactive complex of any one of claims 10 to 27, 30 to 35, and 37 to 48, wherein the variable region and the constant region of the light chain are both characteristic of the first mammal.

**80.** The modified chimeric monoclonal antibody or immunologically reactive complex of any one of claims 10 to 27, 30 to 35, and 37 to 48 , wherein the variable region of the light chain is characteristic of either the first or the second mammal and the constant region of the light chain is characteristic of the other mammal.

**81.** The modified chimeric monoclonal antibody, immunologically reactive complex, or chi.meric polypeptide of any one of claims 10 to 27, 30 to 35, and 37 to 48, wherein the first mammal is mouse and the second mammal is human.

**82.** The modified chimeric monoclonal antibody, immunologically reactive complex, or chimeric polypeptide of any one of claims 10 to 27, 30 to 35, and 37 to 48, wherein the first mammal is human and the second mammal is mouse.

83. A method of producing a modified chimeric monoclonal antibody of any one of claims 1, 5 to 40, 42 to 48 and 78 to 82 which comprises:

(a)

(i) cotransfecting a suitable/nonantibody-producing host cell with two expression plasmids, (A) one of which encodes a polypeptide capable of functioning as the heavy chain of the antibody and having a variable region characteristic of a first mammal and a constant region characteistic of a second mammal, wherein a receptor-binding ligand replaces at least a portion of the constant region of the heavy chain polypeptide and (B) the other of which encodes a polypeptide capable of functioning as the light chain of the antibody and having a variable region characteristic or a mammal and a constant region characteristic of a mammal, or

(ii) cotransfecting a suitable/nonantibody-producing host cell with an expression plasmid, which encodes (A) a polypeptide capable of functioning as the heavy chain of the antibody and having a variable region characteristic of a first mammal and a constant region characteistic of a second mammal, wherein a receptor-binding ligand replaces at least a portion of the constant region of the heavy chain polypeptide and (B) a polypeptide capable of functioning as the light chain of the antibody and having a variable region characteristic of a mammal and a constant region characteristic of a mammal;

(b) treating the cotransfected host cell so as to effect expression of the polypeptides encoded by the plasmid or plasmids and formation of the chimeric monoclonal antibody within the host cell and excretion into the culture medium of the antibody by the host cell; and

(c) recovering the resulting excreted chimeric monoclonal antibody, from the culture medium;

wherein the human cell is a myeloma cell.

84. A process for the preparation of an immunologically reactive complex according to any one of claims 2, 5 to 34, 41 to 48 and 78 to 82 which comprises synthesizing the respective components by using genetic engineering or other methods known in the art.

85. A process for the preparation of a chimeric polypeptide according to any one of claims 3, 4, 8, 9 to 29, 42 to 48, 81 and 82 which comprises synthesizing the polypeptide by using genetic engineering or other methods known in the art.

86. A process for the preparation of the pharmaceutical composition according to claim 49 which comprises combining the active agent with a pharmaceutically acceptable carrier.

**Patentansprüche**

1. Modifizierter chimärer monoclonaler Antikörper, umfassend zwei Moleküle von je zwei unterschiedlichen Polypeptiden, wobei das kürzere als die leichten Ketten des Antikörpers und das längere der Polypeptide als die schweren Ketten des Antikörpers wirkt, wobei jedes Polypeptid, das als eine schwere Kette wirkt, einen variablen Bereich aufweist, der kennzeichnend ist für einen ersten Säuger, und einen konstanten Bereich, der kennzeichnend ist für einen zweiten Säuger, und jedes Polypeptid, das als eine leichte Kette wirkt, einen variablen Bereich aufweist, der kennzeichnend ist für einen Säuger, und einen konstanten Bereich, der kennzeichnend ist für einen Säuger, wobei ein Rezeptor-bindender Ligand kovalent an die Enden der konstanten Bereiche jedes der Polypeptide gebunden ist, die als die schweren Ketten des Antikörpers wirken.

2. Immunologisch reaktiver Komplex, umfassend zwei unterschiedliche Polypeptide, wobei das kürzere als eine leichte Kette und das längere als eine schwere Kette wirkt, wobei das Polypeptid, das als die schwere Kette wirkt, einen variablen Bereich aufweist, der kennzeichnend ist für einen ersten Säuger, und einen konstanten Bereich, der kennzeichnend ist für einen zweiten Säuger, und das Polypeptid, das als die leichte Kette wirkt, einen variablen Bereich aufweist, der kennzeichnend ist für einen Säuger, und einen konstanten Bereich, der kennzeichnend ist für einen zweiten Säuger, wobei ein Rezeptor-bindender Ligand kovalent an die Enden eines konstanten Bereichs eines der Polypeptide gebunden ist.

3. Chimäres Polypeptid, das als eine schwere Kette eines Antikörpers wirken kann, umfassend einen variablen Bereich, der kennzeichnend ist für einen ersten Säuger, und einen konstanten Bereich, der kennzeichnend ist für einen zweiten Säuger, wobei ein Rezeptor-bindender Ligand kovalent an den konstanten Bereich des Polypeptids gebunden ist.

4. Chimäres Polypeptid, das als eine leichte Kette eines Antikörpers wirken kann, umfassend einen variablen Bereich, der kennzeichnend ist für einen ersten Säuger, und einen konstanten Bereich, der kennzeichnend ist für einen zweiten Säuger, wobei ein Rezeptor-bindender Ligand kovalent an den konstanten Bereich des Polypeptids gebunden ist.

5. Modifizierter chimärer monoclonaler Antikörper nach Anspruch 1 oder immunologisch reaktiver Komplex nach Anspruch 2, wobei der variable Bereich und der konstante Bereich der leichten Kette beide für den zweiten Säuger kennzeichnend sind.

6. Modifizierter chimärer monoclonaler Antikörper nach Anspruch 1 oder immunologisch reaktiver Komplex nach Anspruch 2, wobei der variable Bereich und der konstante Bereich der leichten Kette beide für den ersten Säuger kennzeichnend sind.

7. Modifizierter chimärer monoclonaler Antikörper nach Anspruch 1 oder immunologisch reaktiver Komplex nach Anspruch 2, wobei der variable Bereich der leichten Kette kennzeichnend ist entweder für den ersten oder den zweiten Säuger und der konstante Bereich der leichten Kette kennzeichnend ist für den anderen Säuger.

8. Modifizierter chimärer monoclonaler Antikörper nach einem der Ansprüche 1 und 5 bis 7 oder immunologisch reaktiver Komplex nach einem der Ansprüche 2 und 5 bis 7 oder chimäres Polypeptid nach Anspruch 3 oder 4, wobei der erste Säuger eine Maus und der zweite Säuger ein Mensch ist.

9. Modifizierter chimärer monoclonaler Antikörper nach einem der Ansprüche 1 und 5 bis 7 oder immunologisch reaktiver Komplex nach einem der Ansprüche 2 und 5 bis 7 oder chimäres Polypeptid nach Anspruch 3 oder 4, wobei der erste Säuger ein Mensch und der zweite Säuger eine Maus ist.

10. Modifizierter chimärer monoclonaler Antikörper, umfassend zwei Moleküle von je zwei unterschiedlichen Polypeptiden, wobei das kürzere als die leichten Ketten des Antikörpers und das längere der Polypeptide als die schweren Ketten des Antikörpers wirkt, wobei jedes Polypeptid, das als eine schwere Kette wirkt, einen variablen Bereich aufweist, der kennzeichnend ist für einen ersten Säuger, und einen konstanten Bereich, der kennzeichnend ist für einen zweiten Säuger, und jedes Polypeptid, das als eine leichte Kette wirkt, einen variablen Bereich aufweist, der kennzeichnend ist für einen Säuger, und einen konstanten Bereich, der kennzeichnend ist für einen Säuger, wobei ein Rezeptor-bindender Ligand kovalent an die Enden der konstanten Bereiche jedes der Polypeptide gebunden ist, die als die schweren Ketten des Antikörpers wirken, oder mindestens einen Teil des konstanten Bereichs jedes der Polypeptide ersetzt, die als die schweren Ketten des Antikörpers wirken; oder
immunologisch reaktiver Komplex, umfassend zwei unterschiedliche Polypeptide, wobei das kürzere als eine leichte Kette und das längere als eine schwere Kette wirkt, wobei das Polypeptid, das als die schwere Kette wirkt, einen variablen Bereich aufweist, der kennzeichnend ist für einen ersten Säuger, und einen konstanten Bereich, der kennzeichnend ist für einen zweiten Säuger, und das Polypeptid, das als die leichte Kette wirkt, einen variablen Bereich aufweist, der kennzeichnend ist für einen Säuger, und einen konstanten Bereich, der kennzeichnend ist für einen zweiten Säuger,
wobei ein Rezeptor-bindender Ligand kovalent an die Enden eines konstanten Bereichs von einem der Polypeptide gebunden ist, oder mindestens einen Teil eines konstanten Bereichs einer der Polypeptide ersetzt; oder
chimäres Polypeptid, das als schwere oder leichte Kette eines Antikörpers wirken kann, umfassend einen variablen Bereich, der kennzeichnend ist für einen ersten Säuger, und einen konstanten Bereich, der kennzeichnend ist für einen zweiten Säuger,
wobei ein Rezeptor-bindender Ligand kovalent an den konstanten Bereich des Polypeptids gebunden ist oder mindestens einen Teil des konstanten Bereichs des Polypeptids ersetzt;
wobei der Rezeptor-bindende Ligand einen Wachstumsfaktor umfaßt.

11. Modifizierter chimärer monoclonaler Antikörper, immunologisch reaktiver Komplex oder chimäres Polypeptid nach Anspruch 10, wobei der Wachstumsfaktor Insulin umfaßt.

12. Modifizierter chimärer monoclonaler Antikörper, immunologisch reaktiver Komplex oder chimäres Polypeptid nach Anspruch 10, wobei der Wachstumsfaktor einen Insulin-ähnlichen Wachstumsfaktor umfaßt.

13. Modifizierter chimärer monoclonaler Antikörper, immunologisch reaktiver Komplex oder chimäres Polypeptid nach Anspruch 12, wobei der Insulin-ähnliche Wachstumsfaktor den Insulin-ähnlichen Wachstumsfaktor 1 umfaßt.

14. Modifizierter chimärer monoclonaler Antikörper, immunologisch reaktiver Komplex oder chimäres Polypeptid nach Anspruch 13, wobei der Insulin-ähnliche Wachstumsfaktor den Insulin-ähnlichen Wachstumsfaktor 2 umfaßt.

15. Modifizierter chimärer monoclonaler Antikörper, immunologisch reaktiver Komplex oder chimäres Polypeptid nach Anspruch 10, wobei der Wachstumsfaktor einen von Thrombocyten stammenden Wachstumsfaktor umfaßt.

16. Modifizierter chimärer monoclonaler Antikörper, immunologisch reaktiver Komplex oder chimäres Polypeptid nach Anspruch 10, wobei der Wachstumsfaktor einen epidermalen Wachstumsfaktor umfaßt.

17. Modifizierter chimärer monoclonaler Antikörper, immunologisch reaktiver Komplex oder chimäres Polypeptid nach Anspruch 10, wobei der Wachstumsfaktor einen transformierenden Wachstumsfaktor umfaßt.

18. Modifizierter chimärer monoclonaler Antikörper, immunologisch reaktiver Komplex oder chimäres Polypeptid nach Anspruch 17, wobei der transformierende Wachstumsfaktor den transformierenden Wachstumsfaktor-$\alpha$ umfaßt.

19. Modifizierter chimärer monoclonaler Antikörper, immunologisch reaktiver Komplex oder chimäres Polypeptid nach Anspruch 17, wobei der transformierende Wachstumsfaktor den transformierenden Wachstumsfaktor-$\beta$ umfaßt.

20. Modifizierter chimärer monoclonaler Antikörper, immunologisch reaktiver Komplex oder chimäres Polypeptid nach Anspruch 19, wobei der transformierende Wachstumsfaktor-$\beta$, den transformierenden Wachstumsfaktor-$\beta$1 umfaßt.

21. Modifizierter chimärer monoclonaler Antikörper, immunologisch reaktiver Komplex oder chimäres Polypeptid nach Anspruch 19, wobei der transformierende Wachstumsfaktor-$\beta$, den transformierenden Wachstumsfaktor-$\beta$2 umfaßt.

22. Modifizierter chimärer monoclonaler Antikörper, immunologisch reaktiver Komplex oder chimäres Polypeptid nach Anspruch 19, wobei der transformierende Wachstumsfaktor-$\beta$, den transformierenden Wachstumsfaktor-$\beta$3 umfaßt.

23. Modifizierter chimärer monoclonaler Antikörper, immunologisch reaktiver Komplex oder chimäres Polypeptid nach Anspruch 10, wobei der Wachstumsfaktor einen Nervenwachstumsfaktor umfaßt.

24. Modifizierter chimärer monoclonaler Antikörper, immunologisch reaktiver Komplex oder chimäres Polypeptid nach Anspruch 10, wobei der Wachstumsfaktor ein Wachstumshormon umfaßt.

25. Modifizierter chimärer monoclonaler Antikörper, immunologisch reaktiver Komplex oder chimäres Polypeptid nach Anspruch 10, wobei der Wachstumsfaktor einen Wachstumshormon freisetzenden Faktor umfaßt.

26. Modifizierter chimärer monoclonaler Antikörper, umfassend zwei Moleküle von je zwei unterschiedlichen Polypeptiden, wobei das kürzere als die leichten Ketten des Antikörpers und das längere der Polypeptide als die schweren Ketten des Antikörpers wirkt, wobei jedes Polypeptid, das als eine schwere Kette wirkt, einen variablen Bereich aufweist, der kennzeichnend ist für einen ersten Säuger, und einen konstanten Bereich, der kennzeichnend ist für einen zweiten Säuger, und jedes Polypeptid, das als eine leichte Kette wirkt, einen variablen Bereich aufweist, der kennzeichnend ist für einen Säuger, und einen konstanten Bereich, der kennzeichnend ist für einen Säuger, wobei ein Rezeptor-bindender Ligand kovalent an die Enden der konstanten Bereiche von jedem der Polypeptide gebunden ist, die als die schweren Ketten des Antikörpers wirken, oder mindestens einen Teil des konstanten Bereichs jedes der Polypeptide ersetzt, die als die schweren Ketten des Antikörpers wirken; oder immunologisch reaktiver Komplex, umfassend zwei unterschiedliche Polypeptide, wobei das kürzere als eine leichte Kette und das längere als eine schwere Kette wirkt, wobei das Polypeptid, das als die schwere Kette wirkt, einen variablen Bereich aufweist, der kennzeichnend ist für einen ersten Säuger, und einen konstanten Bereich, der kennzeichnend ist für einen zweiten Säuger, und das Polypeptid, das als die leichte Kette wirkt, einen variablen Bereich aufweist, der kennzeichnend ist für einen Säuger, und einen konstanten Bereich, der kennzeichnend ist für einen zweiten Säuger, wobei ein Rezeptor-bindender Ligand kovalent an die Enden eines konstanten Bereiches eines der Polypeptide gebunden ist oder mindestens einen Teil eines konstanten Bereichs einer der Polypeptide ersetzt; oder
chimäres Polypeptid, das als eine schwere Kette oder leichte Kette eines Antikörpers wirken kann, umfassend einen variablen Bereich, der kennzeichnend ist für einen ersten Säuger, und einen konstanten Bereich, der kennzeichnend ist für einen zweiten Säuger,
wobei ein Rezeptor-bindender Ligand kovalent an den konstanten Bereich des Polypeptids gebunden ist oder mindestens einen Teil des konstanten Bereichs des Polypeptids ersetzt;
wobei der Rezeptor-bindende Ligand Tumornekrosefaktor umfaßt.

27. Modifizierter chimärer monoclonaler Antikörper, umfassend zwei Moleküle von je zwei unterschiedlichen Polypeptiden, wobei das kürzere als die leichten Ketten des Antikörpers und das längere der Polypeptide als die schweren Ketten des Antikörpers wirkt, wobei jedes Polypeptid, das als eine schwere Kette wirkt, einen variablen Bereich aufweist, der kennzeichnend ist für einen ersten Säuger, und einen konstanten Bereich, der kennzeichnend ist für einen zweiten Säuger, und jedes Polypeptid, das als eine leichte Kette wirkt, einen variablen Bereich aufweist, der kennzeichnend ist für einen Säuger, und einen konstanten Bereich, der kennzeichnend ist für einen Säuger,
wobei ein Rezeptor-bindender Ligand kovalent an die Enden der konstanten Bereiche jedes der Polypeptide gebunden ist, die als die schweren Ketten des Antikörpers wirken, oder mindestens einen Teil des konstanten Bereichs jedes der Polypeptide ersetzt, die als die schweren Ketten des Antikörpers wirken; oder
immunologisch reaktiver Komplex, umfassend zwei unterschiedliche Polypeptide, wobei das kürzere als eine leichte Kette und das längere als eine schwere Kette wirkt, wobei das Polypeptid, das als eine schwere Kette wirkt, einen variablen Bereich aufweist, der kennzeichnend ist für einen ersten Säuger, und einen konstanten Bereich, der kennzeichnend ist für einen zweiten Säuger, und das Polypeptid, das als die leichte Kette wirkt, einen variablen Bereich aufweist, der kennzeichnend ist für einen Säuger, und einen konstanten Bereich, der kennzeichnend ist für einen zweiten Säuger,
wobei ein Rezeptor-bindender Ligand kovalent an die Enden eines konstanten Bereiches eines der Polypeptide gebunden ist, oder mindestens einen Teil eines konstanten Bereichs einer der Polypeptide ersetzt; oder
chimäres Polypeptid, das als eine schwere Kette oder leichte Kette eines Antikörpers wirken kann, umfassend einen variablen Bereich, der kennzeichnend ist für einen ersten Säuger, und einen konstanten Bereich, der kennzeichnend ist für einen zweiten Säuger,
wobei ein Rezeptor-bindender Ligand kovalent an den konstanten Bereich des Polypeptids gebunden ist oder mindestens einen Teil des konstanten Bereichs des Polypeptids ersetzt;
wobei der Rezeptor-bindende Ligand Transferrin umfaßt.

28. Modifizierter chimärer monoclonaler Antikörper nach einem der Ansprüche 1 und 5 bis 9, immunologisch reaktiver Komplex nach einem der Ansprüche 2 und 5 bis 9 oder chimäres Polypeptid nach Anspruch 3, 4, 8 oder 9, wobei der Rezeptor-bindende Ligand ein Lymphokin umfaßt.

29. Modifizierter chimärer monoclonaler Antikörper, immunologisch reaktiver Komplex oder chimäres Polypeptid nach Anspruch 28, wobei das Lymphokin ausgewählt ist aus Makrophagen-Hemmfaktor, Leukocytenmigration-Hemmfaktor, Makrophagen-aktivierender Faktor, Makrophagen-cytotoxischer Faktor, Interleukin-1, Interleukin-2, Interleukin-3, Interleukin-4, Interleukin-5, Interleukin-6, Interleukin-7, Lymphotoxin, von Monocyten stammender Lymphocyten-aktivierender Faktor und T-Helferzellen ersetzender Faktor.

30. Modifizierter chimärer monoclonaler Antikörper, umfassend zwei Moleküle von je zwei unterschiedlichen Polypeptiden, wobei das kürzere als die leichten Ketten des Antikörpers und das längere der Polypeptide als die schweren Ketten des Antikörpers wirkt, wobei jedes Polypeptid, das als eine schwere Kette wirkt, einen variablen Bereich aufweist, der kennzeichnend ist für einen ersten Säuger, und einen konstanten Bereich, der kennzeichnend ist für einen zweiten Säuger, und jedes Polypeptid, das als eine leichte Kette wirkt, einen variablen Bereich aufweist, der kennzeichnend ist für einen Säuger, und einen konstanten Bereich, der kennzeichnend ist für einen Säuger,
wobei ein Rezeptor-bindender Ligand kovalent an die Enden der konstanten Bereiche jedes der Polypeptide gebunden ist, die als die schweren Ketten des Antikörpers wirken, oder mindestens einen Teil des konstanten Bereichs jedes der Polypeptide ersetzt, die als die schwere Ketten des Antikörpers wirken; oder
immunologisch reaktiver Komplex, umfassend zwei unterschiedliche Polypeptide, wobei das kürzere als eine leichte Kette und das längere als eine schwere Kette wirkt, wobei das Polypeptid, das als die schwere Kette wirkt, einen variablen Bereich aufweist, der kennzeichnend ist für einen ersten Säuger, und einen konstanten Bereich, der kennzeichnend ist für einen zweiten Säuger, und das Polypeptid, das als die leichte Kette wirkt, einen variablen Bereich aufweist, der kennzeichnend ist für einen Säuger, und einen konstanten Bereich, der kennzeichnend ist für einen zweiten Säuger,
wobei ein Rezeptor-bindender Ligand kovalent an die Enden eines konstanten Bereiches eines der Polypeptide gebunden ist oder mindestens einen Teil eines konstanten Bereichs eines der Polypeptide ersetzt;
wobei die variablen Bereiche der Polypeptide eine Domäne eines T-Zell-Rezeptors umfassen.

31. Modifizierter chimärer monoclonaler Antikörper, umfassend zwei Moleküle von je zwei unterschiedlichen Polypeptiden, wobei das kürzere als die leichten Ketten des Antikörpers und das längere der Polypeptide als die schweren Ketten des Antikörpers wirkt, wobei jedes Polypeptid, das als eine schwere Kette wirkt, einen variablen Bereich aufweist, der kennzeichnend ist für einen ersten Säuger, und einen konstanten Bereich, der kennzeichnend ist für einen zweiten Säuger, und jedes Polypeptid, das als eine leichte Kette wirkt, einen variablen Bereich aufweist, der kennzeichnend ist für einen Säuger, und einen konstanten Bereich, der kennzeichnend ist für einen Säuger,
wobei ein Rezeptor-bindender Ligand kovalent an die Enden der konstanten Bereiche jedes der Polypeptide gebunden ist, die als die schweren Ketten des Antikörpers wirken, oder mindestens einen Teil des konstanten Bereichs von jedem der Polypeptide ersetzt, die als die schweren Ketten des Antikörpers wirken; oder
immunologisch reaktiver Komplex, umfassend zwei unterschiedliche Polypeptide, wobei das kürzere als eine leichte Kette und das längere als eine schwere Kette wirkt, wobei das Polypeptid, das als die schwere Kette wirkt, einen variablen Bereich aufweist, der kennzeichnend ist für einen ersten Säuger, und einen konstanten Bereich, der kennzeichnend ist für einen zweiten Säuger, und das Polypeptid, das als die leichte Kette wirkt, einen variablen Bereich aufweist, der kennzeichnend ist für einen Säuger, und einen konstanten Bereich, der kennzeichnend ist für einen zweiten Säuger,
wobei ein Rezeptor-bindender Ligand kovalent an die Enden eines konstanten Bereiches eines der Polypeptide gebunden ist, oder mindestens einen Teil eines konstanten Bereichs einer der Polypeptide ersetzt;
wobei die variablen Bereiche der Polypeptide eine Domäne eines MHC-Antigens umfassen.

32. Modifizierter chimärer monoclonaler Antikörper oder immunologisch reaktiver Komplex nach Anspruch 31, wobei das MHC-Antigen ein HLA-Antigen ist.

33. Modifizierter chimärer monoclonaler Antikörper oder immunologisch reaktiver Komplex nach Anspruch 31, wobei das MHC-Antigen ein H-2-Antigen ist.

34. Modifizierter chimärer monoclonaler Antikörper, umfassend zwei Moleküle von je zwei unterschiedlichen Polypeptiden, wobei das kürzere als die leichten Ketten des Antikörpers und das längere der Polypeptide als die schweren Ketten des Antikörpers wirkt, wobei jedes Polypeptid, das als eine schwere Kette wirkt, einen variablen Bereich aufweist, der kennzeichnend ist für einen ersten Säuger, und einen konstanten Bereich, der kennzeichnend ist für einen zweiten Säuger, und jedes Polypeptid, das als eine leichte Kette wirkt, einen variablen Bereich aufweist, der kennzeichnend ist für einen Säuger, und einen konstanten Bereich, der kennzeichnend ist für einen Säuger,
wobei ein Rezeptor-bindender Ligand kovalent an die Enden der konstanten Bereiche jedes der Polypeptide gebunden ist, die als die schweren Ketten des Antikörpers wirken, oder mindestens einen Teil des konstanten Bereichs jedes der Polypeptide ersetzt, die als die schweren Ketten des Antikörpers wirken; oder
immunologisch reaktiver Komplex, umfassend zwei unterschiedliche Polypeptide, wobei das kürzere als eine leichte Kette und das längere als eine schwere Kette wirkt, wobei das Polypeptid, das als die schwere Kette wirkt, einen variablen Bereich aufweist, der kennzeichnend ist für einen ersten Säuger, und einen konstanten Bereich, der kennzeichnend ist für einen zweiten Säuger, und das Polypeptid, das als die leichte Kette wirkt, einen variablen Bereich aufweist, der kennzeichnend ist für einen Säuger, und einen konstanten Bereich, der kennzeichnend ist für einen zweiten Säuger,
wobei ein Rezeptor-bindender Ligand kovalent an die Enden eines konstanten Bereiches eines der Polypeptide gebunden ist oder mindestens einen Teil eines konstanten Bereichs eines der Polypeptide ersetzt;
wobei die variablen Bereiche der Polypeptide eine Domäne eines Oberflächenglykoproteins CD4 umfassen.

35. Modifizierter chimärer monoclonaler Antikörper, umfassend zwei Moleküle von je zwei unterschiedlichen Polypeptiden, wobei das kürzere als die leichten Ketten des Antikörpers und das längere der Polypeptide als die schweren Ketten des Antikörpers wirkt, wobei jedes Polypeptid, das als eine schwere Kette wirkt, einen variablen Bereich aufweist, der kennzeichnend ist für einen ersten Säuger, und einen konstanten Bereich, der kennzeichnend ist für einen zweiten Säuger, und jedes Polypeptid, das als eine leichte Kette wirkt, einen variablen Bereich aufweist, der kennzeichnend ist für einen Säuger, und einen konstanten Bereich, der kennzeichnend ist für einen Säuger,
wobei ein Rezeptor-bindender Ligand kovalent an die Enden der konstanten Bereiche jedes der Polypeptide gebunden ist, die als die schweren Ketten des Antikörpers wirken, oder mindestens einen Teil des konstanten Bereichs jedes der Polypeptide ersetzt, die als die schweren Ketten des Antikörpers wirken;
wobei die variablen Bereiche der Polypeptide eine Domäne eines Oberflächenglykoproteins CD8 umfassen.

**36.** Chimärer monoclonaler Antikörper nach einem der Ansprüche 1 und 5 bis 35, wobei der Antikörper ein IgG-Antikörper ist.

**37.** Modifizierter chimärer monoclonaler Antikörper, umfassend zwei Moleküle von je zwei unterschiedlichen Polypeptiden, wobei das kürzere als die leichten Ketten des Antikörpers und das längere der Polypeptide als die schweren Ketten des Antikörpers wirkt, wobei jedes Polypeptid, das als eine schwere Kette wirkt, einen variablen Bereich aufweist, der kennzeichnend ist für einen ersten Säuger, und einen konstanten Bereich, der kennzeichnend ist für einen zweiten Säuger, und jedes Polypeptid, das als eine leichte Kette wirkt, einen variablen Bereich aufweist, der kennzeichnend ist für einen Säuger, und einen konstanten Bereich, der kennzeichnend ist für einen Säuger, wobei ein Rezeptor-bindender Ligand kovalent an die Enden der konstanten Bereiche jedes der Polypeptide gebunden ist, die als die schweren Ketten des Antikörpers wirken, oder mindestens einen Teil des konstanten Bereichs jedes der Polypeptide ersetzt, die als die schweren Ketten des Antikörpers wirken; wobei der Antikörper ein IgA-Antikörper ist.

**38.** Modifizierter chimärer monoclonaler Antikörper, umfassend zwei Moleküle von je zwei unterschiedlichen Polypeptiden, wobei das kürzere als die leichten Ketten des Antikörpers und das längere der Polypeptide als die schweren Ketten des Antikörpers wirkt, wobei jedes Polypeptid, das als eine schwere Kette wirkt, einen variablen Bereich aufweist, der kennzeichnend ist für einen ersten Säuger, und einen konstanten Bereich, der kennzeichnend ist für einen zweiten Säuger, und jedes Polypeptid, das als eine leichte Kette wirkt, einen variablen Bereich aufweist, der kennzeichnend ist für einen Säuger, und einen konstanten Bereich, der kennzeichnend ist für einen Säuger, wobei ein Rezeptor-bindender Ligand kovalent an die Enden der konstanten Bereiche jedes der Polypeptide gebunden ist, die als die schweren Ketten des Antikörpers wirken, oder mindestens einen Teil des konstanten Bereichs jedes der Polypeptide ersetzt, die als schwere Ketten des Antikörpers wirken; wobei der Antikörper ein IgD-Antikörper ist.

**39.** Modifizierter chimärer monoclonaler Antikörper, umfassend zwei Moleküle von je zwei unterschiedlichen Polypeptiden, wobei das kürzere als die leichten Ketten des Antikörpers und das längere der Polypeptide als die schweren Ketten des Antikörpers wirkt, wobei jedes Polypeptid, das als eine schwere Kette wirkt, einen variablen Bereich aufweist, der kennzeichnend ist für einen ersten Säuger, und einen konstanten Bereich, der kennzeichnend ist für einen zweiten Säuger, und jedes Polypeptid, das als eine leichte Kette wirkt, einen variablen Bereich aufweist, der kennzeichnend ist für einen Säuger, und einen konstanten Bereich, der kennzeichnend ist für einen Säuger, wobei ein Rezeptor-bindender Ligand kovalent an die Enden der konstanten Bereiche jedes der Polypeptide gebunden ist, die als die schweren Ketten des Antikörpers wirken, oder mindestens einen Teil des konstanten Bereichs jedes der Polypeptide ersetzt, die als die schweren Ketten des Antikörpers wirken; wobei der Antikörper ein IgE-Antikörper ist.

**40.** Modifizierter chimärer monoclonaler Antikörper, umfassend zwei Moleküle von je zwei unterschiedlichen Polypeptiden, wobei das kürzere als die leichten Ketten des Antikörpers und das längere der Polypeptide als die schweren Ketten des Antikörpers wirkt, wobei jedes Polypeptid, das als eine schwere Kette wirkt, einen variablen Bereich aufweist, der kennzeichnend ist für einen ersten Säuger, und einen konstanten Bereich, der kennzeichnend ist für einen zweiten Säuger, und jedes Polypeptid, das als eine leichte Kette wirkt, einen variablen Bereich aufweist, der kennzeichnend ist für einen Säuger, und einen konstanten Bereich, der kennzeichnend ist für einen Säuger, wobei ein Rezeptor-bindender Ligand kovalent an die Enden der konstanten Bereiche jedes der Polypeptide gebunden ist, die als die schweren Ketten des Antikörpers wirken, oder mindestens einen Teil des konstanten Bereichs jedes der Polypeptide ersetzt, die als die schweren Ketten des Antikörpers wirken; wobei der Antikörper ein IgM-Antikörper ist.

**41.** Immunologisch reaktiver Komplex, umfassend zwei unterschiedliche Polypeptide, wobei das kürzere als eine leichte Kette und das längere als eine schwere Kette wirkt, wobei das Polypeptid, das als die schwere Kette wirkt, einen variablen Bereich aufweist, der kennzeichnend ist für einen ersten Säuger, und einen konstanten Bereich, der kennzeichnend ist für einen zweiten Säuger, und das Polypeptid, das als die leichte Kette wirkt, einen variablen Bereich aufweist, der kennzeichnend ist für einen Säuger, und einen konstanten Bereich, der kennzeichnend ist für einen zweiten Säuger, wobei ein Rezeptor-bindender Ligand kovalent an die Enden eines konstanten Bereiches eines der Polypeptide gebunden ist oder mindestens einen Teil eines konstanten Bereichs eines der Polypeptide ersetzt; wobei der rezeptorbindende Ligand mindestens einen Teil des konstanten Bereichs des Polypeptids ersetzt, das als die leichte Kette wirkt.

**42.** Modifizierter chimärer monoclonaler Antikörper, umfassend zwei Moleküle von je zwei unterschiedlichen Polypeptiden, wobei das kürzere als die leichten Ketten des Antikörpers und das längere der Polypeptide als die schweren Ketten des Antikörpers wirkt, wobei jedes Polypeptid, das als eine schwere Kette wirkt, einen variablen Bereich aufweist, der kennzeichnend ist für einen ersten Säuger, und einen konstanten Bereich, der kennzeichnend ist für einen zweiten Säuger, und jedes Polypeptid, das als eine leichte Kette wirkt, einen variablen Bereich aufweist, der kennzeichnend ist für einen Säuger, und einen konstanten Bereich, der kennzeichnend ist für einen Säuger, wobei ein Rezeptor-bindender Ligand kovalent an die Enden der konstanten Bereiche jedes der Polypeptide gebunden ist, die als die schweren Ketten des Antikörpers wirken, oder mindestens einen Teil des konstanten Bereichs jedes der Polypeptide ersetzt, die als die schweren Ketten des Antikörpers wirken; oder

immunologisch reaktiver Komplex, umfassend zwei unterschiedliche Polypeptide, wobei das kürzere als eine leichte Kette und das längere als eine schwere Kette wirkt, wobei das Polypeptid, das als die schwere Kette wirkt, einen variablen Bereich aufweist, der kennzeichnend ist für einen ersten Säuger, und einen konstanten Bereich, der kennzeichnend ist für einen zweiten Säuger, und das Polypeptid, das als die leichte Kette wirkt, einen variablen Bereich aufweist, der kennzeichnend ist für einen Säuger, und einen konstanten Bereich, der kennzeichnend ist für einen zweiten Säuger, wobei ein Rezeptor-bindender Ligand kovalent an die Enden eines konstanten Bereichs eines der Polypeptide gebunden ist oder mindestens einen Teil eines konstanten Bereichs einer der Polypeptide ersetzt; oder

chimäres Polypeptid, das als schwere oder leichte Kette eines Antikörpers wirken kann, umfassend einen variablen Bereich, der kennzeichnend ist für einen ersten Säuger, und einen konstanten Bereich, der kennzeichnend ist für einen zweiten Säuger, wobei ein Rezeptor-bindender Ligand kovalent an den konstanten Bereich des Polypeptids gebunden ist oder mindestens einen Teil des konstanten Bereichs des Polypeptids ersetzt;

an den eine Einheit kovalent gebunden ist.

**43.** Chimärer monoclonaler Antikörper, immunologisch reaktiver Komplex oder chimäres Polypeptid nach Anspruch 42, wobei die Einheit einen Wirkstoff umfaßt.

**44.** Chimärer monoclonaler Antikörper, immunologisch reaktiver Komplex oder chimäres Polypeptid nach Anspruch 43, wobei der Wirkstoff ein cytotoxisches Mittel ist.

**45.** Chimärer monoclonaler Antikörper, immunologisch reaktiver Komplex oder chimäres Polypeptid nach Anspruch 44, wobei das cytotoxische Mittel Methotrexat ist.

**46.** Chimärer monoclonaler Antikörper, immunologisch reaktiver Komplex oder chimäres Polypeptid nach Anspruch 44, wobei das cytotoxische Mittel ein Toxin ist.

**47.** Chimärer monoclonaler Antikörper, immunologisch reaktiver Komplex oder chimäres Polypeptid nach Anspruch 42, wobei die Einheit einen nachweisbaren Marker umfaßt.

**48.** Chimärer monoclonaler Antikörper, immunologisch reaktiver Komplex oder chimäres Polypeptid nach Anspruch 47, wobei der nachweisbare Marker Biotin, ein Fluorophor, ein Chromophor, ein Schwermetall, ein paramagnetisches Isotop oder ein Radioisotop ist.

**49.** Arzneimittel, umfassend den chimären monoclonalen Antikörper, immunologisch reaktiven Komplex oder das chimäre Polypeptid nach einem der Ansprüche 43 bis 46 und einen pharmazeutisch verträglichen Träger.

**50.** Nucleinsäuremolekül, das das chimäre Polypeptid nach einem der Ansprüche 3, 4 und 8 bis 28 codiert.

**51.** Expressionsvektor zur Produktion des chimären Polypeptids nach einem der Ansprüche 3, 4 und 8 bis 28, umfassend eine Nucleinsäure, die das chimäre Polypeptid codiert, und geeignete regulatorische Elemente, die so innerhalb des Vektors angeordnet sind, daß sie die Expres-sion des Polypeptids in einem geeigneten Wirt erlauben.

**52.** Verfahren zur Produktion eines modifizierten chimären monoclonalen Antikörpers, umfassend:

(a)

(i) Cotransfektion einer geeigneten/nicht-Antikörper produzierenden Wirtszelle mit zwei Expressionsplasmiden, wobei (A) eines dieser ein Polypeptid codiert, das als die schwere Kette des Antikörpers wirken kann und einen variablen Bereich aufweist, der kennzeichnend ist für einen ersten Säuger, und einen konstanten Bereich, der kennzeichend ist für einen zweiten Säuger, wobei ein Rezeptor-bindender Ligand mindestens einen Teil des konstanten Bereichs des schwere Kette-Polypeptids ersetzt, und (B) das andere ein Polypeptid codiert, das als die leichte Kette des Antikörpers wirkt und einen variablen Bereich aufweist, der kennzeichnend ist für einen Säuger, und einen konstanten Bereich, der kennzeichnend ist für einen Säuger, oder

(ii) Cotransfektion einer geeigneten/nicht-Antkörper produzierenden Wirtszelle mit einem Expressionsplasmid, das (A) ein Polypeptid codiert, das als die schwere Kette des Antikörpers wirken kann und einen variablen Bereich aufweist, der kennzeichnend ist für einen ersten Säuger, und einen konstanten Bereich, der kennzeichnend ist für einen zweiten Säuger, wobei ein Rezeptor-bindender Ligand mindestens einen Teil des konstanten Bereichs des schwere Kette-Polypeptids ersetzt, und (B) ein Polypeptid codiert, das als die leichte Kette des Antikörpers wirken kann und einen variablen Bereich aufweist, der kennzeichnend ist für einen Säuger, und einen konstanten Bereich, der kennzeichnend ist für einen Säuger;

(b) Behandlung der cotransfizierten Wirtszelle, so daß die Expression der Polypeptide erfolgt, die vom Plasmid oder den Plasmiden codiert werden, und Erzeugung des chimären monoclonalen Antikörpers innerhalb der Wirtszelle und Ausschleusung des Antikörpers in das Kulturmedium durch die Wirtszelle; und

(c) Gewinnung des erhaltenen ausgeschleusten chimären monoclonalen Antikörpers aus dem Kulturmedium;

wobei die menschliche Zelle eine Myelomzelle ist.

53. Verwendung des chimären monoclonalen Antikörpers, des immunologisch reaktiven Komplexes oder des chimären Polypeptids nach einem der Ansprüche 43 bis 46 für die Herstellung eines Arzneimittels nach Anspruch 49 zur Bereitstellung eines Wirkstoffes für eine Zelle, die einen Rezeptor für einen Wachstumsfaktor an ihrer Oberfläche aufweist, wobei der Rezeptor-bindende Ligand des Antikörpers den Wachstumsfaktor umfaßt, der an den Rezeptor bindet, so daß der Antikörper an die Zelle bindet und dadurch den Wirkstoff an die Zelle liefert.

54. Verwendung nach Anspruch 53, wobei die Zelle eine Gehirnzelle ist und wobei der Wachstumsfaktor durch die Bindung an den Rezeptor einen Transport des Antikörpers durch die Blut-Hirn-Schranke bewirkt.

55. Verwendung nach Anspruch 53, wobei die Zelle eine Blutzelle ist.

56. Verwendung nach Anspruch 53, wobei die Zelle eine Muskelzelle ist.

57. Verwendung nach Anspruch 53, wobei die Zelle eine Nervenzelle ist.

58. Verwendung nach Anspruch 53, wobei die Zelle eine Knochenzelle ist.

59. Verwendung nach Anspruch 53, wobei die Zelle eine Epithelzelle ist.

60. Verwendung nach Anspruch 53, wobei der Wachstumsfaktor ausgewählt ist aus insulinähnlichem Wachstumsfaktor 1, insulinähnlichem Wachstumsfaktor 2, Insulin und Transferrin.

61. Verwendung nach Anspruch 54, wobei die Gehirnzelle anomal ist und mit progressiver Dementia verbunden ist und wobei das Arzneimittel eine Menge des chimären Antikörpers umfaßt, die wirksam ist, um die progressive Dementia zum Stillstand zu bringen.

62. Verwendung nach Anspruch 54, wobei die Gehirnzelle anomal ist und mit einer Hirnrinden-Atrophie verbunden ist und wobei das Arzneimittel eine Menge des chimären Antikörpers umfaßt, die wirksam ist, um die Hirnrinden-Atrophie zum Stillstand zu bringen.

63. Verwendung nach Anspruch 54, wobei die Gehirnzelle maligne ist und mit einem Neurosarcom verbunden ist und wobei das Arzneimittel eine Menge des chimären Antikörpers umfaßt, die wirksam ist, um das Neurosarcom zum Stillstand zu bringen.

64. Verwendung nach einem der Ansprüche 54 bis 59, wobei die Gehirnzelle maligne ist und mit einem Lymphom verbunden ist und wobei das Arzneimittel eine Menge des chimären Antikörpers umfaßt, die wirksam ist, um das Lymphom zum Stillstand zu bringen.

**65.** Verwendung nach Anspruch 54, wobei die Gehirnzelle maligne ist und mit einem Carcinosarcom verbunden ist und wobei das Arzneimittel eine Menge des chimären Antikörpers umfaßt, die wirksam ist, um das Carcinosarcom zum Stillstand zu bringen.

**66.** Verwendung nach einem der Ansprüche 54 bis 59, wobei die Gehirnzelle maligne ist und mit einem Sarcom verbunden ist und wobei das Arzneimittel eine Menge des chimären Antikörpers umfaßt, die wirksam ist, um das Sarcom zum Stillstand zu bringen.

**67.** Verwendung nach Anspruch 54, wobei die Gehirnzelle maligne ist, und mit einer carcinomatösen Kleinhirn-Degeneration verbunden ist, und wobei das Arzneimittel eine Menge des chimären Antikörpers umfaßt, die wirksam ist, um die carcinomatöse Kleinhirn-Degeneration zum Stillstand zu bringen.

**68.** Verwendung nach Anspruch 53, wobei die Zelle maligne ist und mit einem Melanom verbunden ist, und wobei das Arzneimittel eine Menge des chimären Antikörpers umfaßt, die wirksam ist, um das Melanom zum Stillstand zu bringen.

**69.** Verwendung nach Anspruch 53, wobei die Zelle maligne ist und mit Brustkrebs verbunden ist und wobei das Arzneimittel eine Menge des chimären Antikörpers umfaßt, die wirksam ist, um Brustkrebs zum Stillstand zu bringen.

**70.** Verwendung nach Anspruch 53, wobei die Zelle maligne ist und mit einem Carcinom verbunden ist und wobei das Arzneimittel eine Menge des chimären Antikörpers umfaßt, die wirksam ist, um das Carcinom zum Stillstand zu bringen.

**71.** Verwendung des chimären monoclonalen Antikörpers, des immunologisch reaktiven Komplexes oder des chimären Polypeptids nach Anspruch 47 oder 48 für die Herstellung eines Diagnosemittels zum Nachweis einer Zelle mit einem Rezeptor für einen Wachstumsfaktor an ihrer Oberfläche, wobei der Rezeptor-bindende Ligand des Antikörpers den Wachstumsfaktor umfaßt, der an den Rezeptor bindet, so daß der Antikörper an die Zelle bindet und dadurch die Zelle nachweist.

**72.** Verwendung nach Anspruch 71, wobei die Zelle eine Gehirnzelle ist und wobei der Wachstumsfaktor durch Bindung an den Rezeptor den Transport des Antikörpers durch die Blut-Hirn-Schranke bewirkt.

**73.** Verwendung nach Anspruch 71 oder 72, wobei der Wachstumsfaktor ausgewählt ist aus insulinähnlichem Wachstumsfaktor 1, insulinähnlichem Wachstumsfaktor 2, Insulin und Transferrin.

**74.** Verwendung nach einem der Ansprüche 71 bis 73, wobei die Gehirnzelle anomal ist und mit einem argyrophilen Plaque verbunden ist und wobei das Diagnosemittel eine Menge des Antikörpers umfaßt, die wirksam ist, um den Nachweis des Plaques zu erlauben.

**75.** Verwendung nach einem der Ansprüche 71 bis 73, wobei die Gehirnzelle anomal ist und mit einem Gehirntumor verbunden ist und wobei das Diagnosemittel eine Menge des Antikörpers umfaßt, die wirksam ist, um den Nachweis des Tumors zu erlauben.

**76.** Verwendung nach Anspruch 53 oder 71, wobei die Zelle eine adipöse Zelle ist.

**77.** Verwendung nach Anspruch 53 oder 71, wobei die Zelle eine chronische myelogene Leukämie-Zelle ist.

**78.** Modifizierter chimärer monoclonaler Antikörper oder immunologisch reaktiver Komplex nach einem der Ansprüche 10 bis 27, 30 bis 35 und 37 bis 48, wobei der variable Bereich und der konstante Bereich der leichten Kette beide kennzeichnend für den zweiten Säuger sind.

**79.** Modifizierter chimärer monoclonaler Antikörper oder immunologisch reaktiver Komplex nach einem der Ansprüche 10 bis 27, 30 bis 35 und 37 bis 48, wobei der variable Bereich und der konstante Bereich der leichten Kette beide kennzeichnend für den ersten Säuger sind.

**80.** Modifizierter chimärer monoclonaler Antikörper oder immunologisch reaktiver Komplex nach einem der Ansprüche 10 bis 27, 30 bis 35 und 37 bis 48, wobei der variable Bereich der leichten Kette kennzeichnend für entweder den ersten oder den zweiten Säuger und der konstante Bereich der leichten Kette kennzeichnend für den anderen Säuger ist.

81. Modifizierter chimärer monoclonaler Antikörper, immunologisch reaktiver Komplex oder chimäres Polypeptid nach einem der Ansprüche 10 bis 27, 30 bis 35 und 37 bis 48, wobei der erste Säuger eine Maus und der zweite Säuger ein Mensch ist.

82. Modifizierter chimärer monoclonaler Antikörper, immunologisch reaktiver Komplex oder chimäres Polypeptid nach einem der Ansprüche 10 bis 27, 30 bis 35 und 37 bis 48, wobei der erste Säuger ein Mensch und der zweite Säuger eine Maus ist.

83. Verfahren zur Herstellung eines modifizierten chimären monoclonalen Antikörpers nach einem der Ansprüche 1, 5 bis 40, 42 bis 48 und 78 bis 82, umfassend:

   (a)

      (i) Cotransfektion einer geeigneten/nicht-Antikörper produzierenden Wirtszelle mit zwei Expressionsplasmiden, wobei (A) eines der beiden ein Polypeptid codiert, das als die schwere Kette des Antikörpers wirken kann und einen variablen Bereich aufweist, der kennzeichnend für einen ersten Säuger ist, und einen konstanten Bereich, der kennzeichnend für einen zweiten Säuger ist, wobei ein Rezeptor-bindender Ligand mindestens einen Teil des konstanten Bereichs des schwere Kette-Polypeptids ersetzt, und (B) das andere der beiden ein Polypeptid codiert, das als die leichte Kette des Antikörpers wirken kann und einen variablen Bereich aufweist, der kennzeichnend für einen Säuger ist, und einen konstanten Bereich, der kennzeichnend für einen Säuger ist, oder

      (ii) Cotransfektion einer geeigneten/nicht-Antkörper produzierenden Wirtszelle mit einem Expressionsplasmid, das (A) ein Polypeptid codiert, das als die schwere Kette des Antikörpers wirken kann und einen variablen Bereich aufweist, der kennzeichnend für einen ersten Säuger ist, und einen konstanten Bereich, der kennzeichnend für einen zweiten Säuger ist, wobei ein Rezeptor-bindender Ligand mindestens einen Teil des konstanten Bereichs des schwere Kette-Polypeptids ersetzt, und (B) ein Polypeptid codiert, das als die leichte Kette des Antikörpers wirken kann und einen variablen Bereich aufweist, der kennzeichnend für einen Säuger ist, und einen konstanten Bereich, der kennzeichnend für einen Säuger ist;

      (b) Behandlung der cotransfizierten Wirtszelle, so daß die Expression der durch das Plasmid oder die Plasmide codierten Polypeptide erfolgen kann, und Erzeugung des chimären monoclonalen Antikörpers innerhalb der Wirtszelle und Ausschleusung des Antikörpers in das Kulturmedium durch die Wirtszelle; und

      (c) Gewinnung des erhaltenen ausgeschleusten chimären monoclonalen Antikörpers aus dem Kulturmedium;

   wobei die menschliche Zelle eine Myelomzelle ist.

84. Verfahren zur Herstellung eines immunologisch reaktiven Komplexes nach einem der Ansprüche 2, 5 bis 34, 41 bis 48 und 78 bis 82, umfassend die Synthese der entsprechenden Komponenten unter Verwendung gentechnischer Verfahren oder anderer auf dem Fachgebiet bekannter Verfahren.

85. Verfahren zur Herstellung eines chimären Polypeptids nach einem der Ansprüche 3, 4, 8, 9 bis 29, 42 bis 48, 81 und 82, umfassend die Synthese des Polypeptids unter Verwendung gentechnischer Verfahren oder anderer auf dem Fachgebiet bekannter Verfahren.

86. Verfahren zur Herstellung des Arzneimittels nach Anspruch 49, umfassend die Kombination des Wirkstoffes mit einem pharmazeutisch verträglichen Träger.

**Revendications**

1. Anticorps monoclonal chimérique modifié comprenant deux molécules de deux polypeptides différents chacune, dont le plus court joue le rôle des chaînes légères de l'anticorps et le plus long joue le rôle des chaînes lourdes de l'anticorps, chaque polypeptide qui joue le rôle d'une chaîne lourde ayant une région variable caractéristique d'un premier mammifère et une région constante caractéristique d'un second mammifère, et chaque polypeptide qui joue le rôle de chaîne légère ayant une région variable caractéristique d'un mammifère et une région constante caractéristique d'un mammifère, dans lequel un ligand de liaison de récepteur est fixé de manière covalente aux extrémités des régions constantes de chacun des polypeptides qui jouent le rôle des chaînes lourdes de l'anticorps.

2. Complexe immunologiquement réactif comprenant deux polypeptides différents, dont le plus court joue le rôle d'une chaîne légère et le plus long joue le rôle d'une chaîne lourde, le polypeptide qui joue le rôle de la chaîne lourde ayant une région variable caractéristique d'un premier mammifère et une région constante caractéristique d'un second mammifère, et le polypeptide qui joue le rôle de la chaîne légère ayant une région variable caractéristique d'un mammifère et une région constante caractéristique d'un second mammifère, dans lequel un ligand de liaison de récepteur est fixé de manière covalente aux extrémités d'une région constante de l'un des polypeptides.

3. Polypeptide chimérique capable de jouer le rôle d'une chaîne lourde d'un anticorps comprenant une région variable caractéristique d'un premier mammifère et une région constante caractéristique d'un second mammifère, dans lequel un ligand de liaison de récepteur est fixé de manière covalente à la région constante du polypeptide.

4. Polypeptide chimérique capable de jouer le rôle d'une chaîne légère d'un anticorps comprenant une région variable caractéristique d'un premier mammifère et une région constante caractéristique d'un second mammifère, dans lequel un ligand de liaison de récepteur est fixé de manière covalente à la région constante du polypeptide.

5. Anticorps monoclonal chimérique modifié selon la revendication 1 ou complexe immunologiquement réactif selon la revendication 2, dans lequel la région variable et la région constante de la chaîne légère sont l'une et l'autre caractéristiques du second mammifère.

6. Anticorps monoclonal chimérique modifié selon la revendication 1 ou complexe immunologiquement réactif selon la revendication 2, dans lequel la région variable et la région constante de la chaîne légère sont l'une et l'autre caractéristiques du premier mammifère.

7. Anticorps monoclonal chimérique modifié selon la revendication 1 ou complexe immunologiquement réactif selon la revendication 2, dans lequel la région variable de la chaîne légère est caractéristique du premier ou du second mammifère et la région constante de la chaîne légère est caractéristique de l'autre mammifère.

8. Anticorps monoclonal chimérique modifié selon l'une quelconque des revendications 1 et 5 à 7 ou complexe immunologiquement réactif selon l'une quelconque des revendications 2 et 5 à 7 ou polypeptide chimérique selon la revendication 3 ou 4, dans lequel le premier mammifère est la souris et le second mammifère est l'homme.

9. Anticorps monoclonal chimérique modifié selon l'une quelconque des revendications 1 et 5 à 7 ou complexe immunologiquement réactif selon l'une quelconque des revendications 2 et 5 à 7 ou polypeptide chimérique selon la revendication 3 ou 4, dans lequel le premier mammifère est l'homme et le second mammifère est la souris.

10. Anticorps monoclonal chimérique modifié comprenant deux molécules de deux polypeptides différents chacune, dont le plus court joue le rôle des chaînes légères de l'anticorps et le plus long joue le rôle des chaînes lourdes de l'anticorps, chaque polypeptide qui joue le rôle d'une chaîne lourde ayant une région variable caractéristique du premier mammifère et une région constante caractéristique d'un second mammifère, et chaque polypeptide qui joue le rôle d'une chaîne légère ayant une région variable caractéristique d'un mammifère et une région constante caractéristique d'un mammifère, dans lequel un ligand de liaison de récepteur est fixé de manière covalente aux extrémités des régions constantes de chacun des polypeptides qui jouent le rôle des chaînes lourdes de l'anticorps, ou remplace au moins une partie de la région constante de chacun des polypeptides qui jouent le rôle des chaînes lourdes de l'anticorps ; ou

complexe immunologiquement réactif comprenant deux polypeptides différents dont le plus court joue le rôle d'une chaîne légère et le plus long joue le rôle d'une chaîne lourde, le polypeptide qui joue le rôle de la chaîne lourde ayant une région variable caractéristique d'un premier mammifère et une région constante caractéristique d'un second mammifère, et le polypeptide qui joue le rôle de la chaîne légère ayant une région variable caractéristique d'un mammifère et une région constante caractéristique d'un second mammifère, dans lequel un ligand de liaison de récepteur est fixé de manière covalente aux extrémités d'une région constante de l'un des polypeptides ou remplace au moins une partie d'une région constante de l'un des polypeptides ; ou polypeptide chimérique capable de jouer le rôle d'une chaîne lourde ou d'une chaîne légère d'un anticorps comprenant une région variable caractéristique d'un premier mammifère et une région constante caractéristique d'un second mammifère, dans lequel un ligand de liaison de récepteur est fixé de manière covalente à la région constante du polypeptide ou remplace au moins une partie de la région constante du polypeptide, dans lequel le ligand de liaison de récepteur comprend un facteur de croissance.

**11.** Anticorps monoclonal chimérique modifié, complexe immunologiquement réactif ou polypeptide chimérique selon la revendication 10, dans lequel le facteur de croissance comprend l'insuline.

**12.** Anticorps monoclonal chimérique modifié, complexe immunologiquement réactif ou polypeptide chimérique selon la revendication 10, dans lequel le facteur de croissance comprend un facteur de croissance analogue à l'insuline.

**13.** Anticorps monoclonal chimérique modifié, complexe immunologiquement réactif ou polypeptide chimérique selon la revendication 12, dans lequel le facteur de croissance analogue à l'insuline comprend le facteur de croissance analogue à l'insuline 1.

**14.** Anticorps monoclonal chimérique modifié, complexe immunologiquement réactif ou polypeptide chimérique selon la revendication 13, dans lequel le facteur de croissance analogue à l'insuline comprend le facteur de croissance analogue à l'insuline 2.

**15.** Anticorps monoclonal chimérique modifié, complexe immunologiquement réactif ou polypeptide chimérique selon la revendication 10, dans lequel le facteur de croissance comprend un facteur mitogénique plaquettaire.

**16.** Anticorps monoclonal chimérique modifié, complexe immunologiquement réactif ou polypeptide chimérique selon la revendication 10, dans lequel le facteur de croissance comprend un facteur de croissance épidermique.

**17.** Anticorps monoclonal chimérique modifié, complexe immunologiquement réactif ou polypeptide chimérique selon la revendication 10, dans lequel le facteur de croissance comprend un facteur de croissance transformant.

**18.** Anticorps monoclonal chimérique modifié, complexe immunologiquement réactif ou polypeptide chimérique selon la revendication 17, dans lequel le facteur de croissance transformant comprend le facteur de croissance transformant $\alpha$.

**19.** Anticorps monoclonal chimérique modifié, complexe immunologiquement réactif ou polypeptide chimérique selon la revendication 17, dans lequel le facteur de croissance transformant comprend le facteur de croissance transformant $\beta$.

**20.** Anticorps monoclonal chimérique modifié, complexe immunologiquement réactif ou polypeptide chimérique selon la revendication 19, dans lequel le facteur de croissance transformant $\beta$ comprend le facteur de croissance transformant $\beta$1.

**21.** Anticorps monoclonal chimérique modifié, complexe immunologiquement réactif ou polypeptide chimérique selon la revendication 19, dans lequel le facteur de croissance transformant $\beta$ comprend le facteur de croissance transformant $\beta$2.

**22.** Anticorps monoclonal chimérique modifié, complexe immunologiquement réactif ou polypeptide chimérique selon la revendication 19, dans lequel le facteur de croissance transformant $\beta$ comprend le facteur de croissance transformant $\beta$3.

**23.** Anticorps monoclonal chimérique modifié, complexe immunologiquement réactif ou polypeptide chimérique selon la revendication 10, dans lequel le facteur de croissance comprend un facteur de croissance des nerfs.

**24.** Anticorps monoclonal chimérique modifié, complexe immunologiquement réactif ou polypeptide chimérique selon la revendication 10, dans lequel le facteur de croissance comprend une hormone de croissance.

**25.** Anticorps monoclonal chimérique modifié, complexe immunologiquement réactif ou polypeptide chimérique selon la revendication 10, dans lequel le facteur de croissance comprend un facteur libérant une hormone de croissance.

**26.** Anticorps monoclonal chimérique modifié comprenant deux molécules de deux polypeptides différents chacune, dont le plus court joue le rôle des chaînes légères de l'anticorps et le plus long joue le rôle des chaînes lourdes de l'anticorps, chaque polypeptide qui joue le rôle d'une chaîne lourde ayant une région variable caractéristique d'un premier mammifère et une région constante caractéristique d'un second mammifère, et chaque polypeptide qui joue le rôle d'une chaîne légère ayant une région variable caractéristique d'un mammifère et une région constante caractéristique d'un mammifère, dans lequel un ligand de liaison de récepteur est fixé de manière covalente aux extrémités des régions constantes de chacun des polypeptides qui jouent le rôle des chaînes lourdes de l'anticorps, ou remplace au moins une partie de la région constante de chacun des polypeptides qui jouent le rôle des chaînes lourdes de l'anticorps ; ou

complexe immunologiquement réactif comprenant deux polypeptides différents dont le plus court joue le rôle dune chaîne légale et le plus long joue le rôle d'une chaîne lourde, le polypeptide qui joue le rôle de la chaîne lourde ayant une région variable caractéristique d'un premier mammifère et une région constante caractéristique d'un second mammifère, et le polypeptide qui joue le rôle de la chaîne légère ayant une région variable caractéristique d'un mammifère et une région constante caractéristique d'un second mammifère, dans lequel un ligand de liaison de récepteur est fixé de manière covalente aux extrémités d'une région constante de l'un des polypeptides ou remplace au moins une partie d'une région constante de l'un des polypeptide ; ou polypeptide chimérique capable de jouer le rôle d'une chaîne lourde ou d'une chaîne légère d'un anticorps comprenant une région variable caractéristique d'un premier mammifère et une région constante caractéristique d'un second mammifère, dans lequel un ligand de liaison de récepteur est fixé de manière covalente à la région constante du polypeptide ou remplace au moins une partie de la région constante du polypeptide,

dans lequel le ligand de liaison de récepteur comprend un facteur nécrosant les tumeurs.

27. Anticorps monodonal chimérique modifié comprenant deux molécules de deux polypeptides différents chacune, dont le plus court joue le rôle du chaînes légères de l'anticorps et le plus long joue le rôle des chaînes lourdes de l'anticorps, chaque polypeptide qui joue le rôle d'une chaîne lourde ayant une région variable caractéristique d'un premier mammifère et une région constante caractéristique d'un second mammifère, et chaque polypeptide qui joue le rôle d'une chaîne légère ayant une région variable caractéristique d'un mammifère et une région constante caractéristique d'un mammifère, dans lequel un ligand de liaison de récepteur est fixé de manière covalente aux extrémités des régions constantes de chacun des polypeptides qui jouent le rôle des chaîns lourdes de l'anticorps, ou remplace au moins une partie de la région constante de chacun des polypeptides qui jouent le rôle des chaînes lourdes de l'anticorps ; ou

complexe immunologiquement réactif comprenant deux polypeptides différents dont le plus court joue le rôle dune chaîne légère et le plus long joue le rôle d'une chaîne lourde, le polypeptide qui joue le rôle de la chaîne lourde ayant une région variable caractéristique d'un premier mammifère et une région constante caractéristique d'un second mammifère, et le polypeptide qui joue le rôle de la chaîne légère ayant une région variable caractéristique d'un mammifère et une région constante caractéristique d'un second mammifère, dans lequel un ligand de liaison de récepteur est fixé de manière covalente aux extrémités d'une région constante de l'un des polypeptides ou remplace au moins une partie d'une région constante de l'un des polypeptides ; ou polypeptide chimérique capable de jouer le rôle d'une chaîne lourde ou d'une chaîne légère d'un anticorps comprenant une région variable caractéristique d'un premier mammifère et une région constante caractéristique d'un second mammifère, dans lequel un ligand de liaison de récepteur est fixé de manière covalente à la région constante du polypeptide ou remplace au moins une partie de la région constante du polypeptide ;

où le ligand de liaison de récepteur comprend la transferrine.

28. Anticorps monoclonal chimérique modifié selon l'une quelconque des revendications 1 et 5 à 9, complexe immunologiquement réactif selon l'une quelconque des revendications 2 et 5 à 9 ou polypeptide chimérique selon la revendication 3, 4, 8 ou 9, dans lequel le ligand de liaison de récepteur comprend une lymphokine.

29. Anticorps monoclonal chimérique modifié, complexe immunologiquement réactif ou polypeptide chimérique selon la revendication 28, dans lequel la lymphokine est choisie dans le groupe consistant en le facteur d'inhibition des macrophages, le facteur d'inhibition de la migration des leucocytes, le facteur d'activation des macrophages, le facteur de cytotoxicité des macrophages, l'interleukine-1, l'interleukine-2, l'interleukine-3, l'interleukine-4, l'interleukine-5, l'interleukine-6, l'interleukine-7, une lymphotoxine, le facteur activant les lymphocytes dérivé des monocytes et le facteur remplaçant les cellules T auxiliaires.

30. Anticorps monoclonal chimérique modifié comprenant deux molécules de deux polypeptides différents chacune, dont le plus court joue le rôle des chaînes légères de l'anticorps et le plus long joue le rôle des chaînes lourdes de l'anticorps, chaque polypeptide qui joue le rôle d'une chaîne lourde ayant une région variable caractéristique d'un premier mammifère et une région constante caractérisque d'un second mammifère et chaque polypeptide qui joue le rôle d'une chaîne légère ayant une région variable caractéristique d'un mammifère et une région constante caractéristique d'un mammifère, dans lequel un ligand de liaison de récepteur est fixé de manière covalente aux extrémités des régions constantes de chacun des polypeptides qui jouent le rôle des chaînes lourdes de l'anticorps, ou remplace au moins une partie de la région constante de chacun des polypeptides qui jouent le rôle des chaînes lourdes de l'anticorps; ou

complexe immunologiquement réactif comprenant deux polypeptides différents dont le plus court joue le rôle d'une chaîne légère et le plus long joue le rôle d'une chaîne lourde, le polypeptide qui joue le rôle de la chaîne lourde ayant une région variable caractéristique d'un premier mammifère et une région constante caractéristique d'un second mammifère, et le polypeptide qui joue le rôle de la chaîne légère ayant une région variable caractéristique d'un mammifère et une région constante caractéristique d'un second mammifère, dans lequel un ligand de liaison de récepteur est fixé de manière covalente aux extrémités d'une région constante de l'un des polypeptides ou remplace au moins une partie d'une région constante de l'un des polypeptides ; où les régions variables des polypeptides comprennent un domaine d'un récepteur de cellules T.

31. Anticorps monoclonal chimérique modifié comprenant deux molécules de deux polypeptides différents chacune, dont le plus court joue le rôle des chaînes légères de l'anticorps et le plus long joue le rôle des chaînes lourdes de l'anticorps, chaque polypeptide qui joue le rôle d'une chaîne lourde ayant une région variable caractéristique d'un premier mammifère et une région constante caractéristique d'un second mammifère, et chaque polypeptide qui joue le rôle d'une chaîne légère ayant une région variable caractéristique d'un mammifère et une région constante caractéristique d'un mammifère, dans lequel un ligand de liaison de récepteur est fixé de manière covalente aux extrémités des régions constantes de chacun des polypeptides qui jouent le rôle des chaînes lourdes de l'anticorps, ou remplace au moins une partie de la région constante de chacun des polypeptides qui jouent le rôle des chaînes lourdes de l'anticorps ; ou

complexe immunologiquement réactif comprenant deux polypeptides différents dont le plus court joue le rôle d'une chaîne légère et le plus long joue le rôle d'une chaîne lourde, le polypeptide qui joue le rôle de la chaîne lourde ayant une région variable caractéristique d'un premier mammifère et une région constante caractéristique d'un second mammifère, et le polypeptide qui joue le rôle de la chaîne légère ayant une région variable caractéristique d'un mammifère et une région constante caractéristique d'un second mammifère, dans lequel un ligand de liaison de récepteur est fixé de manière covalente aux extrémités d'une région constante de l'un des polypeptides ou remplace au moins une partie d'une région constante de l'un des polypeptides ; où les régions variables des polypeptides comprennent un domaine d'un antigène du CMH.

32. Anticorps monoclonal chimérique modifié ou complexe immunologiquement réactif selon la revendication 31, dans lequel l'antigène du CMH est un antigène HLA.

33. Anticorps monoclonal chimérique modifié ou complexe immunologiquement réactif selon la revendication 31, dans lequel l'antigène du CMH est un antigène H-2.

34. Anticorps monoclonal chimérique modifié comprenant deux molécules de deux polypeptides différents chaîne, dont le plus court joue le rôle des chaînes légères de l'anticorps et le plus long joue le rôle des chaînes lourdes de l'anticorps, chaque polypeptide qui joue le rôle d'une chaîne lourde ayant une région variable caractéristique d'un premier mammifère et une région constante caractéristique d'un second mammifère, et chaque polypeptide qui joue le rôle d'une chaîne légère ayant une région variable caractéristique d'un mammifère et une région constante caractéristique d'un mammifère, dans lequel un ligand de liaison de récepteur est fixé de manière covalente aux extrémités des régions constantes de chacun des polypeptides qui jouent le rôle des chaînes lourdes de l'anticorps, ou remplace au moins une partie de la région constante de chacun des polypeptides qui jouent le rôle des chaînes lourdes de l'anticorps ou

complexe immunologiquement réactif comprenant deux polypeptides différents dont le plus court joue le rôle d'une chaîne légère et le plus long joue le rôle d'une chaîne lourde, le polypeptide qui joue le rôle de la chaîne lourde ayant une région variable caractéristique d'un premier mammifère et une région constante caractéristique d'un second mammifère, et le polypeptide qui joue le rôle de la chaîne légère ayant une région variable caractéristique d'un mammifère et une région constante caractéristique d'un second mammifère, dans lequel un ligand de liaison de récepteur est fixé de manière covalente aux extrémités d'une région constante de l'un des polypeptides ou remplace au moins une partie d'une région constante de l'un des polypeptides ; où les régions variables des polypeptides comprennent un domaine d'une glycoprotéine de surface CD4.

35. Anticorps monoclonal chimérique modifié comprenant deux molécules de deux polypeptides différents chacune, dont le plus court joue le rôle des chaînes légères de l'anticorps et le plus long joue le rôle des chaînes lourdes de l'anticorps, chaque polypeptide qui joue le rôle d'une chaîne lourde ayant une région variable caractéristique d'un premier mammifère et une région constante caractéristique d'un second mammifère, et chaque polypeptide qui joue le rôle d'une chaîne légère ayant une région variable caractéristique d'un mammifère et une région constante caractéristique d'un mammifère, dans lequel un ligand de liaison de récepteur est fixé de manière covalente aux extrémités des régions constantes de chacun des polypeptides qui jouent le rôle des chaînes lourdes de l'anticorps, ou remplace au moins une partie de la région constante de chacun des polypeptides qui jouent le rôle des chaînes lourdes de l'anticorps ;

dans lequel les régions variables des polypeptides comprennent un domaine d'une glycoprotéine de surface CD8.

36. Anticorps monoclonal chimérique selon l'une quelconque des revendications 1 et 5 à 35, dans lequel l'anticorps est un anticorps IgG.

37. Anticorps monoclonal chimérique modifié comprenant deux molécules de deux polypeptides différents chacune, dont le plus court joue le rôle des chaînes légères de l'anticorps et le plus long joue le rôle des chaînes lourdes de l'anticorps, chaque polypeptide qui joue le rôle d'une chaîne lourde ayant une région variable caractéristique d'un premier mammifère et une région constante caractéristique d'un second mammifère, et chaque polypeptide qui joue le rôle d'une chaîne légère ayant une région variable caractéristique d'un mammifère et une région constante caractéristique d'un mammifère, dans lequel un ligand de liaison de récepteur est fixé de manière covalente aux extrémités des régions constantes de chacun des polypeptides qui jouent le rôle des chaînes lourdes de l'anticorps, ou remplace au moins une partie de la région constante de chacun des polypeptides qui jouent le rôle des chaînes lourdes de l'anticorps ;

dans lequel l'anticorps est un anticorps IgA.

38. Anticorps monoclonal chimérique modifié comprenant deux molécules de deux polypeptides différents chacune, dont le plus court joue le rôle des chaînes légères de l'anticorps et le plus long joue le rôle des chaînes lourdes de l'anticorps, chaque polypeptide qui joue le rôle d'une chaîne lourde ayant une région variable caractéristique d'un premier mammifère et une région constante caractéristique d'un second mammifère, et chaque polypeptide qui joue le rôle d'une chaîne légère ayant une région variable caractéristique d'un mammifère et une région constante caractéristique d'un mammifère, dans lequel un ligand de liaison de récepteur est fixé de manière covalente aux extrémités des régions constantes de chacun des polypeptides qui jouent le rôle des chaînes lourdes de l'anticorps, ou remplace au moins une partie de la région constante de chacun des polypeptides qui jouent le rôle des chaînes lourdes de l'anticorps ;

dans lequel l'anticorps est un anticorps IgD.

39. Anticorps monoclonal chimérique modifié comprenant deux molécules de deux polypeptides différents chacune, dont le plus court joue le rôle des chaînes légères de l'anticorps et le plus long joue le rôle des chaînes lourdes de l'anticorps, chaque polypeptide qui joue le rôle d'une chaîne lourde ayant une région variable caractéristique d'un premier mammifère et une région constante caractéristique d'un second mammifère, et chaque polypeptide qui joue le rôle d'une chaîne légère ayant une région variable caractéristique d'un mammifère et une région constante caractéristique d'un mammifère, dans lequel un ligand de liaison de récepteur est fixé de manière covalente aux extrémités des régions constantes de chacun des polypeptides qui jouent le rôle des chaînes lourdes de l'anticorps, ou remplace au moins une partie de la région constante de chacun des polypeptides qui jouent le rôle des chaînes lourdes de l'anticorps ;

dans lequel l'anticorps est un anticorps IgE.

40. Anticorps monoclonal chimérique modifié comprenant deux molécules de deux polypeptides différents chacune, dont le plus court joue le rôle des chaînes légères de l'anticorps et le plus long joue le rôle des chaînes lourdes de l'anticorps, chaque polypeptide qui joue le rôle d'une chaîne lourde ayant une région variable caractéristique d'un premier mammifère et une région constante caractéristique d'un second mammifère, et chaque polypeptide qui joue le rôle d'une chaîne légère ayant une région variable caractéristique d'un mammifère et une région constante caractéristique d'un mammifère, dans lequel un ligand de liaison de récepteur est fixé de manière covalente aux extrémités des régions constantes de chacun des polypeptides qui jouent le rôle des chaînes lourdes de l'anticorps, ou remplace au moins une partie de la région constante de chacun des polypeptides qui jouent le rôle des chaînes lourdes de l'anticorps ;

dans lequel l'anticorps est un anticorps IgM.

41. Complexe immunologiquement réactif comprenant deux polypeptides différents dont le plus court joue le rôle d'une chaîne légère et le plus long joue le rôle d'une chaîne lourde, le polypeptide qui joue le rôle de la chaîne lourde ayant une région variable caractéristique d'un premier mammifère et une région constante caractéristique d'un second mammifère et le polypeptide qui joue le rôle de la chaîne légère ayant une région variable caractéristique d'un mammifère et une région constante caractéristique d'un second mammifère, dans lequel un ligand de liaison de récepteur est fixé de manière covalente aux extrémités d'une région constante de l'un des polypeptides ou remplace au moins une partie d'une région constante de l'un des polypeptides ;

dans lequel le ligand de liaison de récepteur remplace au moins une partie de la région constante du polypeptide qui joue le rôle de la chaîne légère.

42. Anticorps monoclonal chimérique modifié comprenant deux molécules de deux polypeptides différents chacune, dont le plus court joue le rôle des chaînes légères de l'anticorps et le plus long joue le rôle des chaînes lourdes de l'anticorps, chaque polypeptide qui joue le rôle d'une chaîne lourde ayant une région variable caractéristique d'un premier mammifère et une région constante caractéristique d'un second mammifère, et chaque polypeptide qui joue le rôle d'une chaîne légère ayant une région variable caractéristique d'un mammifère et une région constante caractéristique d'un mammifère, dans lequel un ligand de liaison de récepteur est fixé de manière covalente aux extrémités des régions constantes de chacun des polypeptides qui jouent le rôle des chaînes lourdes de l'anticorps, ou remplace au moins une partie de la région constante de chacun des polypeptides qui jouent le rôle des chaînes lourdes de l'anticorps ; ou

complexe immunologiquement réactif comprenant deux polypeptides différents dont le plus court joue le rôle d'une chaîne légère et le plus long joue le rôle d'une chaîne lourde, le polypeptide qui joue le rôle de la chaîne lourde ayant une région variable caractéristique d'un premier mammifère et une région constante caractéristique d'un second mammifère, et le polypeptide qui joue le rôle de la chaîne légère ayant une région variable caractéristique d'un mammifère et une région constante caractéristique d'un second mammifère, dans lequel un ligand de liaison de récepteur est fixé de manière covalente aux extrémités d'une région constante de l'un des polypeptides ou remplace au moins une partie d'une région constante de l'un des polypeptides ; ou polypeptide chimérique capable de jouer le rôle d'une chaîne lourde ou d'une chaîne légère d'un anticorps comprenant une région variable caractéristique d'un premier mammifère et une région constante caractéristique d'un second mammifère, dans lequel un ligand de liaison de récepteur est fixé de manière covalente à la région constante du polypeptide ou remplace au moins une partie de la région constante du polypeptide ; auquel une entité est fixée de manière covalente.

43. Anticorps monoclonal chimérique, complexe immunologiquement réactif ou polypeptide chimérique selon la revendication 42, dans lequel l'entité comprend un médicament.

44. Anticorps monoclonal chimérique, complexe immunologiquement réactif ou polypeptide chimérique selon la revendication 43, dans lequel le médicament est un agent cytotoxique.

45. Anticorps monoclonal chimérique, complexe immunologiquement réactif ou polypeptide chimérique selon la revendication 44, dans lequel l'agent cytotoxique est le méthotrexate.

46. Anticorps monoclonal chimérique, complexe immunologiquement réactif ou polypeptide chimérique selon la revendication 44, dans lequel l'agent cytotoxique est une toxine.

47. Anticorps monoclonal chimérique, complexe immunologiquement réactif ou polypeptide chimérique selon la revendication 42, dans lequel l'entité comprend un marqueur détectable.

48. Anticorps monoclonal chimérique, complexe immunologiquement réactif ou polypeptide chimérique selon la revendication 47, dans lequel le marqueur détectable est la biotine, un fluorophore, un chromophore, un métal lourd, un isotope paramagnétique ou un radioisotope.

49. Composition pharmaceutique comprenant l'anticorps monoclonal chimérique, le complexe immunologiquement réactif ou le polypeptide chimérique selon l'une quelconque des revendications 43 à 46 et un support pharmaceutiquement acceptable.

50. Molécule d'acide nucléique codant le polypeptide chimérique selon l'une quelconque des revendications 3, 4 et 8 à 28.

51. Vecteur d'expression pour produire le polypeptide chimérique selon l'une quelconque des revendications 3, 4 et 8 à 28, comprenant un acide nucléique codant le polypeptide chimérique et des éléments régulateurs appropriés situés dans le vecteur de manière à permettre l'expression du polypeptide dans un hôte approprié.

52. Procédé de production d'un anticorps monoclonal chimérique modifié qui comprend :

   (a)

   (i) la cotransfection d'une cellule hôte non productrice d'anticorps appropriée avec deux plasmides d'expression dont l'un (A) code un polypeptide capable de jouer le rôle de la chaîne lourde de l'anticorps et ayant une région variable caractéristique d'un premier mammifère et une région constante caractéristique d'un second mammifère, où un ligand de liaison de récepteur remplace au moins une partie de la région constante du polypeptide de la chaîne lourde et dont l'autre (B) code un polypeptide capable de jouer le rôle de la chaîne légère de l'anticorps et ayant une région variable caractéristique d'un mammifère et une région constante caractéristique d'un mammifère ; ou
   (ii) la cotransfection d'une cellule hôte non productrice d'anticorps appropriée avec un plasmide d'expression qui code (A) un polypeptide capable de jouer le rôle de la chaîne lourde de l'anticorps et ayant une région variable caractéristique d'un premier mammifère et une région constante caractéristique d'un second mammifère, où un ligand de liaison de récepteur remplace au moins une partie de la région constante du polypeptide de la chaîne lourde et (B) un polypeptide capable de jouer le rôle de la chaîne legère de l'anticorps et ayant une région variable caractéristique d'un mammifère et une région constante caractéristique d'un mammifère;

   (b) le traitement de la cellule hôte cotransfectée de manière à réaliser l'expression des polypeptides codés par le plasmide ou les plasmides et la formation de l'anticorps monoclonal chimérique dans la cellule hôte et l'excrétion dans le milieu de culture de l'anticorps par la cellule hôte ; et
   (c) la récupération de l'anticorps monoclonal chimérique excrété résultant à partir du milieu de culture ;

   où la cellule humaine est une cellule de myélome.

53. Utilisation de l'anticorps monoclonal chimérique, du complexe immunologiquement réactif ou du polypeptide chimérique selon l'une quelconque des revendications 43 à 46 pour la préparation d'une composition pharmaceutique selon la revendication 49 pour fournir un médicament à une cellule ayant un récepteur pour un facteur de croissance sur sa surface, où le ligand de liaison de récepteur de l'anticorps comprend le facteur de croissance qui se lie au récepteur de sorte que l'anticorps se lie à la cellule et fournit ainsi le médicament à la cellule.

54. Utilisation selon la revendication 53, dans laquelle la cellule est une cellule cérébrale et dans laquelle le facteur de croissance, lorsqu'il se lie au récepteur, provoque le transport de l'anticorps au travers de la barrière hématoencéphalique.

55. Utilisation selon la revendication 53, dans laquelle la cellule est une cellule sanguine.

56. Utilisation selon la revendication 53, dans laquelle la cellule est une cellule musculaire.

57. Utilisation selon la revendication 53, dans laquelle la cellule est une cellule nerveuse.

58. Utilisation selon la revendication 53, dans laquelle la cellule est une cellule osseuse.

59. Utilisation selon la revendication 53, dans laquelle la cellule est une cellule épithéliale.

60. Utilisation selon la revendication 53, dans laquelle le facteur de croissance est choisi dans le groupe consistant en le facteur de croissance analogue à l'insuline-1, le facteur de croissance analogue à l'insuline-2, l'insuline et la transferrine.

61. Utilisation selon la revendication 54, dans laquelle la cellule cérébrale est anormale et est associée à la démence progressive et dans laquelle la composition pharmaceutique comprend une quantité de l'anticorps chimérique efficace pour arrêter la démence progressive.

62. Utilisation selon la revendication 54, dans laquelle la cellule cérébrale est anormale et est associée à une atrophie du cortex cérébral et dans laquelle la composition pharmaceutique comprend une quantité de l'anticorps chimérique efficace pour arrêter l'atrophie du cortex cérébral.

63. Utilisation selon la revendication 54, dans laquelle la cellule cérébrale est maligne et est associée à un neurosarcome et dans laquelle la composition pharmaceutique comprend une quantité de l'anticorps chimérique efficace pour arrêter le neurosarcome.

64. Utilisation selon l'une quelconque des revendications 54 à 59, dans laquelle la cellule cérébrale est maligne et est associée à un lymphome et dans laquelle ladite composition pharmaceutique comprend une quantité de l'anticorps chimérique efficace pour arrêter le lymphome.

65. Utilisation selon la revendication 54, dans lequel la cellule cérébrale est maligne et est associée à un carcinosarcome et dans laquelle la composition pharmaceutique comprend une quantité de l'anticorps chimérique efficace pour arrêter le carcinosarcome.

66. Utilisation selon l'une quelconque des revendications 54 à 59, dans laquelle la cellule cérébrale est maligne et est associée à un sarcome et dans laquelle ladite composition pharmaceutique comprend une quantité de l'anticorps chimérique efficace pour arrêter le sarcome.

67. Utilisation selon la revendication 54, dans laquelle la cellule cérébrale est maligne et est associée à une dégénérescence cérébelleuse cancéreuse et dans laquelle la composition pharmaceutique comprend une quantité de l'anticorps chimérique efficace pour arrêter la dégénérescence cérébelleuse cancéreuse.

68. Utilisation selon la revendication 53, dans laquelle la cellule est maligne et est associée à un mélanome et dans laquelle ladite composition pharmaceutique comprend une quantité de l'anticorps chimérique efficace pour arrêter le mélanome.

69. Utilisation selon la revendication 53, dans laquelle la cellule est maligne et est associée à un cancer du sein et dans laquelle ladite composition pharmaceutique comprend une quantité de l'anticorps chimérique efficace pour arrêter le cancer du sein.

70. Utilisation selon la revendication 53, dans laquelle la cellule est maligne et est associée à un carcinome et dans laquelle ladite composition pharmaceutique comprend une quantité de l'anticorps chimérique efficace pour arrêter le carcinome.

71. Utilisation de l'anticorps monoclonal chimérique, du complexe immunologiquement réactif ou du polypeptide chimérique selon la revendication 47 ou 48 pour la préparation d'une composition diagnostique pour détecter une cellule ayant un récepteur pour un facteur de croissance sur sa surface, dans laquelle le ligand de liaison de récepteur de l'anticorps comprend le facteur de croissance qui se lie au récepteur, de sorte que l'anticorps se lie à la cellule et détecte ainsi la cellule.

72. Utilisation selon la revendication 71, dans laquelle la cellule est une cellule cérébrale et dans laquelle le facteur de croissance, lorsqu'il se lie au récepteur, provoque le transport de l'anticorps au travers de la barrière hématoencéphalique.

73. Utilisation selon la revendication 71 ou 72, dans laquelle le facteur de croissance est choisi dans le groupe consistant en le facteur de croissance analogue à l'insuline-1, le facteur de croissance analogue à l'insuline-2, l'insuline et la transferrine.

74. Utilisation selon l'une quelconque des revendications 71 à 73, dans laquelle la cellule cérébrale est anormale et est associée à une plaque argyrophile, et la composition diagnostique comprend une quantité de l'anticorps efficace pour permettre la détection de la plaque.

**75.** Utilisation selon l'une quelconque des revendications 71 à 73, dans laquelle la cellule cérébrale est anormale et est associée à une tumeur cérébrale, et la composition diagnostique comprend une quantité de l'anticorps efficace pour permettre la détection de la tumeur.

**76.** Utilisation selon la revendication 53 ou 71, dans laquelle la cellule est une cellule adipeuse.

**77.** Utilisation selon la revendication 53 ou 71, dans laquelle la cellule est une cellule de leucémie myéloïde chronique.

**78.** Anticorps monoclonal chimérique modifié ou complexe immunologiquement réactif selon l'une quelconque des revendications 10 à 27, 30 à 35 et 37 à 48, dans lequel la région variable et la région constante de la chaîne légère sont l'une et l'autre caractéristiques du second mammifère.

**79.** Anticorps monoclonal chimérique modifié ou complexe immunologiquement réactif selon l'une quelconque des revendications 10 à 27, 30 à 35 et 37 à 48, dans lequel la région variable et la région constante de la chaîne légère sont l'une et l'autre caractéristiques du premier mammifère.

**80.** Anticorps monoclonal chimérique modifié ou complexe immunologiquement réactif selon l'une quelconque des revendications 10 à 27, 30 à 35 et 37 à 48, dans lequel la région variable de la chaîne légère est caractéristique du premier ou du second mammifère et la région constante de la chaîne légère est caractéristique de l'autre mammifère.

**81.** Anticorps monoclonal chimérique modifié, complexe immunologiquement réactif ou polypeptide chimérique selon l'une quelconque des revendications 10 à 27, 30 à 35 et 37 à 48, dans lequel le premier mammifère est la souris et le second mammifère et l'homme.

**82.** Anticorps monoclonal chimérique modifié, complexe immunologiquement réactif ou polypeptide chimérique selon l'une quelconque des revendications 10 à 27, 30 à 35 et 37 à 48, dans lequel le premier mammifère est l'homme et le second mammifère est la souris.

**83.** Procédé de production d'un anticorps monoclonal chimérique modifié selon l'une quelconque des revendications 1, 5 à 40, 42 à 48 et 78 à 82 qui comprend :

(a)

(i) la cotransfection d'une cellule hôte non productrice d'anticorps appropriée avec deux plasmides d'expression dont l'un (A) code un polypeptide capable de jouer le rôle de la chaîne lourde de l'anticorps et ayant une région variable caractéristique d'un premier mammifère et une région constante caractéristique d'un second mammifère, où un ligand de liaison de récepteur remplace au moins une partie de la région constante du polypeptide de la chaîne lourde et dont l'autre (B) code un polypeptide capable de jouer le rôle de la chaîne légère de l'anticorps et ayant une région variable caractéristique d'un mammifère et une région constante que d'un mammifère ; ou
(ii) la cotransfection d'une cellule hôte non productrice d'anticorps appropriée avec un plasmide d'expression qui code (A) un polypeptide capable de jouer le rôle de la chaîne lourde de l'anticorps et ayant une région variable caractéristique d'un premier mammifère et une région constante caractéristique d'un second mammifère, où un ligand de liaison de récepteur remplace au moins une partie de la région constante du polypeptide de la chaîne lourde et (B) un polypeptide capable de jouer le rôle de la chaîne légère de l'anticorps et ayant une région variable caractéristique d'un mammifère et une région constante caractéristique d'un mammifère;

(b) le traitement de la cellule hôte cotransfectée de manière à réaliser l'expression des polypeptides codés par le plasmide ou les plasmides et la formation de l'anticorps monoclonal chimérique dans la cellule hôte et l'excrétion dans le milieu de culture de l'anticorps par la cellule hôte ; et
(c) la récupération de l'anticorps monoclonal chimérique excrété résultant à partir du milieu de culture ;

où la cellule humaine est une cellule de myélome.

84. Procédé de préparation d'un complexe immunologiquement réactif selon l'une quelconque des revendications 2, 5 à 34, 41 à 48 et 78 à 82 qui comprend la synthèse des composants respectifs par génie génétique ou par d'autres méthodes connues dans la technique.

85. Procédé de préparation d'un polypeptide chimérique selon l'une quelconque des revendications 3, 4, 8, 9 à 29, 42 à 48, 81 et 82 qui comprend la synthèse du polypeptide par génie génétique ou par d'autres méthodes connues dans la technique.

86. Procédé de préparation de la composition pharmaceutique selon la revendication 49 qui comprend la combinaison de l'agent actif avec un support pharmaceutiquement acceptable.

## FIGURE 1(A)

### HUMAN IgG3 CH2

```
        ←————IVS——+—CH2————————▶
WT:  TCTTCCTCA    GCA  CCT  GAA  CTC  CTG
                  Ala  Pro  Glu  Leu  Leu
                            ⬇
        ←————IVS——+——CH2————————▶
MT:  TCTTCCTCA    GCA  GCT  GAA  CTC  CTG
                       ————————
                          PvuII
```

### Rat IGF1 cDNA

```
        ←————Leader Seq——+—Exon2————▶
WT:  TCG  GCC  ACA  GCC  GGA  CCA  GAG  ACC
     Ser  Ala  Thr  Ala  Gly  Pro  Glu  Thr
                         ⬇
        ←————Leader Seq——+—Exon2————▶
MT:  TCG  GCC  ACA  GCT  GGA  CCA  GAG  ACC
                    ————————
                        PvuII
```

### IgG3-IGF1 Fusion Gene

```
        ←—IVS————+—CH2—|LS|————————Exon2————▶
     TCTTCCTCA GCA   GCT  GGA  CCA  GAG  ACC
                Ala   Ala  Gly  Pro  Glu  Thr
```

## FIGURE 1(B)

## FIGURE 2(A)

## FIGURE 2(B)

# FIGURE 2(C)

**UNPROCESSED
HOMODIMER
(Top)**

**HETERODIMER
(Middle)**

**PROCESSED
HOMODIMER
(Bottom)**

# FIGURE 3(A)

(IgG3-IGF1)

(IgG3-IGF1) $_2$L$_2$ {

## FIGURE 3(B)

## FIGURE 4

**FIGURE 5(A)**

FIGURE 5(B)

## FIGURE 6

# FIGURE 7

FIGURE 8

# FIGURE 9

## FIGURE 10

Mouse V
Region

EcoRI    L VDJ

EcoRI

pBR322 ori

SV40

Sal I

CH1

E. coli-gpt

Hinges

Human IgG3
C Region

pSV2 VDNS HUG3 HTF

CH2
Pvu II

Bam HI

Bam HI
Pvu II
Pvu II

EcoRI

EcoRI

Human Transferrin

FIGURE 11

EP 0 521 985 B1